# EUROPEAN PATENT SPECIFICATION

(11) **EP 3 577 462 B1**
(45) Date of publication and mention of the grant of the patent: **17.04.2024**
(21) Application number: 18705844.1
(22) Date of filing: 05.02.2018
(51) Int. Cl.: C07K 14/62, C07K 14/705, A61K 38/00

(54) **INTERACTION BETWEEN C-PEPTIDES AND ELASTIN RECEPTOR, A MODEL FOR UNDERSTANDING VASCULAR DISEASE**
WECHSELWIRKUNG ZWISCHEN C-PEPTIDEN UND ELASTIN-REZEPTOR, EIN MODELL ZUM VERSTÄNDNIS DER GEFÄSSERKRANKUNG
INTERACTION ENTRE LES PEPTIDES-C ET LE RÉCEPTEUR DE L'ELASTINE, UN MODÈLE POUR COMPRENDRE LA MALADIE VASCULAIRE

(30) Priority: 06.02.2017 EP 17154889
(43) Date of publication of application: 11.12.2019
(73) Proprietor: Resiliun B.V., 7958 NZ Koekange (NL)
(72) Inventor: WENSVOORT, Gert, 7958 NZ Koekange (NL)
(74) Representative: Hoffmann Eitle
(86) International application number: PCT/EP2018/052824
(87) International publication number: WO 2018/141970

(56) References cited:
- EP-A1- 2 626 704
- WO-A1-2013/153177
- WO-A1-2014/108396
- WO-A1-2015/140317
- US-A1- 2014 274 891
- N. PATEL ET AL: "Fasting Serum C-Peptide Levels Predict Cardiovascular and Overall Death in Nondiabetic Adults", JOURNAL OF THE AMERICAN HEART ASSOCIATION, vol. 1, no. 6, 13 December 2012 (2012-12-13), pages e003152-e003152, XP055461108, DOI: 10.1161/JAHA.112.003152
- S. BLAISE ET AL: "Elastin-Derived Peptides Are New Regulators of Insulin Resistance Development in Mice", DIABETES, vol. 62, no. 11, 6 August 2013 (2013-08-06), pages 3807-3816, XP055461109, US ISSN: 0012-1797, DOI: 10.2337/db13-0508
- HELENE SKJ?T-ARKIL ET AL: "Acute Myocardial Infarction and Pulmonary Diseases Result in Two Different Degradation Profiles of Elastin as Quantified by Two Novel ELISAs", PLOS ONE, vol. 8, no. 6, 21 June 2013 (2013-06-21), page e60936, XP055183266, DOI: 10.1371/journal.pone.0060936
- CHARLOTTE BLANCHEVOYE ET AL: "Interaction between the Elastin Peptide VGVAPG and Human Elastin Binding Protein", JOURNAL OF BIOLOGICAL CHEMISTRY, vol. 288, no. 2, 11 January 2013 (2013-01-11), pages 1317-1328, XP055461117, US ISSN: 0021-9258, DOI: 10.1074/jbc.M112.419929
- Matthew Vincent: "Ligands of the elastin receptor stimulate elastogenesis through cross-activation of the IFG-1 signalling pathway", , 31 December 2014 (2014-12-31), XP002779326, Retrieved from the Internet: URL:http://hdl.handle.net/1807/68068 [retrieved on 2018-03-20]
- CODY J. WENTHUR ET AL: "Drugs for Allosteric Sites on Receptors", ANNUAL REVIEW OF PHARMACOLOGY AND TOXICOLOGY., vol. 54, no. 1, 6 January 2014 (2014-01-06), pages 165-184, XP055461123, US ISSN: 0362-1642, DOI: 10.1146/annurev-pharmtox-010611-134525

## Description

### Technical field

The invention belongs to the field of human and veterinary medicine, and belongs to the field of pharmacy, biotechnology and drug development. The invention relates to the etiology of metabolic syndrome and provides a method for diagnosing vascular disease risk of a human.

### Background

Our all-too-human habit to overeat and the easy availability of everyday food have resulted in a worldwide obesity epidemic with dire consequences to our health. One-and-half (1.5) billion people are overweight (of which 0.5 billion obese), and a great many of those suffer from a chronic inflammatory disease often called metabolic syndrome: the major cause of unhealthy aging and death in high- and middle-income countries. These 1.5 billion people are at increased risk to develop cardiovascular disease (chronic inflammation of the blood vessels [atheromatous disease, arteriosclerosis, atherosclerosis], increased blood pressure [hypertension] and increased abnormal fat levels [dyslipidaemia] in the blood), leading up to heart attack and stroke. At least 30% of these 1.5 billion are at further risk to develop diabetes type 2 (World Health Organization). Others develop too early manifestations of aging such as kidney failure or dementia. Heart failure, as non-fatal and fatal myocardial infarction and peripheral arterial disease (PAD) are the most common initial manifestations of cardiovascular disease in type 2 diabetes, others are transient ischemic attacks (TIA) or ischaemic stroke and stable angina. Living a sedentary life and smoking further increases risks of dying from these conditions. Currently, no satisfying medical understanding (other than excess diet) exists of the causal events leading to the initially mild but ultimately chronic inflammatory disease that underlies these staggering figures. Why this food-intake-induced inflammation occurs and affects so many people is largely unknown and subject of much debate.

C-peptide is the linking peptide between the A- and B-chains in the proinsulin molecule. After cleavage in the endoplasmic reticulum of pancreatic islet beta-cells, insulin and a 35-amino acid peptide are generated. The latter is processed to the 31-amino acid peptide, C-peptide, by enzymatic removal of two basic residues on either side of the molecule. C-peptide is cosecreted with insulin in equimolar amounts from the pancreatic islet beta-cells into the portal circulation. Besides its contribution to the folding of the two-chain insulin structure, further biologic activity of C-peptide was questioned for many years after its discovery.

C-peptide is a 31-amino acid peptide having the sequence
SEQ ID NO: 1 (EAEDLQVGQVELGGGPGAGSLQPLALEGSLQ, in the one-letter amino acid code).

C-peptide classically is ascribed a tripartite overall structure, with more conserved N- and C-terminal segments and a more variable mid-sequence, or internal, and hydrophobic midportion. Thus, in the case of C-peptide the N-terminal segment is often regarded as residues 1-12 (SEQ ID NO: 2 (EAEDLQVGQVEL)), the mid-portion as residues 13-25 (SEQ ID NO: 3 (GGGPGAGSLQPLA)), and the C-terminal segment as residues 26-31 (SEQ ID NO: 4 (LEGSLQ)). The tetrapeptide SEQ ID NO: 5 (EAED) is thought to be required in the process of folding the two-chain insulin structure in the beta cells (Chen at al., J Biochem. 2002 Jun;131(6):855-9). Recently, some studies suggested that the C-terminal pentapeptide (SEQ ID NO: 6 EGSLQ) in C-peptide and SEQ ID NO: 7 (EVARQ) in rat C-peptide) of C-peptide which shows a well-defined secondary structure may induce intracellular Ca2+ increases in human renal tubular cells (Shafqat et al., Cell Mol Life Sci. 2002 Jul;59(7):1185-9.).

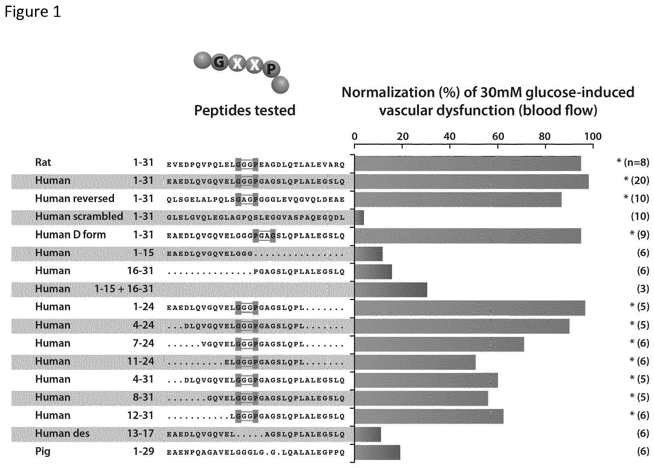

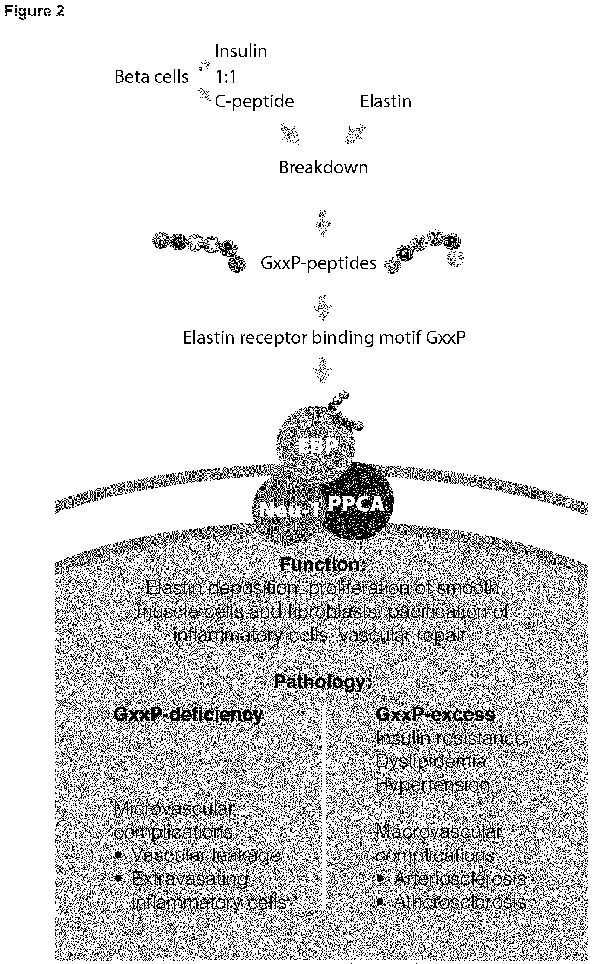

C-peptide is produced in equal amounts to insulin and is the best additional measure of endogenous insulin secretion in patients with diabetes. Measurement of insulin secretion using C-peptide is considered helpful in clinical practice: differences in insulin secretion are fundamental to the different treatment requirements of Type 1 and Type 2 diabetes. Jones and Hattersley (Diabet Med. 2013 Jul;30(7):803-17) review the use of C-peptide measurement in the clinical management of patients with diabetes, including the interpretation and choice of C-peptide test and its use to assist diabetes classification and choice of treatment, and recommendations for where C-peptide should be used, choice of test and interpretation of results. As the relationships between C-peptide levels and metabolic control and chronic complications are poorly known in type 2 diabetes, due to the slow decline of beta-cell function, Bo et al., (Acta Diabetol. 2000;37(3):125-9) evaluated these associations in a cohort of type 2 diabetic patients. Biological effects of C-peptide are thought to be mediated by interaction with insulin or via specific or nonspecific membrane interaction. Some studies in the art support the theory of specific interactions with a yet to be indentified GPCR. However, the D-enantiomer of C-peptide has the same biological activity as the L-enantiomer (Ido et al, Science, 1997, 277(5325):563-6), thus finding reverse (retro) and all-D-amino acid (enantio) C-peptides equipotent to native C-peptide, they conclude the activity of SEQ ID NO: 8 (GGGPGAG) to be not mediated by a receptor, thereby teaching away from a receptor for C-peptide, which has suggested to those in the art that other, receptor-independent, interactions are important for function. Formation of cation-selective channels in lipid bilayers has also led to suggestions of a more nonspecific interaction. Thus, a receptor for C-peptide has remained elusive. Ido et al. (Science, 1997 Jul 25;277(5325):563-6; and figure 1 in this application) show C-peptide fragments with hydrophobic midportion SEQ ID NO: 8 (GGGPGAG) to normalize glucose-induced vascular dysfunction in rat granular tissue, they however, have not provided testing of C-peptide fragments in combination with treatment of those rats with insulin, and teach away from receptor-mediated activity of fragments having said hydrophobic midportion. In US20020107175 a C-peptide fragment SEQ ID NO: 9 (ELGGGPGAG) and some of its smaller fragments stimulate Na.sup.+K.sup.+ATPase activity of rat renal tubule cells. US20060234914 and 20070082842 list N-terminal- and/or C-terminal-C-peptide fragments that comprise at least one glutamine (in three letter code Glu; in one-letter code E) to provide biological activity not related to the activity of above discussed hydrophobic midportion SEQ ID NO: 8 (GGGPGAG), which midportion sequence is not found in any of the fragments listed in US20060234914 nor 20070082842.

Type 1 diabetes is generally characterized by insulin and C-peptide deficiency, due to an autoimmune destruction of the pancreatic islet beta-cells. The patients are therefore dependent on exogenous insulin to sustain life. Several factors may be of importance for the pathogenesis of the disease, e.g., genetic background, environmental factors, and an aggressive autoimmune reaction following a temporary infection (Akerblom H K et al.: Annual Medicine 29(5): 383-385, (1997)). Currently insulin-requiring patients are provided with exogenous insulin which has been separated from the C-peptide, and thus do not receive exogenous C-peptide therapy. By contrast, most type 2 diabetic subjects initially still produce both insulin and C-peptide endogenously but are generally characterized by insulin resistance in skeletal muscle, adipose tissue, and liver, among other tissues.

Many type 1 and end-phase type 2 diabetic patients (that do not longer produce insulin and C-peptide) eventually develop and suffer from a constellation of long-term complications of diabetes that in many cases are more severe and widespread than in early phase or new-onset type 2 diabetes (wherein insulin and C-peptide are stil produced but the patient is resistant to insulin. For example, microvascular complications involving the retina, kidneys, and nerves are a major cause of morbidity and mortality in patients with type 1 diabetes or end-phase type 2 diabetes but are generally considered not prominent in patients that are resistant to insulin. There is increasing support for the concept that C-peptide deficiency may play a role in the development of the long-term complications of insulin-requiring diabetic patients. Additionally, in vivo as well as in vitro studies in diabetic human models and in patients with type 1 diabetes demonstrate that C-peptide possesses hormonal activity (Wahren J et al.: American Journal of Physiology 278: E759-E768, (2000); Wahren J et al.: In International Textbook of Diabetes Mellitus Ferranninni E, Zimmet P, De Fronzo R A, Keen H, Eds. John Wiley & Sons, (2004), p. 165-182).

Further, WO 2014/108396 A1 relates to fasting levels of growth hormone as a predictive marker of cardiovascular risk. WO 2015/140317 A1 describes new markers for the assessment of the risk for development of a cardiovascular disorder. WO 2013/153177 A1 relates to the prognosis of adverse events in patients with suspected chronic heart failure. EP 2 626 704 A1 describes the diagnosis of pancreatic diabetes in patients with normal blood leucocyte counts using procalcitonin. US 2014/274891 A1 relates to a method of generating an index score for MBL deficiency to predict cardiodiabetes risk. Patel et al. (J Am Heart Assoc 1(6): e003152; 2012) suggest that fasting serum C-peptide levels predict cardiovascular and overall death in nondiabetic adults. Blaise et al. (Diabetes 62(11): 3807-3816; 2013) describe elastin-derived peptides as new regulators of insulin resistance development in mice. Skjøt-Arkil et al. (PLoS One 8(6): e60936; 2013) suggest that acute myocardial infarction and pulmonary diseases result in two different degradation profiles of elastin as quantified by two novel ELISAs. Blanchevoye et al. (J Biol Chem 288(2): 1317-1328; 2013) describe an interaction between the elastin peptide VGVAPG and human elastin binding protein. The Master Thesis from Matthew Vincent (Laboratory Medicine and Pathobiology, University of Toronto; 2014) suggests that ligands of the elastin receptor stimulate elastogenesis through cross-activation of the IGF-1 signaling pathway. Wenthur et al. (Annu Rev Pharmacol Toxicol 54: 165-184; 2014) review drugs for allosteric sites on receptors.

### The invention

The present invention is defined by the appended claims.

In a first embodiment the invention provides a method to earlier detect risks on vascular disease than currently is done. Excessive consumption of sugar, refined starch and fat, smoking cigarettes, processed meats, a lack of exercise, high-cholesterol levels, high-blood pressure, obesity, inflammation and diabetes; each are well known factors that contribute to the risk of vascular disease. Scientific research has validated many individual testable indicators of disease (biomarkers), each testing different aspects of vascular disease. Until now, however, no common factor has been identified that connects the many risk factors known and allows early detection of combined vascular risks. The invention provides a single common biomarker motif that allows detection of combined vascular risks in one single test. This biomarker motif predicts reduced vascular elasticity, increased vascular stiffness, atherosclerosis, ruptures of aorta and infarcts of heart and brain. This biomarker motif relates to sugar-, starch- and fat-rich diets, to smoking, smog, processed meats, obesity, inflammation and diabetes. Herewith the invention provides a diagnostic method or test for early detection of this common biomarker motif and timely diagnose vascular disease in humans and animals.

Surprisingly, the invention shows that the so-called inflammation in metabolic syndrome in most mammals (notably exclusions are pigs and naked mole rats) is augmented by a hitherto overlooked lock-and-key activation of the elastin receptor, a protein involved in vascular (blood vessel) and elastin repair, with the C-peptide, a small protein that is produced in a 1:1 ratio alongside with widely known insulin. The elastin receptor is the lock that is activated by a key motif of amino acids (PG-domain) found in most mammalian C-peptides and in breakdown products (PG-domain-fragments) thereof. Until now, no one has ever discovered this lock-and-key interaction between the two, now providing novel inroads in development of novel peptides and the use of peptides for diagnosis and treatment of metabolic syndrome, including type 1 and type 2 diabetes. The finding is exploiting the finding that not only the normal keys of the elastin receptor (elastin peptides), but also the C-peptide, a peptide we produce together with insulin every time glucose rises in our blood after a meal, interacts in a lock-and-key mode (docks) with the elastin receptor. Until now, scientific research has resulted in the identification and validation of various biomarkers that each test different aspects of vascular disease. However, among the many dietary and non-dietary risk factors, science has not yet identified one common factor to test for accumulated vascular risk that allows sufficient early detection of human vascular disease to start guided prevention and early treatment and significantly avoid currently high vascular morbidity and mortality. For example, the Dutch Heart Foundation adopted as its current ambition to earlier recognize cardiovascular diseases in subjects at risk. This would allow more effective treatment before these diseases become apparent, thus preventing irreparable damage and saving lives. In 2014, DHF together with scientists, patients and the general public set a research agenda based on urgency and impact. The theme 'Earlier recognition of cardiovascular diseases' came at the top of this agenda. Herein the invention provides in the identification of a key-biomarker motif that allows early detection of accumulated vascular risks in one test. The motif is found in several validated biomarkers of vascular disease that relate to aging, smoking, smog and processed meats, to lack of exercise, sugar- and fat-rich diets and diabetes, to obesity and inflammation, and to pregnancies and cardiac stress. Based on this common biomarker motif, the invention provides a blood test to timely diagnose vascular disease in humans and animals.

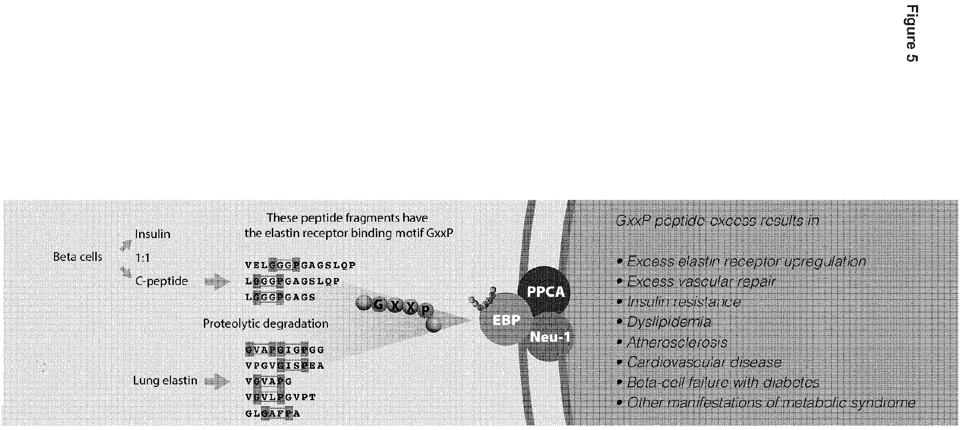

As explained further in this description, peptides derived from proteolytic breakdown of extracellular matrix (ECM) proteins, such as elastin peptides (EDP), having conserved sequence motifs (herein als called PG-domain) xGxxPG, xxGxPG, and GxxP (G being glycine, P proline, x any amino acid), bind to the elastin-receptor-complex (ERC). The ERC is a complex of elastin binding protein (EBP), neuraminidase-1 (NEU-1) and protective protein/cathepsin A (PPCA), three proteins which are present on the surface of leukocytes, fibroblasts, endothelial and smooth muscle cells. EBP is an RNA-spliced variant of lysosomal beta-galactosidase. Upon binding to ERC, peptides with PG-domain motif initiate proliferation of muscle cells and fibroblasts, pacification of inflammatory cells, elastin deposition, and vascular repair. When excessively activated, e.g. after elastin breakdown due to aging or to smoking, cells with ERC may give rise to the onset of vascular disease. The prototype EBP-binding motif SEQ ID NO: 41 (VGVAPG) is derived from elastin, a major component of the arterial wall, the skin, and the lung. Other proteins with EBP-binding motifs are fibrilin SEQ ID NO: 149 (EGFEPG), laminin SEQ ID NO: 137 (LGTIPG) and several collagens. Circulating elastin peptides with EBP-binding motifs are found in aged subjects and in those who smoke, and are associated with atherosclerosis, arterial stiffness, abdominal aortic aneurysms, and myocardial infarction in humans. Elastin peptides with EBP-binding motifs induce atherosclerosis and resistance to insulin in mice. Central to our disclosure, we have found that several blood-biomarkers of human vascular disease carry GxxP-motifs, including C-peptide, galectin-3, the prohormone cardiac stress marker procalcitonin and NT-proBNP, the obesity marker collagen 6A3, but also surrogate markers of vascular stress such as the innate immunity marker pyrin, pregnancy marker of angiogenesis beta-hCG and the neuroendocrine regulatory prohormone POMC. We provide herein that biomarkers with motif GxxP contribute to vascular disease through activation of the ERC. We thus conclude that the EBP-binding motif GxxP in C-peptide is both necessary and sufficient to elicit vascular bioactivity of C-peptide, indicating that C-peptide signals via the elastin receptor. This finding allows new and profound insights into the possible causes of vascular disease. At first sight, intake of sugar shares no causal factor with smoking, but this notion suggests the contrary. Both sugar and smoking may cause vascular disease by excess upregulation of vascular repair through the ERC, as illustrated below in figure 5.

Our finding provides a common etiology of ERC-mediated vascular disease wherein smoking (or smog) generates EBP-binding lung elastin peptide, while also diets high in glucose or starch keep EBP-binding C-peptide levels up. We also found other biomarkers of vascular disease to possess the EBP-binding motif, extending our perspective of a common ERC-mediated etiology of vascular disease. Typically, lack of exercise maintains high blood glucose, and saturated fats increase glucose-stimulated C-peptide excretion. Also, various industrially processed meats (machine de-boned and enzymatically treated slaughter remains, also called "pink slime") contain elastin fragments from tendons and ligaments, again aligning risk-factors through C-peptide with risk factors generating elastin peptides. In particular, the GxxP-motif found in pregnancy biomarker beta-hCG (and possibly the gender-effects found with endometrial expressed galectin-3) links our novel etiology to manifestations to vascular disease associated with pregnancies and monthly regeneration of uterine lining in women. We provide the insight that the EBP-binding motif shared by many biomarkers of vascular disease is a key-biomarker motif in peptides that modulate the repair system of our blood vessels. Overly high levels of such biomarkers from various sources predict high elastin fragmentation and excessive vascular repair, with atherosclerosis, reduced vascular elasticity, increased vascular stiffness, atherosclerosis, ruptures of aorta and infarcts of heart and brain. This finding is of immediate use in a diagnostic test for early diagnosis of vascular disease to determine earlier and with more precision our risks on (cardio- and cerebro-) vascular disease, as provided herein.

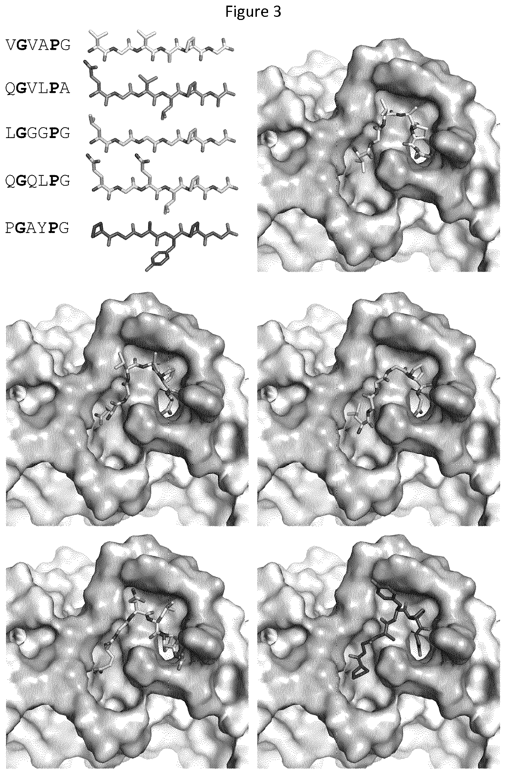

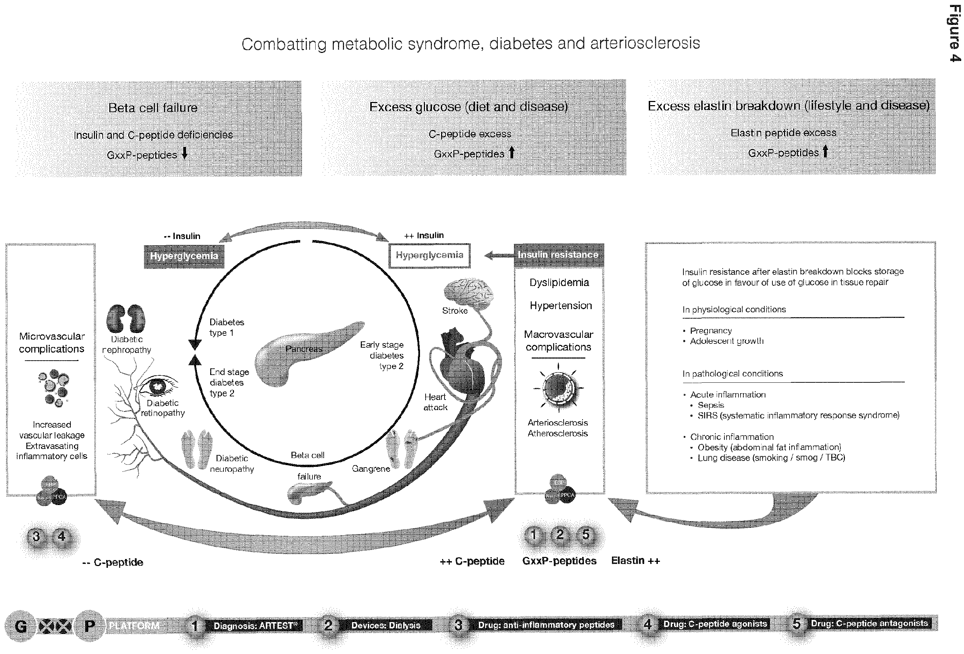

The invention provides a method for diagnosing vascular disease risk of a human, comprising testing a biological sample, preferably a blood or urine sample, of said human for the presence of at least two biomarkers each having a PG-domain different from each other and capable of binding (docking) to an elastin-binding-protein (EBP), as for example illustrated in figure 3, wherein said biomarkers are selected from the group of C-peptide, peptide fragments of C-peptide, elastin, peptide fragments of elastin, fibrillin, peptide fragments of fibrillin, laminin, peptide fragments of laminin, galectin-3, peptide fragments of galectin-3, POMC, peptide fragments of POMC, COL6A3, peptide fragments of COL6A3, pyrin or peptide fragments of pyrin, and wherein said PG-domain is an amino acid motif xGxxPG (G being glycine, P proline, x any amino acid). Preferred embodiemnts of the invention are provided wherein said at least two biomarker peptides have a PG-domain motif selected from the group of peptide motifs with SEQ ID NO: 149, SEQ ID NO: 137, SEQ ID NO: 34, SEQ ID NO: 44, SEQ ID NO: 41, SEQ ID NO: 201, SEQ ID NO: 202, and SEQ ID NO: 196.

The invention also provides a method for diagnosing vascular disease risk of a human, comprising testing a biological sample, preferably a blood or urine sample, of said human for the presence of at least three biomarker peptides each having a PG-domain different from each other and capable of binding (docking) to an elastin-binding-protein (EBP), as for example illustrated in figure 3 for biomarkers C-peptide, elastin and galectin 3, or provided herein for other sets of biomarkers such as other sets of three selected from biomarker peptides that have a PG-domain motif selected from the group of peptide motifs with SEQ ID NO: 34, SEQ ID NO: 44, and SEQ ID NO: 41,

The invention also provides a method for diagnosing vascular disease risk of a human, comprising testing a biological sample, preferably a blood or urine sample, of said human for the presence of at least four biomarker peptides each having a PG-domain different from each other and capable of binding (docking) to an elastin-binding-protein (EBP), as for example illustrated herein for sets of biomarkers

In one preferred embodiment, the invention provides testing said sample with a mass-spectrometer for the presence of at least two biomarker peptides that have a PG-domain motif

In another preferred embodiment, the invention provides testing said sample with a multiple antibody test, such as a commonly known multiplex antibody assay, the type of antibody-based test coomonly used in research to simultaneously measure multiple analytes in a single run/cycle of the assay, said antibodies each specifically directed against at each of least two, preferably three, more preferably four biomarker peptides have a PG-domain motif, such as listed above. It is preferred that said antibodies are specifically directed against at least two biomarker peptides selected from the group of peptides with motifs SEQ ID NO: 149, SEQ ID NO: 137, SEQ ID NO: 34, SEQ ID NO: 44, SEQ ID NO: 41, SEQ ID NO: 201, SEQ ID NO: 202, and SEQ ID NO: 196.

In another preferred embodiment, the invention provides testing said sample with a single-binding-molecule test, said single-binding-molecule specifically directed against at least two, preferably at least three, more preferably at least four biomarker peptides have a PG-domain motif. Preferably, said single-binding-molecule is specifically directed against at biomarker peptides selected from the group of peptides with motifs SEQ ID NO: 149, SEQ ID NO: 137, SEQ ID NO: 34, SEQ ID NO: 44, SEQ ID NO: 41, SEQ ID NO: 201, SEQ ID NO: 202, and SEQ ID NO: 196. Preferably, said single-binding-molecule is derived from the elastin-binding-protein. Preferably, said single-binding-molecule at least comprises a peptide sequence with motif SEQ ID NO: 31, more preferably said single-binding-molecule at least comprises a peptide sequence with motif SEQ ID NO: 131.

Also disclosed is a diagnostic kit for use in a method for diagnosing disease or assessing vascular disease risk of an animal, preferably a human, as provided herein. Such a diagnostic kit preferably comprise antibodies are specifically directed against at least two biomarker peptides selected from the group of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196. In another aspect, such a diagnostic kitcomrises a single-binding-molecule specifically directed against at least two, preferably at least three, more preferably at least four biomarker peptides have a PG-domain motif. It is herein provided that said single-binding-molecule in said kit is specifically directed against at biomarker peptides selected from the group of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196., in particular wherein said single-binding-molecule is derived from the elastin-binding-protein. Preferably, said single-binding-molecule in said ki at least comprises a peptide sequence with motif SEQ ID NO: 31, more preferably said single-binding-molecule at least comprises a peptide sequence with motif SEQ ID NO: 131. The disclosure also provides a single-binding-molecule (for example for use in said kit) labeled with biotin or streptavidin, or a fluor moiety or a horse-radish peroxidase enzyme, or another diagnostic label as known in the art, to allow detection with routine methods, such as in microscale thermophoresis or other methods known in (veterinary) medical diagnosis.

The disclosure also relates to the use of peptide agonists and/or peptide antagonists of C-peptide's interaction with the elastin receptor for veterinary treatment of non-human disease. Therewith the disclosure provides fields of use of peptides in veterinary medicine and in the field of experimentally testing drugs in experimental animals. In a first aspect, the disclosure disclosure provides a method for testing a candidate drug compound for its likelihood to modulate vascular disease risk in an animal, comprising testing said compound for its capacity to modulate binding of a peptide having a PG-domain motif in a single-binding-molecule test, said single-binding-molecule specifically directed against at least two biomarker peptides with a PG-domain motif. It is prefered that said single-binding-molecule is derived from the elastin-binding-protein, in particular wherein said single-binding-molecule at least comprises a peptide sequence with motif SEQ ID NO: 31, more in particular wherein said single-binding-molecule at least comprises a peptide sequence with motif SEQ ID NO: 131.

In a further aspect, the disclosure provides a method for testing a candidate drug compound for its likelihood to modulate vascular disease risk in an animal, comprising testing said compound for its capacity to modulate binding of a peptide having a PG-domain motif in a single-binding-molecule test, wherein said candidate drug compound comprises at least a functional PG-domain, allowing to test for candidate drug compounds having agonist activity on the elastin-binding protein. Such agonist drug compound, in particular agonist peptide compound, as provided herein is a useful peptide for use as a drug to counteract (control, reduce or treat) companion animals or experimental disease in experimental animals, in particular inflammatory disease or to any peptide component of a drug to counteract disease in companion animals or experimental disease in experimental animals such as inflammatory disease or to any peptide used in the preparation of a drug to counteract disease in companion animals or experimental disease in experimental animals in inflammatory disease (counteract in this descripton also generally indentified as treat or use in treatment), wherein the peptide has at least one elastin receptor binding motif GxxP, or its functionally equivalent xGxP, and wherein G represents the one-letter code for the amino acid glycine, P for the amino acid proline, and x for any amino acid. In some aspects, said peptide has at least one elastin receptor binding motif xGxxPG or xxGxPG. Such peptides are useful in the treatment of inflammatory conditions, such as acute kidney injury, also in acute systemic inflammatory conditions such as for example sepsis or systemic inflammatory response syndrome (SIRS), leading to vascular damage and often aggravated by (multiple organ) organ failure, or inflammatory conditions with diabetes, when given with an anti-diabetic composition such as insulin. It is preferred that such a peptide is selected from the group of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196, or is selected from the group of retro-inverso variants of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196, or from functional equivalents thereof.

The disclosure also provides a method for testing a candidate drug compound for its likelihood to modulate vascular disease risk in an animal wherein said vascular disease comprises type 1 diabetes or end-phase type 2 diabetes. Such a compound, herein also called C-peptide agonist, shall mean any agonist peptide for use as a medicine to treat disease in companion animals or experimental disease in experimental animals with microvascular complications or any agonist peptide component of a medicine to treat disease in companion animals or experimental disease in experimental animals with microvascular complications or any agonist peptide used in the preparation of a medicine to treat disease in companion animals or experimental disease in experimental animals with microvascular complications, wherein said peptide has at least one elastin receptor binding motif GxxP, or its functionally equivalent xGxP, wherein G stands for the amino acid glycine, P stands for the amino acid proline, and x stands for any amino acid, and is capable of combining with a elastin receptor on a cell and initiating the same physiological activity typically produced by the binding of C-peptide to the elastin receptor. Preferably said peptide has at least one elastin receptor binding motif xGxxPG or xxGxPG. Such peptides are useful in the treatment of inflammatory conditions, such as acute kidney injury, also in acute systemic inflammatory conditions such as for example sepsis or systemic inflammatory response syndrome (SIRS), leading to vascular damage and often aggravated by (multiple organ) organ failure, or inflammatory conditions with diabetes, when given with an anti-diabetic composition such as insulin. It is preferred that such a peptide is selected from the group of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196, or is selected from the group of retro-inverso variants of peptides with motifs SEQ ID NO: 149, SEQ ID NO: 137, SEQ ID NO: 34, SEQ ID NO: 44, SEQ ID NO: 45, SEQ ID NO: 41, SEQ ID NO: 200, SEQ ID NO: 186, SEQ ID NO: 201, SEQ ID NO: 202, and SEQ ID NO: 196, or from functional equivalents thereof.

In a further aspect, the disclosure provides a method for testing a candidate drug compound for its likelihood to modulate vascular disease risk in an animal, in particular wheierin wherein said vascular disease comprises manifestions of metabolic syndrome, such as atherosclerosis and new-onset type 2 diabetes, comprising testing said compound for its capacity to modulate binding of a peptide to binding of a peptide having a PG-domain motif in a single-binding-molecule test, wherein said candidate drug compound at least comprises a peptide sequence motif SEQ ID NO: 31 or SEQ ID NO: 131, allowing to test for candidate drug compounds having antagonist activity on the elastin-binding protein, In another preferred aspect said candidate drug compound comprises at least a retro-inverso variant of a peptide sequence motif SEQ ID NO: 31 or SEQ ID NO: 131.

The present application also provides an isolated or synthetic peptide for use in treatment of non-human disease, in particular in veterinary treatment of disease in companion animals or experimental disease in experimental animals, such as in treatment of non-human inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one elastin receptor binding motif GxxPG and has at least one amino acid Q, wherein G represents the one-letter code for the amino acid glycine, P for the amino acid proline, Q for the amino acid glutamine and x for any amino acid, the peptide consisting of 6-30 amino acids. Typically preferred peptides provided herein are selected from the group peptides listed under SEQ ID NOs: 10, 11, 12, 13, 17, 18, 19, 20, 14, 15, 16, 21, 25, 175, 3, 22, 23, 26, 176, 24, 27, 28, 29, 43, 93, and 94, and retro-inverso variant peptides derived from peptides listed under SEQ ID NOs: 10, 11, 12, 13, 17, 18, 19, 20, 14, 15, 16, 21, 25, 175, 3, 22, 23, 26, 176, 24, 27, 28, 29, 43, 93, and 94. Note: Retro-inverse peptides are composed of D-amino acids assembled in a reverse order from that of the parent L-sequence, thus maintaining the overall topology of the native sequence. No stereoisomers of glycine exist, here (and in retro-inverso peptides bearing for example retro-inverso GxxP or xGxP motifs) G is not, whereas other amino acids, such as L, P and A are, instrumental to the all-D-amino acid character of the retro-inverso peptide herein provided. The disclosure also provides synthetic peptides wherein the elastin receptor binding PG-domain motif has been repeated at least twice, preferably thrice, optionally said repeats are separated by a linker, such a linker may comprise one or more amino acids, such as one or more amino acids selected from the group of glycine, alanine, leucine, valine, isoleucine or glutamine. In a preferred aspect, the disclosure provides a peptide capable of combining with an elastin receptor on a cell and initiating the same physiological activity typically produced by the binding of C-peptide to the elastin receptor.

In a preferred aspect, the disclosure also provides a peptide capable of combining with a elastin receptor on a cell and initiating the same physiological activity typically produced by the binding of C-peptide to the elastin receptor. The disclosure also provides such an isolated or synthetic peptide for veterinary or experimental use in treatment of non-human disease, in particular in treatment of disease companion animals or experimental disease in experimental animals, such as in treatment of inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one elastin receptor binding motif GxxPG and has at least one amino acid Q, wherein G represents the one-letter code for the amino acid glycine, P for the amino acid proline, Q for the amino acid glutamine and x for any amino acid, the peptide consisting of 6-20 amino acids. Typically preferred peptides provided herein are selected from the group peptides listed under SEQ ID NOs: 14, 15, 16, 21, 25, 175, 3, 22, 23, 26, 176, 24, 27, 28, 29, 43, 93, and 94, and retro-inverso variant peptides derived from peptides listed under SEQ ID NOs: 14, 15, 16, 21, 25, 175, 3, 22, 23, 26, 176, 24, 27, 28, 29, 43, 93, and 94.

In a preferred aspect, the disclosure also provides a peptide capable of combining with anelastin receptor on a cell and initiating the same physiological activity typically produced by the binding of C-peptide to the elastin receptor. The disclosure also provides such an isolated or synthetic peptide for use in treatment of non-human disease, in particular in treatment of disease companion animals or experimental disease in experimental animals, such as in treatment of inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one elastin receptor binding motif GxxPG and has at least one amino acid Q, wherein G represents the one-letter code for the amino acid glycine, P for the amino acid proline, Q for the amino acid glutamine and x for any amino acid, the peptide consisting of 6-15 amino acids. Typically, preferred peptides provided herein are selected from the group peptides listed under SEQ ID NOs: 3, 22, 23, 26, 176, 24, 27, 28, 29, 43, 93, and 94, and retro-inverso variant peptides derived from peptides listed under SEQ ID NOs: 3, 22, 23, 26, 176, 24, 27, 28, 29, 43, 93, and 94.

In a preferred aspect, the disclosure also provides a peptide capable of combining with a elastin receptor on a cell and initiating the same physiological activity typically produced by the binding of C-peptide to the elastin receptor. The disclosure also provides such an isolated or synthetic peptide for use in treatment of non-human disease, in particular in treatment of disease in companion animals or experimental disease in experimental animals, such as in treatment of inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one elastin receptor binding motif GxxPG and has at least one amino acid Q, wherein G represents the one-letter code for the amino acid glycine, P for the amino acid proline, Q for the amino acid glutamine and x for any amino acid, the peptide consisting of 6-12 amino acids. Typically, preferred peptides provided herein are selected from the group peptides listed under SEQ ID NOs: 24, 27, 28, 29, 43, 93, and 94, and retro-inverso variant peptides derived from peptides listed under SEQ ID NOs: 24, 27, 28, 29, 43, 93, and 94.

In a preferred aspect, the disclosure also provides a peptide capable of combining with a elastin receptor on a cell and initiating the same physiological activity typically produced by the binding of C-peptide to the elastin receptor. The disclosure also provides such an isolated or synthetic peptide for use in treatment of non-human disease, in particular in treatment of disease in companion animals or experimental disease in experimental animals, such as in treatment of inflammation, and/or in treatment of type 1 diabetes and/or end-stage type 2 diabetes, more preferably use in treatment of micro-vascular complications, preferably as seen with type 1 diabetes and/or end-stage type 2 diabetes, wherein the peptide has at least one elastin receptor binding motif GxxPG and has at least one amino acid Q, wherein G represents the one-letter code for the amino acid glycine, P for the amino acid proline, Q for the amino acid glutamine and x for any amino acid, the peptide consisting of 6-9 amino acids. Typically, preferred peptides provided herein are selected from the group peptides listed under SEQ ID NOs: 29, 43, 93, and 94, and retro-inverso variant peptides derived from peptides listed under SEQ ID NOs: 29, 43, 93, and 94.

In another preferred aspect, the disclosure also provides a peptide capable of inhibiting (inhibits) the binding of C-peptide through C-peptide's motif GxxPG to the elastin receptor, in a more preferred aspect, the disclosure provides a peptide capable of reducing (reduces) the physiological activity of C-peptide. In particular the disclosure provides an isolated or synthetic peptide having at least the motif QDEA (SEQ ID NO: 31) for use in treatment of non-human disease, such as in treatment of non-human insulin resistance and/or treatment of non-human dyslipidemia, and/or non-human hypertension, and/or non-human macrovascular complications, preferably complications seen in arteriosclerosis, atherosclerosis, peripheral arterial disease and/or new-onset type 2 diabetes, wherein the peptide inhibits the binding of C-peptide to the elastin receptor and reduces the physiological activity of C-peptide, the peptide consisting of 4-40 amino acids. Typically, preferred peptides provided herein are selected from the group peptides listed under SEQ ID NOs: 99, 100, 101, 131, 102, 103, 104, 105, 31, and functional fragments or variants thereof and retro-inverso variant peptides derived from peptides listed under SEQ ID NOs: 99, 100, 101, 131, 102, 103, 104, 105, 31, and functional fragments or variants thereof. Functional fragments or variants are typically found in chemotaxis assay as provided herein testing the capacity of peptides to inhibit binding of C-peptide through C-peptide's motif GxxPG to the elastin receptor, such a peptide capable of reducing (reduces) chemotaxis activity of C-peptide.

In another aspect, the disclosure also provides a peptide capable of inhibiting (inhibits) the binding of C-peptide through C-peptide's motif GxxPG to the elastin receptor, in a more preferred aspect, the disclosure provides a peptide capable of reducing (reduces) the physiological activity of C-peptide. In particular the disclosure provides an isolated or synthetic peptide having at least the motif QDEA (SEQ ID NO: 31) for use in treatment of non-human disease, such as in treatment of non-human insulin resistance and/or treatment of non-human dyslipidemia, and/or non-human hypertension, and/or non-human macrovascular complications, preferably complications seen in arteriosclerosis, atherosclerosis, peripheral arterial disease and/or new-onset type 2 diabetes, wherein the peptide inhibits the binding of C-peptide to the elastin receptor and reduces the physiological activity of C-peptide, the peptide consisting of 4-20 amino acids. Typically, preferred peptides provided herein are selected from the group peptides listed under SEQ ID NOs: 131, 102, 103, 104, 105, 31, and functional fragments or variants thereof and retro-inverso variant peptides derived from peptides listed under SEQ ID NOs: 131, 102, 103, 104, 105, 31, and functional fragments or variants thereof.

In another aspect, the disclosure also also provides a peptide capable of inhibiting (inhibits) the binding of C-peptide through C-peptide's motif GxxPG to the elastin receptor, in a more preferred aspect, the disclosure provides a peptide capable of reducing (reduces) the physiological activity of C-peptide. In particular the disclosure provides an isolated or synthetic peptide having at least the motif QDEA (SEQ ID NO: 31) for use in treatment of non-human disease, such as in treatment of non-human insulin resistance and/or treatment of non-human dyslipidemia, and/or non-human hypertension, and/or non-human macrovascular complications, preferably complications seen in arteriosclerosis, atherosclerosis, peripheral arterial disease and/or new-onset type 2 diabetes, wherein the peptide inhibits the binding of C-peptide to the elastin receptor and reduces the physiological activity of C-peptide, the peptide consisting of 4-15 amino acids. Typically, preferred peptides provided herein are selected from the group peptides listed under SEQ ID NOs: 103, 104, 105, 31, and functional fragments or variants thereof and retro-inverso variant peptides derived from peptides listed under SEQ ID NOs: 103, 104, 105, 31, and functional fragments or variants thereof.

In another aspect, the disclosure also provides a peptide capable of inhibiting (inhibits) the binding of C-peptide through C-peptide's motif GxxPG to the elastin receptor, in a more preferred aspect, the disclosure provides a peptide capable of reducing (reduces) the physiological activity of C-peptide. In particular the disclosure provides an isolated or synthetic peptide having at least the motif QDEA (SEQ ID NO: 31) for use in treatment of non-human disease, such as in treatment of non-human insulin resistance and/or treatment of non-human dyslipidemia, and/or non-human hypertension, and/or non-human macrovascular complications, preferably complications seen in arteriosclerosis, atherosclerosis, peripheral arterial disease and/or new-onset type 2 diabetes, wherein the peptide inhibits the binding of C-peptide to the elastin receptor and reduces the physiological activity of C-peptide, the peptide consisting of 4-9 amino acids. Typically, preferred peptides provided herein are selected from the group peptides listed under SEQ ID NOs: 105, 31, and functional fragments or variants thereof and retro-inverso variant peptides derived from peptides listed under SEQ ID NOs: 105, 31, and functional fragments or variants thereof.

The invention shows that the so-called inflammation in metabolic syndrome is augmented by a hitherto overlooked lock- and-key activation of the elastin receptor, a protein involved in vascular (blood vessel) and elastin repair, with the C-peptide, a small protein that is produced in a 1:1 ratio alongside with widely known insulin. The elastin receptor is the lock that is activated by a key motif of amino acids (PG-domain) found in C-peptide and in breakdown products (PG-domain-fragments) thereof. Until now, no one has ever discovered this lock-and-key interaction between the two, now providing novel inroads in diagnosis, prevention and development of novel peptides and the use of peptides for treatment of metabolic syndrome, exploiting the finding that not only the normal keys of the elastin receptor (elastin peptides), but also the C-peptide, a peptide we produce together with insulin every time glucose rises in our blood after a meal, interacts in a lock-and-key mode (docks) with the elastin receptor. Herein a peptide or peptide fragment having a PG-domain is particulary defined as a peptide having at least one xGxP, GxxP, GxxPG or xGxPG motif, G being Glycine, P being Proline, x being any amino acid, the amino acid following P preferably allowing for a type VIII-beta turn, a condition which is met when P is C-terminally followed by a G.

The disclosure provides herewith a method for diagnosing disease or assessing disease risk comprising detecting the combined presence of C-peptide or fragments thereof and of elastin peptide or fragments thereof in a biological sample of an animal. The disclosure also provides a method for reducing disease, said method comprising removing fragments of C-peptide and/or fragments of elastin peptide from blood of an animal or human. The disclosure also provides a method for preventing or treating disease comprising providing a non-human animal with a peptide capable of agonising an elastin receptor. Such peptides as herein provided are preferably selected from the group of fragments of C-peptide and functional equivalents thereof.

In a first aspect, the disclosure provides a peptide for use in the treatment of non-human inflammation, preferably of a non-human subject, such as a companion animal in need thereof or an experimental animal to study (outcome of) disease, optionally in a non-human subject having been diagnosed as suffering from type 1 diabetes, most preferably when said subject is also treated with insulin, wherein said peptide is selected from the group of, preferably isolated and/or synthetic, preferably non-peggylated, C-peptide fragments 1-24 SEQ ID NO: 17 (EAEDLQVGQVELGGGPGAGSLQPL), 4-24 SEQ ID NO: 20 (DLQVGQVELGGGPGAGSLQPL), 7-24 SEQ ID NO: 175 (VGQVELGGGPGAGSLQPL), 11-24 SEQ ID NO: 176 (ELGGGPGAGSLQPL), 4-31 SEQ ID NO: 10 (DLQVGQVELGGGPGAGSLQPLALEGSLQ), 8-31 SEQ ID NO: 13 (GQVELGGGPGAGSLQPLALEGSLQ) and 12-31 SEQ ID NO: 14 (LGGGPGAGSLQPLALEGSLQ), as listed in figure 1 in this application, said peptide showing the GxxP motif SEQ ID NO: 38 (GGPG), and a significant normalization (%) of 30 mM glucose-induced vascular dysfunction in rats. Also, a peptide with motif SEQ ID NO: 38 (GGPG), functionally equivalent to a peptide listed in figure 1 herein, for use in the treatment of non-human inflammation, optionally in a non-human subject such as a companion animal in need thereof or an experimental animal to study (outcome of) disease, optionally having been diagnosed as suffering from type 1 diabetes, most preferably when said subject is also treated with insulin, most preferably when said subject is also treated with insulin, as provided herein is the synthetic and isolated peptide SEQ ID NO: 10 (DLQVGQVELGGGPGAGSLQPLALEGSLQ) as derivable from the C-peptide sequence. Also, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 11 (LQVGQVELGGGPGAGSLQPLALEGSLQ) as obtainable from the C-peptide sequence. Also, a peptide with motif SEQ ID NO: 38 (GGPG), functionally equivalent to a peptide listed in figure 1 herein, for use in the treatment of non-human inflammation, such as a companion animal in need thereof or an experimental animal to study( outcome) of disease, optionally in a non-human subject having been diagnosed as suffering from type 1 diabetes, most preferably when said subject is also treated with insulin, most preferably when said subject is also treated with insulin, as provided herein is the synthetic and isolated peptide SEQ ID NO: 12 (VGQVELGGGPGAGSLQPLALEGSLQ) as derivable from the C-peptide sequence. Also, a peptide with motif SEQ ID NO: 38 (GGPG), functionally equivalent to a peptide listed in figure 1 herein, for use in the treatment of non-human inflammation, in particularly in a non-human subject having been diagnosed as suffering from type 1 diabetes, most preferably when said subject is also treated with insulin, as provided herein is the synthetic and isolated peptide SEQ ID NO: 13 (GQVELGGGPGAGSLQPLALEGSLQ) as derivable from the C-peptide sequence. Also, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 14 (LGGGPGAGSLQPLALEGSLQ) as derivable from the C-peptide sequence. Also, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 15 (VGQVELGGGPGAGSLQPLAL) as derivable from the C-peptide sequence. Also, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 16 (EVGQVELGGGPGAGSLQPL) as derivable from the C-peptide sequence. In another aspect a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 17 (EAEDLQVGQVELGGGPGAGSLQPLAL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 18 (EAEDLQVGQVELGGGPGAGSLQPL) as derivable from the C-peptide sequence. In another aspect a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 19 (LQVGQVELGGGPGAGSLQPLAL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 20 (DLQVGQVELGGGPGAGSLQPL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 21 (LQVGQVELGGGPGAGSLQPL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 22 (LGGGPGAGSLQPL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 23 (VGQVELGGGPGAGSL) as derivable from the C-peptide sequence. In another aspect, a peptide with motif SEQ ID NO: 38 (GGPG), functionally equivalent to a peptide listed in figure 1 herein, for use in the treatment of non-human inflammation, optionally in a non-human subject having been diagnosed as suffering from type 1 diabetes, most preferably when said subject is also treated with insulin, as provided herein is the synthetic and isolated peptide SEQ ID NO: 24 (GGGPGAGSLQ) as derivable from the C-peptide sequence.

The disclosure also provides an isolated and/or synthetic, preferably non-peggylated, peptide identified herein as a regulatory model element peptide or fragment thereof that it is identified herein in specific regulatory elements modulating inflammation and tissue repair as provided herein. The disclosure provides an isolated and/or synthetic peptide wherein said regulatory model element peptide or fragment preferably carries a xGxxPG or xxGxPG motif and preferably can be derived for example from proteins identified in Table 3 herein. In a preferred aspect, the model element is recognized while it is flanked by at least one N-terminal and at least one C-terminal basic amino acid residue R (arginine) or K (lysine). Smaller fragments from within the element not carrying the two flanking basic residues are also usefull in modulating inflammation and tissue repair. In one aspect, a regulatory model element peptide fragment for use in the treatment of non-human inflammation as provided herein is the synthetic and isolated peptide SEQ ID NO: 25 (FRAAPLQGMLPGLLAPLRT) as derivable from the COL6A3 sequence (in Uniprot database COL6A3 is known under identifier P12111 and the SEQ ID NO: 25 (FRAAPLQGMLPGLLAPLRT) sequence is found in the sequence if isoform 1 from position 606-626). It is herein provided that said peptide is useful in the treatment of non-human inflammation and/or tissue repair, as are fragments thereof. In another aspect, a regulatory model element peptide fragment for use in the treatment of non-human inflammation and/or tissue repair as provided herein is the synthetic and isolated peptide SEQ ID NO: 26 (AAPLQGMLPGLLAPL) as derivable from the COL6A3 sequence. In another aspect, a regulatory model element peptide fragment for use in the treatment of non-human inflammation and/or tissue repair as provided herein is the synthetic and isolated peptide SEQ ID NO: 27 (LQGMLPGLLAPL) as derivable from the COL6A3 sequence. In another aspect, a regulatory model element peptide fragment for use in the treatment of non-human inflammation and/or tissue repair as provided herein is the synthetic and isolated peptide SEQ ID NO: 28 (LQGMLPGLLA) as derivable from the COL6A3 sequence. In another aspect, a regulatory model element peptide fragment for use in the treatment of non-human inflammation and/or tissue repair as provided herein is the synthetic and isolated peptide SEQ ID NO: 29 (LQGLMPG) as derivable from the COL6A3 sequence. In another aspect, a regulatory model element peptide fragment for use in the treatment of non-human inflammation and/or tissue repair as provided herein is the synthetic and isolated peptide SEQ ID NO: 30 (GMLPGLLA) as derivable from the COL6A3 sequence.

In another aspect, a regulatory model element peptide fragment for use in the treatment of non-human inflammation and/or tissue repair as provided herein is the synthetic and isolated peptide SEQ ID NO: 177 (CGNLSTCMLGTYTQDFNKFHTFPQTAIGVGAPG) as derivable from the procalcitonin sequence. In another aspect, a regulatory model element peptide fragment for use in the treatment of non-human inflammation and/or tissue repair as provided herein is the synthetic and isolated peptide SEQ ID NO: 178 (MLGTYTQDFNKFHTFPQTAIGVGAPG) as derivable from the procalcitonin sequence.

In another aspect, a regulatory model element peptide fragment for use in the treatment of non-human inflammation and/or tissue repair as provided herein is the synthetic and isolated peptide SEQ ID NO: 179 (FNKFHTFPQTAIGVGAPG) as derivable from the procalcitonin sequence. In another aspect, a regulatory model element peptide fragment for use in the treatment of non-human inflammation and/or tissue repair as provided herein is the synthetic and isolated peptide SEQ ID NO: 180 (FPQTAIGVGAPG) as derivable from the procalcitonin sequence. In another aspect, a regulatory model element peptide fragment for use in the treatment of non-human inflammation and/or tissue repair as provided herein is the synthetic and isolated peptide SEQ ID NO: 181 (AIGVGAPG) as derivable from the procalcitonin sequence. In another aspect, a regulatory model element peptide fragment for use in the treatment of non-human inflammation and/or tissue repair as provided herein is the synthetic and isolated peptide SEQ ID NO: 182 (SHPLGSPGSASDLETSGLQEQ) as derivable from the NTproBNP sequence. In another aspect, a regulatory model element peptide fragment for use in the treatment of non-human inflammation and/or tissue repair as provided herein is the synthetic and isolated peptide SEQ ID NO: 183 (PLGSPGSASDLETSGLQEQ) as derivable from the NTproBNP sequence. In another aspect, a regulatory model element peptide fragment for use in the treatment of non-human inflammation and/or tissue repair as provided herein is the synthetic and isolated peptide SEQ ID NO: 184 (PLGSPGSASDLETS) as derivable from the NTproBNP sequence. In another aspect, a regulatory model element peptide fragment for use in the treatment of non-human inflammation and/or tissue repair as provided herein is the synthetic and isolated peptide SEQ ID NO: 185 (PLGSPGSAS) as derivable from the NTproBNP sequence. In another aspect, a regulatory model element peptide fragment for use in the treatment of non-human inflammation and/or tissue repair as provided herein is the synthetic and isolated peptide SEQ ID NO: 186 (PLGSPG) as derivable from the NTproBNP sequence. In another aspect, a regulatory model element peptide fragment for use in the treatment of non-human inflammation and/or tissue repair as provided herein is the synthetic and isolated peptide SEQ ID NO: 187 (EDVSAGEDCGPLPEGGPEPRSDGAKPGPREG) as derivable from the POMC sequence. In another aspect, a regulatory model element peptide fragment for use in the treatment of non-human inflammation and/or tissue repair as provided herein is the synthetic and isolated peptide SEQ ID NO: 188 (GEDCGPLPEGGPEPRSDGAKPGPREG) as derivable from the POMC sequence. In another aspect, a regulatory model element peptide fragment for use in the treatment of non-human inflammation and/or tissue repair as provided herein is the synthetic and isolated peptide SEQ ID NO: 189 (PLPEGGPEPRSDGAKPGPREG) as derivable from the POMC sequence. In another aspect, a regulatory model element peptide fragment for use in the treatment of non-human inflammation and/or tissue repair as provided herein is the synthetic and isolated peptide SEQ ID NO: 190 (PLPEGGPEPRSDGAKPG) as derivable from the POMC sequence. In another aspect, a regulatory model element peptide fragment for use in the treatment of non-human inflammation and/or tissue repair as provided herein is the synthetic and isolated peptide SEQ ID NO: 191 (SDGAKPG) as derivable from the POMC sequence. In another aspect, a regulatory model element peptide fragment for use in the treatment of non-human inflammation and/or tissue repair as provided herein is the synthetic and isolated peptide SEQ ID NO: 192 (RRNASSAGRLQGLAGGAPGQKECR) as derivable from the pyrin sequence. It is herein provided that said peptide is useful in the treatment of non-human inflammation and/or tissue repair, as are fragments thereof. In another aspect, a regulatory model element peptide fragment for use in the treatment of non-human inflammation and/or tissue repair as provided herein is the synthetic and isolated peptide SEQ ID NO: 193 (RLQGLAGGAPGQKECR) as derivable from the pyrin sequence. In another aspect a regulatory model element peptide fragment for use in the treatment of non-human inflammation and/or tissue repair as provided herein is the synthetic and isolated peptide SEQ ID NO: 194 (RRNASSAGRLQGLAGGAPGQ) as derivable from the pyrin (marenostrin) sequence.

In another aspect, a regulatory model element peptide fragment for use in the treatment of non-human inflammation and/or tissue repair as provided herein is the synthetic and isolated peptide SEQ ID NO: 195 (LQGLAGGAPGQ) as derivable from the pyrin sequence. In another aspect, a regulatory model element peptide fragment for use in the treatment of non-human inflammation and/or tissue repair as provided herein is the synthetic and isolated peptide SEQ ID NO: 196 (AGGAPG) as derivable from the pyrin sequence. The disclosure also provides a pharmaceutical composition comprising a peptide consisting of 4-30 amino acids, preferably of 4-20, more preferably of 4-15, more preferably 4-12, most preferably of 4-9 amino acids, the peptide comprising at least one PG-domain, preferably with a xGxP or GxxP, GxxPG or xGxPG motif.

The disclosure also provides a method for preventing or treating disease comprising providing a non-human, such as a companion animal or an experimental animal, with a peptide capable of antagonising, blocking, inhibiting or preventing of binding of fragments of C-peptide and/or of elastin peptide to an elastin receptor. The disclosure also provides a method wherein said peptide or fragments comprise a binding site allowing binding to an elastin receptor. The disclosure also provides a method wherein said binding site comprises an amino acid sequence motif GxxP, allowing a type VIII beta-turn. The disclosure also provides a method wherein said binding site comprises an amino acid sequence motif xGxPG or GxxPG. The disclosure also provides means for diagnosing disease or assessing disease risk allowing detecting the combined presence of fragments of C-peptide and of elastin peptide in a biological sample of an animal. The disclosure also provides means wherein said peptide fragments comprise a binding site allowing binding to an elastin receptor. The disclosure also provides means wherein said binding site comprises an amino acid sequence motif xGxP or GxxP, said P allowing a type VIII beta-turn. The disclosure also provides means wherein said binding site comprises an amino acid sequence motif GxxPG. The disclosure also provides means for reducing disease allowing removing fragments of C-peptide and of elastin peptide from blood of an animal. The disclosure also provides means wherein said peptide fragments comprise a binding site allowing binding to an elastin receptor. The disclosure also provides means wherein said binding site comprises an amino acid sequence motif xGxP or GxxP, the ammino acid located C-terminally following said P allowing a type VIII beta-turn. The disclosure also provides means wherein said binding site comprises an amino acid sequence motif xGxPG or GxxPG. The disclosure also provides a method for detecting a test compound for preventing or treating disease in an animal, said method comprising testing said compound for its capacity to block, inhibit or prevent binding of C-peptide or of fragments thereof to an elastin receptor. The disclosure also provides a method for detecting a test compound for preventing or treating disease in an animal, said method comprising testing said compound for its capacity to modulate binding of C-peptide or of fragments thereof to an elastin receptor.

The disclosure also provides a method wherein said peptide fragments comprise a binding site allowing binding to an elastin receptor. The disclosure also provides a method wherein said binding site comprises an amino acid sequence motif xGxP or GxxP, allowing a type VIII beta-turn.

The disclosure also provides a method wherein said binding site comprises an amino acid sequence motif xGxPG or GxxPG. The disclosure also provides a use of method according to the disclosure for developing a pharmaceutical composition for preventing or treating disease in a non-human.

The disclosure also provides a pharmaceutical composition obtainable with a method according to the disclosure. The disclosure also provides a pharmaceutical composition comprising an oligopeptide consisting of 4-30 amino acids, preferably of 4-20, more preferably of 4-15, more preferably 4-12, most preferably of 4-9 amino acids, the peptide comprising at least one PG-domain, preferably with a xGxP or GxxP, GxxPG or xGxPG motif.

The disclosure also provides a composition wherein said motif allows said peptide to modulate binding of C-peptide to an elastin receptor. The disclosure also provides a pharmaceutical composition comprising a peptide consisting of 4-30 amino acids, preferably of 4-20, more preferably of 4-15, more preferably 4-12, most preferably of 4-9 amino acids, the peptide comprising the motif SEQ ID NO: 31 (QDEA). The disclosure also provides method for preventing or treating disease of a non-human comprising providing said non-human with a peptide or composition as provided herein. The disclosure provides a method for diagnosing disease or assessing disease risk, such as risk on cardiovascular disease, atheromatous disease or arterioscleoris or atherosclerosis herein also collectively called arterial risk, of an animal comprising detecting the combined presence of C-peptide or fragments of C-peptide and of elastin peptide or fragments of elastin peptide in a biological sample of said animal. It is preferred that said presence is detetected by detecting peptides having common denominator of C-peptide and elastin peptide, said denominator preferably being PG-domain, preferably having a peptide amino acid sequence xGxP or GxxP, preferably xPxPG or GxxPG, in a biological sample of said animal. In a method for diagnosing disease or assessing disease risk of an animal as provided herein, it is prefered that said animal is a mammal, such as a sheep, cow, horse, pig, cat, dog, primate, rat, fat sand rat, naked mole rate or mouse, it is more preferred that said mammal is non-human. It is most preferred that said peptide or fragments comprise an amino acid motif allowing binding to an elastin receptor. The disclosure also provides an arterial risk test ifor the measurement of peptides having a PG-domain, preferably a peptide having a xGxP or GxxP-peptide domain or motif in serum, plasma and urine.

The disclosure also provides a method for preventing or treating disease of an animal, said method comprising removing C-peptide, fragment of C-peptide, elastin peptide or fragment of elastin peptide from blood in the cardiovascular system of said animal, for example by dialysis or proteolysis. In an aspect of a method for preventing or treating disease of an animal, it is prefered that said animal is a mammal, such as a sheep, cow, horse, pig, cat, dog, primate, rat, fat sand rat, naked mole rate or mouse, it is more preferred that said mammal is non-human. It is most preferred that said peptide or fragments comprise a PG-domain amino acid motif allowing binding to an elastin receptor.

The disclosure also provides method for preventing or treating disease of an animal comprising providing said animal with a compound capable of antagonising, blocking, inhibiting or preventing of binding of fragments of C-peptide and/or of elastin peptide to an elastin receptor of said animal. In an aspect of a method for preventing or treating disease of an animal, it is prefered that said animal is a mammal, such as a sheep, cow, horse, pig, cat, dog, primate, rat, fat sand rat, naked mole rate or mouse. It is most preferred that said peptide or fragments comprise an amino acid motif allowing binding to an elastin receptor.

The disclosure also provides a method for preventing or treating disease of an animal comprising providing said animal with a peptide capable of agonising an elastin receptor of said animal. In an aspect of a method for preventing or treating disease of an animal, it is prefered that said animal is a mammal, such as a sheep, cow, horse, pig, cat, dog, primate, rat, fat sand rat, naked mole rate or mouse. It is most preferred that said peptide or fragments comprise an amino acid motif allowing binding to an elastin receptor.

The disclosure also provides means for diagnosing disease or assessing disease risk allowing detecting the combined presence of fragments of C-peptide and of elastin peptide in a biological sample of an animal. With said means for diagnosing disease or assessing disease risk of an animal as provided herein, it is prefered that said animal is a mammal, such as a sheep, cow, horse, pig, cat, dog, primate, rat, fat sand rat, naked mole rate or mouse, it is more preferred that said mammal is non-human. It is most preferred that said peptide or fragments comprise an amino acid motif allowing binding to an elastin receptor.

The disclosure also provides means and a method for detecting a compound suitable for preventing or treating disease in an animal, said method comprising testing said compound for its capacity to bind, modulate, block, inhibit or prevent binding of C-peptide or of fragments thereof to an elastin receptor. The disclosure also provides use of means or a method for detecting a compound suitable for preventing or treating disease in an animal for developing a composition, preferably a pharmaceutical composition or medicament, for preventing or treating disease in an animal, it is prefered that said animal is a mammal, such as a sheep, cow, horse, pig, cat, dog, primate, rat, fat sand rat, naked mole rate or mouse, it is more preferred that said mammal is non-human. It is most preferred that said peptide or fragments comprise an amino acid motif allowing binding to an elastin receptor.

The disclosure also provides a composition, preferably a pharmaceutical composition or medicament, comprising an oligopeptide consisting of 4-30 amino acids, preferably of 4-20, more preferably of 4-15, more preferably 4-12, most preferably of 4-9 amino acids, the peptide comprising at least one xGxP, GxxP, GxxPG or xGxPG motif, said motif preferably allowing said peptide to modulate binding of C-peptide to an elastin receptor. In a preferred aspect, said composition is prepeared for the the treatment of diabetes, preferably for the treatment or prevention of microvascular disorders seen with diabetes wherein the endogenous C-peptide level is low, such as with type 1 diabetes or with end-stage type 2 diabetes.

The disclosure also provides use of an peptide consisting of 4-30 amino acids, preferably of 4-20, more preferably of 4-15, more preferably 4-12, most preferably of 4-9 amino acids, the peptide comprising at least one xGxP, GxxP, GxxPG or xGxPG motif, said motif preferably allowing said peptide to modulate binding of C-peptide to an elastin receptor, for the production of a medicament, preferably of a medicament for the treatment or prevention of microvascular disorders seen with diabetes wherein the endogenous C-peptide level is low, such as with type 1 diabetes or with end-stage type 2 diabetes.

The disclosure also provides a composition, preferably a pharmaceutical composition or medicament, comprising an peptide consisting of 4-30 amino acids, preferably of 4-20, more preferably of 4-15, more preferably 4-12, most preferably of 4-9 amino acids, the peptide comprising the motif SEQ ID NO: 31 (QDEA), preferably said motif allowing said peptide to modulate binding of C-peptide to an elastin receptor, preferably for the treatment or prevention of conditions of metabolic syndrome as defined herein, preferably for the treatment or prevention of cardiovascular disease or macrovascular disease or atheromatous disease such as atherosclerosis or arteriosclerosis.

The disclosure also provides use of an peptide consisting of 4-30 amino acids, preferably of 4-20, more preferably of 4-15, more preferably 4-12, most preferably of 4-9 amino acids, the peptide comprising the motif SEQ ID NO: 31 (QDEA), preferably said motif allowing said peptide to modulate binding of C-peptide to an elastin receptor, for the production of a medicament, preferably of a medicament for the treatment or prevention of conditions of metabolic syndrome as defined herein, preferably for the treatment or prevention of cardiovascular disease or macrovascular disease or atheromatous disease such as atherosclerosis or arteriosclerosis.

The invention shows that the so-called inflammation in metabolic syndrome is augmented by an hitherto overlooked lock- and-key activation of the elastin receptor, a protein involved in vascular (blood vessel) elastin repair, with the C-peptide, a small protein that is produced in a 1:1 ratio alongside with widely known insulin. The elastin receptor is the lock that is activated by a key motif of amino acids (GxxP) found in C-peptide and in breakdown products (GxxP-fragments) thereof. Until now, no one has ever discovered this lock-and-key interaction between the two, now providing novel inroads in diagnosis, prevention and development of novel compounds for treatment of metabolic syndrome, exploiting the finding that not only the normal keys of the elastin receptor (elastin peptides), but also the C-peptide, a peptide we produce together with insulin every time glucose rises in our blood after a meal, interacts in a lock-and-key mode with the elastin receptor. In summary, the invention provides the insight that excess food intake directly switches C-peptide on as the key unlocking metabolic syndrome. Everyday overeating results in everyday increased C-peptide levels (and GxxP motif containing break-down fragments thereof) in the blood. As the elastin receptor is mainly found on cells that produce elastin and on cells that repair our blood vessels (together called vascular cells), everyday GxxP lock-and-key activation of the elastin receptor by excess C-peptide and its fragments results in everyday blood vessel damage done. As 30% of the walls of our blood-vessels are made up of elastin and inflammatory cells continuously repair damage done to blood vessels, disturbing elastin repair and provoking inflammation of blood vessels cannot remain without consequences. Indeed, continued elastin receptor activation by C-peptide and GxxP fragments leaves a state of vascular overrepair, hitherto called inflammation, and otherwise called atherosclerosis, a condition characterized by thickening of blood vessel walls with activated inflammatory and blood vessel cells that underlies all conditions of metabolic syndrome. Over years, and in trickling fashion that varies from person to person, more-and-more damage is done to the elasticity and strength of our vasculature of various organs (such as heart, blood vessels, pancreas, kidney, brain) that generally leads to atherosclerosis, hypertension and dyslipidaemia, and ultimately leads to various manifestations as cardiovascular disease, diabetes type 2, chronic kidney failure and vascular dementias.

The finding explains how every day excess food intake results in overrepair of blood vessels, also called chronic inflammation. In short: every time we consume food with glucose (sugar) we produce insulin and thus C-peptide, every time we eat too much glucose, we produce more-and-more insulin, and thus more-and-more (excess) C-peptide. It is this excess C-peptide and the fragments thereof that still carry the key unlocking motif GxxP that cause excess elastin receptor activation, leading to vascular overrepair with inflammation. Overeating every day directly causes excess production of C-peptide and its GxxP-fragments that every day adds up to elastin receptor induced overrepair, leading up to the chronic overrepair and so-called inflammation, the dislipidaemia, hypertension and ultimately unhealthy blood vessels seen in metabolic syndrome. With this insight, the finding provided roads to develop and use products (diagnostic tests) to measure GxxP-containing C-peptide fragments to (early) detect disease, to use these diagnostic test results to develop personalized dietary preventive strategies to avoid build up or cause reduction of the level of GxxP-fragments in our blood, and roads to develop and use products (drug compounds) to block GxxP lock-and-key interaction to prevent disease among which hypertension and atherosclerosis. Also, the invention explains the added risks of a sedentary live. In short: not using our unhealthy intake of sugary food as fuel for our muscles urges our bodies to produce more-and-more insulin to help the liver to change the excess sugar into fat that can comes back in the blood leaving us with dislipidaemia. Again, excess C-peptide is produced along with insulin, again setting the lock-and-key elastin receptor activation in motion, leading up to the deregulation of fat metabolism as described. Thirdly, the invention explains the added risks of smoking as well. In short: smoking (or similarly: air pollution) causes damage to the elastic tissue of the lung, thereby releasing fragments of elastin having the GxxP motif (herein called elastin peptides) which are known to cause elastin receptor activation. Thus, smoking adds more peptides with the GxxP motif to the already circulating C- peptide fragments with that motif. This accumulation of diet-induced C-peptide and smoking induced elastin peptide ads up to aggravated overrepair and so-called inflammation and thus aggravated cardiovascular or chronic kidney disease in those people that both smoke and indulge in too much sugar from their diet. The invention is showing an as yet fully unknown common causal relationship between diseases caused by different lifestyle conditions, overeating, being sedentary and smoking. The disclosure provides diagnostic tests to measure GxxP-containing peptide fragments to detect and prevent disease, to develop improved dialysis devices to remove these fragments from the blood of patients suffering from chronic kidney disease and to develop drugs to block GxxP lock- and key interaction to prevent disease. Both C-peptide and elastin peptides, and their breakdown products or fragments are relatively stable in blood and urine. Where insulin is rapidly degraded in the liver and disappears from the blood, C-peptide (and breakdown fragments with the GxxP motif) as well as elastin peptides have a much longer life in the blood and are only excreted by the kidney. Thus, whether eating-on and producing new insulin with new C-peptide or continuing smoking and producing more elastin decay of the lungs; levels of C-peptide and elastin peptides build up and over time cause more and more vascular damage via GxxP-mediated activation of the elastin receptor. GxxP lock-and key interaction of both C-peptide and elastin peptides may be blocked with appropriate peptides to prevent disease depending on the outcome of these diagnostic tests as provided herein. In a further aspect, the disclosure provides a GxxP-receptor screening method (herein als called platform) to detect binding of GxxP fragments with the elastin receptor. With this screening platform (e.g. the C-peptide and the elastin receptor put together in a reaction vessel such as a test tube, a well of an ELISA plate or another testing device) the disclosure provides a diagnostic test to cumulatively detect GxxP fragments, optionally followed by developing software algorithms to allow personalized prevention via devices such as smartphones using the outcome of those tests. With the technology provided with the GxxP-receptor screening platform the disclosure also provides methods and devices that may remove circulating GxxP fragments by dialyses from blood of patients suffering from chronic kidney failure. Also, the disclosure provides methods using the GxxP-receptor screening platform to identify drug candidates (test compounds) and to develop drugs that stimulate (agonists) or inhibit (antagonists) GxxP-receptor binding. In one embodiment the invention provides a method for diagnosing vascular disease risk, preferably wherein said disease comprises or is cardiovascular disease or wherein said disease comprises or is atherosclerosis or of arteriosclerosis, the method comprising detecting the combined presence of C-peptide or C-peptide fragments and of elastin-derived peptide or elastin peptides in a biological sample, such as tissue, blood, or plasma, or urine or sputum, of an animal. Increased circulating C-peptide (fragments) reflects increased level of diet-induced disease or risk thereon, in particular cardiovascular disease or disease risk, circulating elastin peptide degradation products or elastin peptides reflect increased level of elastin-breakdown-induced disease or risk thereon. It is provided herein that cumulating test results for C-peptide or C-peptide fragment detection with test results for elastin degradation product testing provides improved assessment of disease or disease risk, in particular of cardiovascular disease or disease risk, in particular of atherosclerosis or of arteriosclerosis. Such detecting method is provided for example via combined testing of a single sample of an animal with established detection methods for C-peptide and for elastin degradation products (such as desmosine, isodesmosin, or elastin derived peptides) as known in the art and then combining the results. The final outcome with which disease or disease risk is assessed is a cumulation of both test results. Alternatively, the same animal is sampled more often at the same occasion, and said consecutive samples are each tested with a different test, respectively, after which results are combined. It is provided herein that cumulating test results for C-peptide or C-peptide fragment detection with testresults for elastin peptide testing provides improves assessment of disease or disease risk, in particular of cardiovascular disease or disease risk. Suitable immunoassay methods that now in the light of the invention may be combined for C-peptide and elastin degradation product detection are for example discussed in Little RR, et al., . Clin Chem. 2008 Jun;54(6):1023-6; Wiedmeyer HM, et al., Clin Chem. 2007 Apr;53(4):784-7; Fülöp T Jr, et al., Clin Physiol Biochem. 1990;8(6):273-82; Osakabe T, et al., Biol Pharm Bull. 1999 Aug;22(8):854-7. In another embodiment, C-peptide and elastin degradation products are detected using liquid chromatography coupled to mass spectrometry, in particular by using high-performance liquid chromatography/electrospray tandem mass spectrometry (LC/MSMS). Suitable mass spectrometry methods are for example discussed in Kinumi T, Mizuno R, Takatsu A. J Chromatogr B Analyt Technol Biomed Life Sci. 2014 Mar 15;953-954:138-42; He J, Turino GM, Lin YY. Exp Lung Res. 2010 Nov;36(9):548-57.; Slowik N, Ma S, He J, Lin YY, Soldin OP, Robbins RA, Turino GM. Chest. 2011 Oct;140(4):946-53.

In a preferred aspect the disclosure provides a method for diagnosing disease or assessing disease risk, preferably cardiovascular disease or disease risk, comprising detecting the combined presence of GxxP-fragments of C-peptide and of elastin-derived peptide in a biological sample, such as tissue, blood, or plasma, or urine or sputum, of an animal. Thus, specifically GxxP-peptide fragments are detected. In a more preferred aspect the disclosure provides a method for diagnosing disease or assessing disease risk, preferably cardiovascular disease or disease risk, comprising detecting the combined presence of fragments of C-peptide and of elastin-derived peptide in a biological sample, such as tissue, blood, or plasma, or urine or sputum, of an animal, wherein specifically peptide fragments are detected that are capable of binding to an elastin receptor, preferably through a GxxP or xGxP motif. In this aspect, detection is aimed at combined detection of C-peptide or elastin peptide fragments that carry a motif GxxP or xGxP, for human samples, detection is thus one the one hand directed at detecting C-peptide derived fragments bearing the elastin-receptor binding SEQ ID NO: 38 (GGGP) or SEQ ID NO: 34 (GGGPG) motif found (as provided herein below) in C-peptide and on the other hand also directed at detecting peptide fragments bearing or having for example the sequence SEQ ID NO: 217 (GVPGLGVGAGVPGLGV) or SEQ ID NO: 218 (GAGVPG )or or or SEQ ID NO: 219 (GISPE) or SEQ ID NO: (220) LQGVLPAL or SEQ ID NO: 221 (GVLPA) or SEQ ID NO: 222 (PGLGVGVGVP )or SEQ ID NO: 41 (VGVAPG) or SEQ ID NO: 53 (GVAPG) or SEQ ID NO: 60 (PGAIPG) or SEQ ID NO: 223 (GVGVGVPG) or SEQ ID NO: 224 (GVGVPG) or SEQ ID NO: 225 (GLVPGGP) or SEQ ID NO: 58 (GFGPG) or SEQ ID NO: 226 (PGFPPG) or SEQ ID NO: 149 (EGFEPG) or SEQ ID NO: 227 (EKGPDP) or SEQ ID NO: 96 (GAYPG) or SEQ ID NO: 57 (GVYPG) other elastin-receptor-binding peptides derivable from elastin (Uniprot P15502), beta-hCG (Choriogonadotropin subunit beta variants such as Uniprot A6NKQ9) or galectin-3 (Uniprot Q08380) or from other proteins such as herein listed below.

The disclosure also provides a method of treating or preventing metabolic syndrome or insulin resistance or hypertension or atherosclerosis or dyslipidaemia or diabetes type-2 or a related metabolic disorder in an animal suffering therefrom or in need thereof, the method comprising: administering to the animal an antagonist of the C-peptide/elastin binding protein interaction. In one aspect, said antagonist is an antibody, preferably wherein the antibody is an antibody that binds specifically to C-peptide or a fragment thereof and/or wherein the antibody is an antibody that binds specifically to elastin binding protein. It is preferred that said antibody is a humanized antibody. In a further aspect said method further comprises administering insulin in the absence of C-peptide to the animal. In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: administering to the animal an antagonist of the C-peptide/elastin binding protein interaction. In a further aspect said method further comprises administering insulin in the absence of C-peptide to the animal. In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: administering to the animal an antagonist of the C-peptide/elastin binding protein interaction, the method additionally comprising administering to the animal an antagonist to alpha-enolase. In a further aspect said method further comprises administering insulin in the absence of C-peptide to the animal. In yet another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: administering to the animal an antagonist of the C-peptide/elastin binding protein interaction, the method additionally comprising administering to the animal an antagonist to GPR146. In a further aspect said method further comprises administering insulin in the absence of C-peptide to the animal.

In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: administering to the animal an inhibitor of a prohormone converstase, preferably wherein the prohormone convertase is prohormone covertase 1 and/ or prohormone convertase 2 or preferably wherein the inhibitor is selected from the group consisting of Chlorpyrifos, L-alanyl-L-lysyl-L-arginylmethyldimethylsulphonium, and L-alanyl-L-arginyl-L-arginylmethyldimethylsulphonium. In a further aspect said method further comprises administering insulin in the absence of C-peptide to the animal.

In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: administering to the animal a protease capable of cleaving at the elastin receptor binding motif of C-peptide, preferably wherein the protease is A2pro. In a further aspect said method further comprises administering insulin in the absence of C-peptide to the animal.

In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: removing C-peptide and/or elastin peptidefrom circulation in the animal, preferably wherein the C-peptide and/or ealstin peptide is removed from circulation using dialysis. In a further aspect said method further comprises administering insulin to the animal.

In another aspect, the disclosure provides a method of treating or preventing metabolic syndrome or insulin resistance or hypertension or atherosclerosis or diabetes type-2 or metabolic disorder in an animal suffering therefrom or in need thereof, the method comprising: cleaving C-peptide at the elastin receptor binding motif during dialysis. In a further aspect said method further comprises administering insulin in the absence of C-peptide to the animal.

The disclosure also provides use of an isolated fragment of a C-peptide as an agent that modulates binding or interaction of a C-peptide with an elastin receptor, and/or use of an isolated fragment of an elastin receptor as an agent that modulates binding or interaction of a C-peptide with an elastin receptor. The disclosure also provides a method for producing a pharmaceutical composition for preventing and/or treating an inflammatory disease, preferably type-1 diabetes, the method comprising the steps of: providing at least one peptide consisting of 4-30 amino acids, preferably of 4-20, more preferably of 4-15, more preferably 4-12, most preferably of 4-9 amino acids, the peptide comprising at least one xGxP, GxxP, GxxPG or xGxPG motif (said motif herein als called a PG-domain, G being Glycine, P being Proline), or providing a retro-inverso variant peptide comprising at least one xGxP, GxxP, GxxPG or xGxPG motif, the peptide capable of interacting with an elastin receptor type, and formulating the at least one peptide provided in step a) or a pharmaceutically acceptable salt thereof in a pharmaceutical composition together with at least one anti-diabetic agent such as insulin, and the disclosure provides a composition obtainable or produced by said the method according to the disclosure. Examples of such peptide (fragments or variants) comprise peptides such as, peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), or retro-inverso variant peptide of SEQ ID NO: 32 (LGGGPGAG) or a fragment thereof, or of SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP), as further discussed below. The disclosure also provides a pharmaceutical composition comprising an anti-diabetic agent, and an peptide consisting of 4-30 amino acids, preferably of 4-20, more preferably of 4-15, more preferably 4-12, most preferably of 4-9 amino acids, the peptide comprising at least one xGxP, GxxP, GxxPG or xGxPG motif (G being Glycine, P being Proline), or providing a retro-inverso variant peptide comprising at least one xGxP, GxxP, GxxPG or xGxPG motif, the peptide capable of interacting with an elastin receptor type, and a pharmaceutically acceptable carrier. The disclosure also provides a method producing a pharmaceutical composition for preventing and/or treating an inflammatory disease, preferably a chronic inflammatory disease, comprising the steps of: providing at least one peptide consisting of 4-30 amino acids, preferably of 4-20, more preferably of 4-15, more preferably 4-12, most preferably of 4-9 amino acids, the peptide comprising at least one xGxP, GxxP, GxxPG or xGxPG motif (G being Glycine, P being Proline), or providing a retro-inverso variant peptide consisting of 4-30 amino acids, preferably of 4-20, more preferably of 4-15, more preferably 4-12, most preferably of 4-9 amino acids, comprising at least one xGxP, GxxP, GxxPG or xGxPG motif, the peptide capable of interacting with an elastin receptor type, and formulating the at least one peptide provided or a pharmaceutically acceptable salt thereof in a pharmaceutical composition together with at least one interleukin-1 receptor antagonist, preferably wherein the interleukin 1 receptor antagonist (IL-1Ra) is a recombinant protein (rIL-1Ra), preferably a recombinant human protein (rhIL-1Ra), preferably anakinra. The disclosure also provides a composition obtainable or produced by the method according to the disclosure. The disclosure for example provides a pharmaceutical composition comprising an interleukin-1 receptor antagonist, at least one peptide consisting of 4-30 amino acids as provided herein, the peptide comprising at least one xGxP, GxxP, GxxPG or xGxPG motif (G being Glycine, P being Proline), or a retro-inverso variant peptide comprising at least one xGxP, GxxP, GxxPG or xGxPG motif, the peptide capable of interacting with an elastin receptor type, and a pharmaceutically acceptable carrier.

The disclosure als provides a peptide derived from a fragment of mammalian insulin C-peptide for use in therapy, preferably for use in the treatment of diabetes and/or diabetic complications, or for reducing inflammatory activity. It is preferred that said peptide or fragment is from two (2) to nine (9) amino acids in length, more preferably three (3) to six (6) amino acids in length, most preferably from four (4) tot five (5) amino acids in length. The disclosure als provides a peptide derived from a fragment of mammalian insulin C-peptide, the peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide having the ability to interact with elastin receptor type binding or modulate inflammatory activity of innate immune cells. It is preferred that said peptide or fragment is from two (2) to nine (9) amino acids in length, more preferably three (3) to six (6) amino acids in length, most preferably from four (4) tot five (5) amino acids in length. The disclosure als provides an isolated or synthetic peptide, essentially being homologous to a fragment of mammalian insulin C-peptide, the peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide having the ability to interact with elastin receptor type binding or modulate inflammatory activity of innate immune cells. It is preferred that said peptide or fragment is from two (2) to nine (9) amino acids in length, more preferably three (3) to six (6) amino acids in length, most preferably from four (4) tot five (5) amino acids in length.

The disclosure also provides retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP). It is preferred that said retro-inverso variant peptide is from two (2) to nine (9) amino acids in length, more preferably three (3) to six (6) amino acids in length, most preferably from four (4) tot five (5) amino acids in length.

The disclosure also provides a pharmaceutical composition comprising at least one peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells together with at least one pharmaceutically acceptable carrier or excipient. It is preferred that said peptide or fragment is from two (2) to nine (9) amino acids in length, more preferably three (3) to six (6) amino acids in length, most preferably from four (4) tot five (5) amino acids in length. In a preferred aspect, said pharmaceutical composition is further comprising at least one additional active agent effective to combat diabetes, diabetic complications, or to treat an inflammatory condition, such as insulin or metformin, and/or or wherein the additional active agent is an interleukin-1 receptor antagonist or an antibody directed against an interleukin-1, preferably directed against interleukin-1beta, or an agonist of alpha-enolase or an agonist of GPR146.

The disclosure also provides a pharmaceutical composition comprising at least one retro-inverso variant peptide of SEQ ID NO: 32 (LGGGPGAG) or a fragment thereof, or of SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells together with at least one pharmaceutically acceptable carrier or excipient. It is preferred that said peptide or fragment is from two (2) to nine (9) amino acids in length, more preferably three (3) to six (6) amino acids in length, most preferably from four (4) tot five (5) amino acids in length. In a preferred aspect, said pharmaceutical composition is further comprising at least one additional active agent effective to combat diabetes, diabetic complications, or to treat an inflammatory condition, such as insulin or metformin, and/or or wherein the additional active agent is an interleukin-1 receptor antagonist or an antibody directed against an interleukin-1, preferably directed against interleukin-1beta, or an agonist of alpha-enolase or an agonist of GPR146.

The disclosure also provides use of at least one peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), or of a retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP), said peptide or fragment or variant having the ability to interact with elastin receptor type binding or modulate inflammatory activity of innate immune cells, for treating diabetes, diabetic complications, or for reducing inflammatory activity or for preparing a medicament for treating diabetes and diabetic complications or for reducing inflammatory activity, said use preferably further comprising the use of insulin or an interleukin-1 receptor antagonist or an antibody directed against an interleukin-1, preferably directed against interleukin-1beta. In a preferred aspect, said medicament is utilized for treating type-1 diabetes, optionally with nephropathy, neuropathy or retinopathy or for retarding the development of late type-2 diabetic complications or said medicament is utilized for treating an inflammatory condition.

The disclosure also provides a product containing at least one peptide SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells, or a product containing a retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP), together with at least one additional active agent effective to combat diabetes or diabetic complications as a combined preparation for simultaneous, separate or sequential use in the treatment diabetes and/or diabetic complications. In a further aspect, said product is provided with at least one additional active agent effective to treat microvascular disease. Such a product as provided herein may be used for treating nephropathy or for preparing a medicament for treating nephropathy, or for treating neuropathy or for preparing a medicament for treating neuropathy, or for treating retinopathy or for preparing a medicament for treating retinopathy. Such a product may additionally be used or provided with an agent for glycemic control, preferably insulin or an antidiabetic agent functionally equivalent to insulin, preferably wherein the antidiabetic agent comprises regular insulin or an insulin analogue such as insulin lispro, insulin glulisine, insulin aspart, insulin degludec, insulin glargine, or wherein the antidiabetic agent comprises a sulfonylurea or a meglitinide or metformin.

The disclosure also provides use of a peptide, such as v14 peptide or derivatives therof for the production of a medicament or use of that peptide for preventing or treating disease in a non-human animal, said peptide capable of blocking, inhibiting or preventing of binding of fragments of C-peptide and of elastin peptide to an elastin receptor. In a preferred aspect, the disclosure provides such a peptide or use therof for preventing or treating disease in a non-human, said peptide capable of blocking, inhibiting or preventing of binding of fragments of C-peptide to an elastin receptor.

The disclosure also provides a method of identifying a candidate modulator or test compound as an agent that modulates binding or interaction of a C-peptide with an elastin receptor, said method comprising: providing a protenacoeus substance according the disclosure comprising a first, preferably isolated or synthetic, peptide fragment of a C-peptide or a retro-inverso variant therof and a second, preferably isolated or synthetic, fragment of an elastin receptor, in the presence and absence of said candidate modulator under conditions permitting binding of said first fragment with said second fragment, measuring binding of said first fragment to said second fragment, wherein a decrease or increase in binding in the presence of said candidate modulator, relative to binding in the absence of said candidate modulator, identifies said candidate modulator as an agent that modulates binding or interaction of a C-peptide with an elastin receptor. It is preferred that said first fragment and/or said second fragment is detectably labeled, preferably with a moiety selected from the group consisting of a radioisotope, a fluorophore, a quencher of fluorescence, an enzyme, and an affinity tag.. In a further aspect, the disclosure provides a method wherein said substance comprising a first, preferably isolated or synthetic, peptide fragment of a C-peptide or a retro-inverso variant therof and a second, preferably isolated or synthetic, fragment of an elastin receptor, comprises a cell expressing said first fragment and/or said second fragment. In yet another aspect, said first and/or said second fragment are in solution.

The disclosure also provides method of detecting, in a sample, the presence of an agent or test compound that modulates binding or interaction of a C-peptide with an elastin receptor: providing a proteinaceous substance comprising a first, preferably isolated or synthetic, peptide fragment of a C-peptide or a retro-inverso variant therof and a second, preferably isolated or synthetic, fragment of an elastin receptor, as provided herein above in the presence and absence of said sample under conditions permitting binding of said first fragment with said second fragment, measuring binding of said first fragment to said second fragment, wherein a decrease or increase in binding in the presence of said sample, relative to binding in the absence of said sample, identifies said sample as comprising an agent that modulates binding or interaction of a C-peptide with an elastin receptor. It is preferred that said first fragment and/or said second fragment is detectably labeled preferably with a moiety selected from the group consisting of a radioisotope, a fluorophore, a quencher of fluorescence, an enzyme, and an affinity tag. In a further aspect, the disclosure provides a method wherein said substance comprises a cell expressing said first fragment and/or said second fragment. In yet another aspect, said first and/or said second fragment are in solution.

The disclosure also provides a non-human animal, such as a laboratory animal, preferably a mouse, or a rat or a fat sand rat or a naked mole rat, provided with a gene construct allowing overexpression of a C-peptide. The disclosure also provides a non-human animal, such as a laboratory animal, preferably a mouse, or a rat or a fat sand rat or a naked mole rat, provided with a gene construct allowing expression of a C-peptide with a modified GxxP motif, preferably wherein the codon for P has been replaced with a codon for another amino acid, such as an L (leucine).

The disclosure also provides means to detect a collection of peptides or fragments thereof, in a sample, said peptides or fragments each comprising at least one xPxG, PxxG, GPxxG or GPxGx motif, said sample preferably a sample from a vertebrate, preferably from a primate, most preferably from a human, preferably wherein said sample is a serum or a plasma or a urine sample, more preferably means are provided to detect at least one peptide comprising a SEQ ID NO: 41 (VGVAPG) sequence and at least one peptide comprising a SEQ ID NO: 34 (GGGPG) sequence. Such an aspect of means as provided by the disclosure is preferably used to to determine a risk on developing or having insulin resistance or hypertension or atherosclerosis or diabetes type-2 or metabolic disorder or microvascular disease or macrovascular disease. Most preferred is a diagnostic test as provided herein comprising such means.

The disclosure provides a method for diagnosing disease or assessing disease risk comprising detecting the combined presence of fragments of C-peptide (preferably fragments comprising a SEQ ID NO: 34 (GGGPG) sequence) and of elastin peptide (preferably fragments comprising a SEQ ID NO: 41 (VGVAPG) sequence) in a biological sample of an animal. The disclosure also provides a method for reducing disease comprising removing, preferably by dialysis, or by proteolytic cleavage, or with a binding substance such as an antibody, a fragment of C-peptide (preferably a fragment comprising a SEQ ID NO: 34 (GGGPG) sequence) and/or of elastin peptide (preferably a fragment comprising a SEQ ID NO: 41 (VGVAPG) sequence) from blood of an animal. The disclosure also provides a method for preventing or treating disease comprising providing an animal with a compound capable of blocking, inhibiting or preventing of binding of fragments of C-peptide (preferably fragments comprising a SEQ ID NO: 40 (GGGPG) sequence) and of elastin peptide (preferably fragments comprising a SEQ ID NO: 41 (VGVAPG) sequence) to an elastin receptor, preferably wherein wherein said peptide fragments comprise a binding site allowing binding to an elastin receptor, preferably wherein said binding site comprises an amino acid sequence motif GxxP, allowing a type VIII beta-turn, preferably wherein said site comprises an amino acid sequence motif GxxPG.

In a preferred aspect, the disclosure provides means for diagnosing disease or assessing disease risk allowing detecting the combined presence of fragments of C-peptide and of elastin peptide in a biological sample of an animal, preferably wherein said peptide fragments comprise a binding site allowing binding to an elastin receptor, more preferably wherein said binding site comprises an amino acid sequence motif GxxP, allowing a type VIII beta-turn, most preferably wherein said binding site comprises an amino acid sequence motif GxxPG. In a preferred aspect, the disclosure provides a method for diagnosing disease or assessing disease risk comprising detecting the combined presence of fragments of C-peptide, preferably fragments comprising a SEQ ID NO: 34 (GGGPG) sequence, and of elastin peptide, preferably fragments comprising a SEQ ID NO: 41 (VGVAPG) sequence, in a biological sample of an animal. The disclosure also provides a method for reducing disease comprising removing, preferably by dialysis, or by proteolytic cleavage, or with a binding substance such as an antibody, a fragment of C-peptide (preferably a fragment comprising a SEQ ID NO: 34 (GGGPG) sequence) and/or of elastin peptide (preferably a fragment comprising a SEQ ID NO: 41 (VGVAPG) sequence) from blood of an animal. The disclosure also provides a method for preventing or treating disease comprising providing an animal with a compound capable of blocking, inhibiting or preventing of binding of a fragment of C-peptide (preferably a fragment comprising a SEQ ID NO: 34 (GGGPG) sequence) and of elastin peptide (preferably a fragment comprising a SEQ ID NO: 41 (VGVAPG) sequence) to an elastin receptor, preferably wherein wherein said peptide fragments comprise a binding site allowing binding to an elastin receptor, preferably wherein said binding site comprises an amino acid sequence motif GxxP, allowing a type VIII beta-turn, preferably wherein said site comprises an amino acid sequence motif GxxPG. It is preferred that said compound capable of blocking, inhibiting or preventing of binding of a fragment of C-peptide is an antibody, preferably a humanised antibody.

In a preferred aspect, the disclosure provides means for diagnosing disease or assessing disease risk allowing detecting the combined presence of fragments of C-peptide and of elastin peptide in a biological sample of an animal, preferably wherein said peptide fragments comprise a binding site allowing binding to an elastin receptor, more preferably wherein said binding site comprises an amino acid sequence motif GxxP, allowing a type VIII beta-turn, most preferably wherein said binding site comprises an amino acid sequence motif GxxPG.

The disclosure also provides a method of treating or preventing metabolic syndrome or insulin resistance or hypertension or atherosclerosis or dyslipidaemia or diabetes type-2 or a related metabolic disorder in a non-human animal suffering therefrom or in need thereof, the method comprising: administering to the non-human animal an antagonist of the C-peptide/elastin binding protein interaction. In a further aspect said method further comprises administering insulin in the absence of C-peptide to the non-human animal. In another aspect, the disclosure provides a method of treating or preventing metabolic syndrome or insulin resistance or hypertension or atherosclerosis or diabetes type-2 or metabolic disorder in a non-human animal suffering therefrom or in need thereof, the method comprising: administering to the non-human animal an antagonist of the C-peptide/elastin binding protein interaction, the method additionally comprising administering to the non-human animal an antagonist to alpha-enolase. In a further aspect said method further comprises administering insulin in the absence of C-peptide to the non-human animal. In yet another aspect, the disclosure provides a method of treating or preventing metabolic syndrome or insulin resistance or hypertension or atherosclerosis or diabetes type-2 or metabolic disorder in a non-human animal suffering therefrom or in need thereof, the method comprising: administering to the non-human animal an antagonist of the C-peptide/elastin binding protein interaction, the method additionally comprising administering to the non-human animal an antagonist to GPR146. In a further aspect said method further comprises administering insulin in the absence of C-peptide to the non-human animal.

The disclosure also provides use in a non-human animal of an isolated fragment of a C-peptide as an agent that modulates binding or interaction of a C-peptide with an elastin receptor, and/or use of an isolated fragment of an elastin receptor as an agent that modulates binding or interaction of a C-peptide with an elastin receptor. Examples of such peptide (fragments or variants) comprise peptides such as, peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), or retro-inverso variant peptide of SEQ ID NO: 32 (LGGGPGAG) or a fragment thereof, or of SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP), as further discussed below.

The disclosure als provides a peptide derived from a fragment of mammalian insulin C-peptide for use in non-human animal therapy, preferably for use in the treatment of non-human animal diabetes and/or non-human animal diabetic complications, or for reducing inflammatory activity. It is preferred that said peptide or fragment is from two (2) to nine (9) amino acids in length, more preferably three (3) to six (6) amino acids in length, most preferably from four (4) tot five (5) amino acids in length. The disclosure als provides a peptide derived from a fragment of mammalian insulin C-peptide, the peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide having the ability to interact with elastin receptor type binding or modulate inflammatory activity of innate immune cells. It is preferred that said peptide or fragment is from two (2) to nine (9) amino acids in length, more preferably three (3) to six (6) amino acids in length, most preferably from four (4) tot five (5) amino acids in length. The disclosure als provides an isolated or synthetic peptide, essentially being homologous to a fragment of mammalian insulin C-peptide, the peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide having the ability to interact with elastin receptor type binding or modulate inflammatory activity of innate immune cells. It is preferred that said peptide or fragment is from two (2) to nine (9) amino acids in length, more preferably three (3) to six (6) amino acids in length, most preferably from four (4) tot five (5) amino acids in length.

The disclosure also provides retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP). It is preferred that said retro-inverso variant peptide is from two (2) to nine (9) amino acids in length, more preferably three (3) to six (6) amino acids in length, most preferably from four (4) tot five (5) amino acids in length.

The disclosure also provides use in non-human animal of a peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells together with at least one pharmaceutically acceptable carrier or excipient. It is preferred that said peptide or fragment is from two (2) to nine (9) amino acids in length, more preferably three (3) to six (6) amino acids in length, most preferably from four (4) tot five (5) amino acids in length. In a preferred aspect, said peptide is combined with at least one additional active agent effective to combat diabetes, diabetic complications, or to treat an inflammatory condition, such as insulin or metformin, and/or or wherein the additional active agent is an interleukin-1 receptor antagonist or an antibody directed against an interleukin-1, preferably directed against interleukin-1-beta, or an agonist of alpha-enolase or an agonist of GPR146.

The disclosure also provides use in non-human animal of a retro-inverso variant peptide of SEQ ID NO: 32 (LGGGPGAG) or a fragment thereof, or of SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells together with at least one pharmaceutically acceptable carrier or excipient. It is preferred that said peptide or fragment is from two (2) to nine (9) amino acids in length, more preferably three (3) to six (6) amino acids in length, most preferably from four (4) tot five (5) amino acids in length. In a preferred aspect, said peptide is combined with at least one additional active agent effective to combat diabetes, diabetic complications, or to treat an inflammatory condition, such as insulin or metformin, and/or or wherein the additional active agent is an interleukin-1 receptor antagonist or an antibody directed against an interleukin-1, preferably directed against interleukin-1-beta, or an agonist of alpha-enolase or an agonist of GPR146.

The disclosure also provides use of at least one peptide comprising SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), or of a retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP), said peptide or fragment or variant having the ability to interact with elastin receptor type binding or modulate inflammatory activity of innate immune cells, for treating diabetes, diabetic complications, or for reducing inflammatory activity or for preparing a medicament for treating diabetes and diabetic complications or for reducing inflammatory activity, said use preferably further comprising the use of insulin or an interleukin-1 receptor antagonist or an antibody directed against an interleukin-1, preferably directed against interleukin-1-beta. In a preferred aspect, said medicament is utilized for treating type-1 diabetes, optionally with nephropathy, neuropathy or retinopathy or for retarding the development of late type-2 diabetic complications or said medicament is utilized for treating an inflammatory condition.

The disclosure also provides a product containing at least one peptide SEQ ID NO: 32 (LGGGPGAG) or a fragment thereof, or SEQ ID NO: 33 (LAGGPGAG) or a fragment thereof, or having SEQ ID NO: 34 (LGGGPG) or SEQ ID NO: 35 (LAGGPG) or a fragment thereof, preferably wherein the peptide or fragment is selected from the group consisting of SEQ ID NO: 36 (LGGGP), SEQ ID NO: 37 (LAGGP), SEQ ID NO: 38 (GGGP), and SEQ ID NO: 39 (GAGP), said peptide or fragment having the ability to interact with elastin receptor type binding or modulate inflammatory activity of innate immune cells, or a product containing a retro-inverso variants of SEQ ID NO: 32 (LGGGPGAG) or a fragment thereof, or SEQ ID NO: 33 (LAGGPGAG) or a fragment thereof, or of SEQ ID NO: 34 (LGGGPG) or SEQ ID NO: 35 (LAGGPG) or a fragment thereof, preferably wherein the peptide or fragment is selected from the group consisting of retro-inverso variants of SEQ ID NO: 36 (LGGGP), SEQ ID NO: 37 (LAGGP), SEQ ID NO: 38 (GGGP) or SEQ ID NO: 39 (GAGP), together with at least one additional active agent effective to combat diabetes or diabetic complications as a combined preparation for simultaneous, separate or sequential use in the treatment diabetes and/or diabetic complications. In a further aspect, said product is provided with at least one additional active agent effective to treat microvascular disease. Such a product as provided herein may be used for treating nephropathy or for preparing a medicament for treating nephropathy, or for treating neuropathy or for preparing a medicament for treating neuropathy, or for treating retinopathy or for preparing a medicament for treating retinopathy. Such a product may additionally be used or provided with an agent for glycemic control, preferably insulin or an antidiabetic agent functionally equivalent to insulin, preferably wherein the antidiabetic agent comprises regular insulin or an insulin analogue such as insulin lispro, insulin glulisine, insulin aspart, insulin degludec, insulin glargine, or wherein the antidiabetic agent comprises a sulfonylurea or a meglitinide or metformin.

The disclosure also provides use of a peptide, such as v14 peptide (SEQ ID NO: 131)or derivatives therof for the production of a medicament, or use of that peptide for preventing or treating disease in a non-human animal, said peptide capable of blocking, inhibiting or preventing of binding of fragments of C-peptide and of elastin peptide to an elastin receptor. In a preferred aspect, the disclosure provides such a peptide or use therof for preventing or treating disease in a non-human animal, said peptide capable of blocking, inhibiting or preventing of binding of fragments of C-peptide to an elastin receptor.

The disclosure also provides a method for preventing or treating disease comprising providing a non-human animal with a peptide capable of blocking, inhibiting or preventing of binding of fragments of C-peptide (preferably fragments comprising a SEQ ID NO: 40 (GGGPG) sequence) and of elastin peptide (preferably fragments comprising a SEQ ID NO: 41 (VGVAPG) sequence) to an elastin receptor, preferably wherein wherein said peptide fragments comprise a binding site allowing binding to an elastin receptor, preferably wherein said binding site comprises an amino acid sequence motif GxxP, allowing a type VIII beta-turn, preferably wherein said site comprises an amino acid sequence motif GxxPG.

Synthetic peptides with amino acid sequence motif GxxP, allowing a type VIII beta-turn, preferably wherein said site comprises an amino acid sequence motif GxxPG, as provided herein are useful in the treatment of inflammatory conditions, such as acute kidney injury, also in acute systemic inflammatory conditions such as for example sepsis or systemic inflammatory response syndrome (SIRS), leading to vascular damage and often aggravated by (multiple organ) organ failure, or inflammatory conditions with diabetes, when given with an anti-diabetic composition such as insulin. In a further aspect of the disclosure, peptides with amino acid sequence motif GxxP, allowing a type VIII beta-turn, preferably wherein said site comprises an amino acid sequence motif GxxPG, as provided herein are encapsulated in an acid resistant capsule. Such (pharmaceutical) capsules are widely used in the pharmaceutical field as oral dosage forms for administration to non-human animals. Filled with a peptide according to the disclosure, such a capsule is useful for the enteral administration of a synthetic peptide provided with at least one, preferably two or three pentapeptide motifs GxxPG or xGxPG (G being glycine, P being proline, and x any amino acid), preferably wherein at least one amino acid at one position x is selected from the group of glycine, alanine, leucine, valine or isoleucine, said peptide also provided with at least one glutamine. Such administration would alleviate or treat diseases such as Crohn's disease in which gut endothelial cells need regeneration. Also, such administration would be useful in treating gastro-intestinal damage obtained after excess radiation. The peptides with amino acid sequence motif GxxP, allowing a type VIII beta-turn, preferably wherein said site comprises an amino acid sequence motif GxxPG, as provided herein may also be advantageously combined with other therapeutic immunomodulators, such as with immunomodulatory peptides, such as peptides with SEQ ID NO: 1 (LQGV), AQG, or SEQ ID NO: 2 (AQGV), or with other immunomodulators, such as with immunomodulatory antibodies or proteins directed against cytokines as TNF-alpha, IL-1 or IL-6.

### Figure legends

### Figure 1

Ido et al. (Science, 1997 Jul 25;277(5325):563-6) show C-peptide's midportion SEQ ID NO: 8 (GGGPGAG) to normalize glucose-induced vascular dysfunction. However, finding reverse (retro) and all-D-amino acid (enantio) C-peptides equipotent to native C-peptide, they conclude the activity of SEQ ID NO: 8 (GGGPGAG) to be not mediated by a receptor, thereby teaching away from a receptor for C-peptide. This specification shows that the opposite is a case. All peptides with the midportion motif GxxP (SEQ ID NO: 38 (GGGP) in human and rat C-peptide, SEQ ID NO: 39 (GAGP) in reverse C-peptide and stereochemically equivalent PGAG in D-form C-peptide) normalize vascular dysfunction while none of the peptides without that motif do. Thus, the EBP-binding motif GxxP in SEQ ID NO: 8 (GGGPGAG) is both necessary and sufficient to normalize vascular dysfunction. Hence, C-peptide can be considered a ligand of the EBP that modulates vascular repair via the ERC. Efficacy is expressed as an average percent of the effect of 100 nM C-peptide. Significantly different for 30 mM glucose: *P < 0.05. GxxP motifs in peptides bind to the elastin receptor when allowing for a close to a type VIII beta-turn confirmation, a condition considered always to be met by the motif xGxxPG. All peptides having the GxxP motif (SEQ ID NO: 38 (GGGP) or SEQ ID NO: 39 (GAGP), or all-D PGAG which is stereometrically equivalent to all-L-SEQ ID NO: 39 (GAGP)) show significant normalization of vascular dysfunction while none of the peptides without the motif show significant effects, illustrating that the elastin receptor binding motif GxxP is both necessary and sufficient to elicit the biological activity of C-peptide. Figure adapted from Ido Y, et al. Prevention of vascular and neural dysfunction in diabetic rats by C-peptide. Science 1997; 277: 563-66.

### Figure 2

The elastin receptor complex (EBP/Neu-1/PPCA), is commonly found on vascular repair cells (leucocytes, smooth muscle cells, fibroblasts and endothelial cells) and activated by breakdown peptide fragments carrying a common motif GxxP. Summarized description of pathological vascular effects of GxxP-peptide deficiency versus GxxP-excess.

### Figure 3

In-silico studies show docking of GxxP-peptides from biomarkers elastin, hCG, C-peptide and galectin-3 in a composite model of EBP, demonstrating the principle of the blood test wherein peptides with multiple heterogeneous EBP-binding motifs off different source proteins act as common diagnostic biomarker for early detecting vascular disease.

Various GxxP hexa-peptides were docked in the peptide-binding site of the elastin binding protein (EBP) using Vina/Autodock and PyMOL (1, 2, 3). The binding conformation of each peptide was chosen from the top 20 best scoring poses. A homology model of EBP (4) was used as receptor in the docking procedure. Peptides tested were:
SEQ ID NO: 41 (VGVAPG) (prototype GxxP-peptide ligand of EBP (4))
SEQ ID NO: 34 (LGGGPG) (selected from C-peptide (5))
SEQ ID NO: 43 (QGQLPG) (immunomodulatory peptide provided herein)
SEQ ID NO: 44 (PGAYPG) (selected from Galectin-3 (6))
SEQ ID NO: 45 (QGVLPA) (selected from loop 2 of beta-hCG (7))

References: 1 Trott, O & Olson, AJ (2010) AutoDock Vina: improving the speed and accuracy of docking with a new scoring function, efficient optimization and multithreading, J Comp Chem 31: 455-461.

2 Seeliger, D & de Groot, BL (2010) Ligand docking and binding site analysis with PyMOL and Autodock/Vina. J Comput-Aided Mol Des 24 :417-422.

3 www.pymol.org

4 Blanchevoye, C et al. (2013) Interaction between the elastin peptide SEQ ID NO: 41 (VGVAPG) and human elastin binding protein, J Biol Chem 288:1317-28.

5 Ido, Y et al. (1997) Prevention of vascular and neural dysfunction in diabetic rats by C-peptide Science 277:563-6

6 De Boer, R et al. (2011) Plasma Galectin-3 Is Associated with Near-Term Rehospitalization in Heart Failure: A Meta-Analysis Journal of Cardiac Failure Vol 17, Issue 8, S93

7 Khan, NA et al. (2010) Mitigation of septic shock in mice and rhesus monkeys by human chorionic gonadotrophin-related oligopeptides, Clin Exp Immunol 160:466-478

Similarly, All-D-amino acid peptide GPGAG fits in the model of EBP designed for docking prototype elastin peptide SEQ ID NO: 41 (VGVAPG) as well. Also, L-amino acid peptides SEQ ID NO: 40 (GGGPG) and SEQ ID NO: 46 (GAGPG) fit the model as well. EBP-associated bioactivity is considered to depend on whether the GXXP-peptide can adapt to a type VIII beta-turn confirmation at the proline (P).

### Figure 4

Overview of pathophysiological pathways in metabolic syndrome, atherosclerosis and diabetes

### Figure 5

A common etiology of vascular disease. Both dietary sugar as well as smoking may cause vascular disease through excess activation of ERC. Intake of sugar increases blood glucose. That activates pancreatic beta cells to excrete C-peptide into the blood. Degradation of C-peptide generates peptides with the EBP-binding motif. Smoking degrades lung elastin, which releases EBP-binding peptides in the blood. Both may result in excess EBP-binding peptides, that excessively upregulate vascular repair. In mice, chronic dosing with EBP-binding elastin peptides was shown to promote atherosclerosis, dyslipidemia and insulin resistance, hallmarks of risks on coronary heart disease (CHD). Thus, finding a vascular bioactive EBP-binding motif central in C-peptide, acutely links increased circulating C-peptide levels to ERC-mediated vascular disease.

This new perspective sheds new light on diseases that are associated with atherosclerosis and insulin resistence (e.g. cardiovascular disease, stroke, peripheral arterial disease, dementia, chronic kidney disease and beta cell failure leading to diabetes). It not only ties together sugar and smoking but also various other co-existing risk factors that result from diet (excess intake of refined starches or of processed meat products with excess elastin), or lifestyle (exposure to smog or lack of exercise). The invention puts elastin receptor activation by C-peptide forward as cause of insulin resistance, hypertension and chronic-low inflammation or blood vessel over repair in metabolic syndrome and ties this syndrome together with other conditions of insulin resistance, such as COPD due to smoking and exposure to fine particular matter, where elastin-derived peptides may activate the elastin receptor to cause insulin resistance and over-repair.

### Description of disclosures

Provided herein is a new target for control of metabolic syndrome and for the development of immune-modulatory peptides directed against metabolic syndrome and against microvascular complications in diabetes: C-peptide's interaction with the elastin receptor. This specification provides a substantial jump in thinking about the cause of metabolic syndrome and of microvascular complications in diabetes. The application describes the presence of a canonical elastin receptor binding motif, GxxP or xGxxPG, in C-peptide, a fact that has been overlooked by the medical community at large. What is more, the motif is located in a hydrophobic midportion of C-peptide which was already in 1997 identified as central to its biological activity, but rejected as possible receptor binding site. This application shows that rejection to be invalid. The invention puts elastin receptor activation by C-peptide forward as cause of insulin resistance, hypertension and chronic-low over-repair in metabolic syndrome and ties this syndrome together with other conditions of insulin resistance, such as COPD due to smoking and exposure to fine particular matter, where elastin-derived peptides may activate the elastin receptor to cause insulin resistance and over-repair.

Certain embodiments of the invention provided herein relate to the etiology of metabolic syndrome and provide methods for antagonist peptide development, treatment and/or prevention of metabolic syndrome. In certain embodiments, antagonist peptide development, treatment or prevention may be directed to one or more of insulin resistance, atherosclerosis, cardiovascular disease, and/or micro- and macrovascular pathologies associated with diabetes mellitus. All amino acid sequences herein are depicted in the one-letter-code. C-peptide is found with all mammals that produce insulin, as it is co-produced and co-excreted with insulin by beta-cells of the pancreas. Common C-peptide's amino acid sequence is SEQ ID NO: 1 (EAEDLQVGQVELGGGPGAGSLQPLALEGSLQ). It is herein disclosed that C-peptides from a wide variety of species bear elastin receptor binding motifs within their hydrophobic midportion (in humans SEQ ID NO: 8 (GGGPGAG)).

A receptor for C-peptide is herein identified as the elastin binding protein, which can be found in the elastin receptor complex as the alternatively spliced galactosidase derived from beta-galactosidase, encoded by the GLB1 gene (Ubiprot identifier P16278). The isoform 1 of the gene product relates to the beta galactosidase (beta-Gal) whereas the isoform 2 relates to the alternatively spliced galactosidase (S-Gal). http://www.piphuman.eu/site/home.html Beta-Gal (isoform 1) cleaves beta-linked terminal galactosyl residues from gangliosides, glycoproteins, and glycosaminoglycans, and is located mainly in the lysosomes.

Isoform 2 (S-Gal) has little or no beta-galactosidase catalytic activity but plays functional roles in the formation of extracellular elastic fibers (elastogenesis) and in the development of connective tissue. S-Gal is considered identical to the elastin-binding protein (EBP), a major component of the non-integrin cell surface receptor expressed on fibroblasts, smooth muscle cells, chondroblasts, leukocytes, and certain cancer cell types. In elastin producing cells, EBP associates with tropoelastin intracellularly and functions as a recycling molecular chaperone that facilitates the secretions of tropoelastin and its assembly into elastic fibers.

Provided are methods for producing pharmaceutical compositions for treating a non-human suffering from metabolic syndrome or a related disorder or considered to be suffering from metabolic syndrome or a related disorder or at risk of suffering from metabolic syndrome or a related disorder. Such methods typically include synthesizing or isolating an antagonist of the interaction or binding of C-peptide with an elastin receptor. The antagonist may or may not be mixed with a pharmacologically acceptable excipient. If desired, the resulting mixture may be labeled as suitable for treating metabolic syndrome or a related disorder, or the prevention of metabolic syndrome or a related disorder.

As used herein, an antagonist of the interaction or binding of C-peptide with an elastin receptor may be any molecule that disrupts, abrogates, interferes with, or diminishes the ability of an elastin receptor to bind to C-peptide. Examples of such antagonist include, but are not limited to, antibodies or small molecules with the above-defined activity. Antibodies may include, but are not limited to, antibodies (monoclonal and/or polyclonal), single chain antibodies, bispecific antibodies, single domain antibodies, and antibody fragments.

In certain aspects, the antagonist peptide may bind to or interact with either C-peptide or with the elastin receptor. Further, the antagonist may bind to the elastin receptor-binding motif in C-peptide or to the site in the elastin receptor that binds to the elastin receptor-binding motif. Alternatively, the antagonist may bind to a site proximal or distal to the elastin receptor-binding motif in C-peptide or to the site in the elastin receptor that binds to the elastin receptor-binding motif but allows action as an antagonist of the C-peptide/elastin receptor interaction. In this way, the antagonist may affect the interaction between C-peptide and the elastin receptor while not interfering with the interaction between the elastin and other binding partners.

Also provided are methods for treating an animal suffering from or considered to be suffering from metabolic syndrome or a related disorder. Such methods typically include synthesizing or isolating an antagonist of the interaction or binding of C-peptide with an elastin receptor and providing the animal with the antagonist. The antagonist may be mixed with a pharmacologically acceptable excipient and the resulting mixture may be labeled as suitable for treating metabolic syndrome or a related disorder, or the prevention of metabolic syndrome.

Disclosed are methods for treating an animal considered to be at risk of suffering from metabolic syndrome or a related disorder. Such methods typically include synthesizing or isolating an antagonist of the interaction or binding of C-peptide with an elastin receptor and providing the animal with the antagonist. The antagonist may be mixed with a pharmacologically acceptable excipient and the resulting mixture may be labeled as suitable for an animal considered to be at risk of suffering from metabolic syndrome or a related disorder.

Disclosed are methods for producing a pharmaceutical composition for treating an animal suffering from type-2 diabetes or considered to be suffering from type-2 diabetes, or at risk of suffering from metabolic syndrome or a related disorder. Such methods typically include synthesizing or isolating an antagonist of the interaction or binding of C-peptide with an elastin receptor and providing the animal with the antagonist. The antagonist may be mixed with a pharmacologically acceptable excipient and the resulting mixture may be labeled as suitable for treating type-2 diabetes, or a related disorder.

Disclosed are methods for treating an animal suffering from or considered to be suffering from type-2 diabetes. Such methods typically include synthesizing or isolating an antagonist of the interaction or binding of C-peptide with an elastin receptor and providing the animal with the antagonist. The antagonist may be mixed with a pharmacologically acceptable excipient and the resulting mixture may be labeled as suitable for treating type-2 diabetes, or the prevention of type-2 diabetes.

Disclosed are methods for treating an animal considered to be at risk of suffering from type-2 diabetes. Such methods typically include synthesizing or isolating an antagonist of the interaction or binding of C-peptide with an elastin receptor and providing the animal with the antagonist. The antagonist may be mixed with a pharmacologically acceptable excipient and the resulting mixture may be labeled as suitable for the treatment or the prevention of type-2 diabetes.

By way of non-limiting theory as to function, damage to and destruction of the beta-cells is not only causal in type-1 diabetes but is also seen in the development of diabetes types 1.5 and 2, and metabolic syndrome as a whole as well, and the phenomena seen with insulin resistance are secondary or parallel to initial events in the pancreatic beta-cells. The damage to and destruction of the beta-cells is primarily caused by an overproduction of C-peptide that is secreted by these cells, deposited in its periphery, and leading to low-grade and initially heterogenic chronic inflammation of beta-cells and islets of Langerhans by C-peptides interaction with cells bearing the elastin receptor, an interaction mediated in C-peptides by binding of the receptor to the hydrophobic midportion SEQ ID NO: 32 (LGGGPGAG). Before, during or after these initial events or in conjunction therewith and when overproduction of C-peptide is maintained, low-grade inflammation is extended to peripheral tissues where C-peptide is deposited as well and again cells bearing the elastin receptor are stimulated. Aspects herein provide antagonists of the C-peptide/EBP interaction and methods of treating a non-human so as to antagonize the C-peptide/EBP interaction.

Provided are methods for producing pharmaceutical compositions for treating an animal suffering from metabolic syndrome or a related disorder or considered to be suffering from metabolic syndrome or a related disorder, or at risk of suffering from metabolic syndrome or a related disorder. Such methods typically include synthesizing or isolating an inhibitor of a prohormone convertase. Prohormone convertases (PCs) are the enzymes involved in the process of proinsulin to insulin and C-peptide. In examples, the inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inibitor may or may not be mixed with a pharmacologically acceptable excipient. If desired, the resulting mixture may be labeled as suitable for treating metabolic syndrome or a related disorder, or the prevention of metabolic syndrome or a related disorder. In certain aspects, the composition may also comprise insulin sufficient to replace the insulin lost by the inhibition of one or more PCs. In certain aspects, the composition may not comprise C-peptide.

Also provided are methods for treating an animal suffering from or considered to be suffering from metabolic syndrome or a related disorder. Such methods typically include synthesizing or isolating an inhibitor of a prohormone convertase. The inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inibitor may or may not be mixed with a pharmacologically acceptable excipient. If desired, the resulting mixture may be labeled as suitable for treating metabolic syndrome or a related disorder, or the prevention of metabolic syndrome. In certain aspects, the composition may also comprise insulin sufficient to replace the insulin lost by the inhibition of one or more PCs. In certain aspects, the composition may not comprise C-peptide.

Disclosed are methods for treating an animal considered to be at risk of suffering from metabolic syndrome or a related disorder. Such methods typically include synthesizing or isolating an inhibitor of a prohormone convertase. In examples, the inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inibitor may or may not be mixed with a pharmacologically acceptable excipient. If desired, the resulting mixture may be labeled as suitable for an animal considered to be at risk of suffering from metabolic syndrome or a related disorder. In certain aspects, the composition may also comprise insulin sufficient to replace the insulin lost by the inhibition of one or more PCs. In certain aspects, the composition may not comprise C-peptide.

Disclosed are methods for producing a pharmaceutical composition for treating an animal suffering from type-2 diabetes or considered to be suffering from type-2 diabetes, or at risk of suffering from metabolic syndrome or a related disorder. Such methods typically include synthesizing or isolating an inhibitor of a prohormone convertase. In examples, the inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inibitor may or may not be mixed with a pharmacologically acceptable excipient. If desired, the resulting mixture may be labeled as suitable for treating type-2 diabetes, or a related disorder. In certain aspects, the composition may also comprise insulin sufficient to replace the insulin lost by the inhibition of one or more PCs. In certain aspects, the composition may not comprise C-peptide.

Disclosed are methods for treating an animal suffering from or considered to be suffering from type-2 diabetes. Such methods typically include synthesizing or isolating an inhibitor of a prohormone convertase. In examples, the inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inibitor may or may not be mixed with a pharmacologically acceptable excipient. If desired, the resulting mixture may be labeled as suitable for treating type-2 diabetes, or the prevention of type-2 diabetes. In certain aspects, the composition may also comprise insulin sufficient to replace the insulin lost by the inhibition of one or more PCs. In certain aspects, the composition may not comprise C-peptide.

Disclosed are methods for treating an animal considered to be at risk of suffering from type-2 diabetes. Such methods typically include synthesizing or isolating an inhibitor of a prohormone convertase. In examples, the inhibitor may be an inhibitor of PC2, such as, but not limited to Chlorpyrifos. In further examples, the inhibitor may be an inhibitor of PC1 and/or PC2 such as, but not limited to, L-alanyl-L-lysyl-L-arginylmethyldimethylsulphonium and L-alanyl-L-arginyl-L-arginylmethyldimethylsulphonium. The inhibitor may or may not be mixed with a pharmacologically acceptable excipient. If desired, the resulting mixture may be labeled as suitable for the treatment or the prevention of type-2 diabetes. In certain aspects, the composition may also comprise insulin sufficient to replace the insulin lost by the inhibition of one or more PCs. In certain aspects, the composition may not comprise C-peptides.

Provided are methods for producing pharmaceutical compositions for treating an animal suffering from metabolic syndrome or a related disorder or considered to be suffering from metabolic syndrome or a related disorder, or at risk of suffering from metabolic syndrome or a related disorder. Such methods typically include synthesizing or isolating a protease that cleaves C-peptide or a fragment there of such that it can no longer bind to EBP. In examples, the protease may cleave at xG:xP, Gx:xP, Gx:xPG, or xG:xPG (where the ":" represents the cleavage site), such as, but not limited to A2pro. The protease may or may not be mixed with a pharmacologically acceptable excipient. If desired, the resulting mixture may be labeled as suitable for treating metabolic syndrome or a related disorder, or the prevention of metabolic syndrome or a related disorder.

Also provided are methods for treating an animal suffering from or considered to be suffering from metabolic syndrome or a related disorder. Such methods typically include synthesizing or isolating a protease that cleaves C-peptide or a fragment there of such that it can no longer bind to EBP. In examples, the protease may cleave at xG:xP, Gx:xP, Gx:xPG, or xG:xPG (where the ":" represents the cleavage site), such as, but not limited to A2pro. The protease may or may not be mixed with a pharmacologically acceptable excipient. If desired, the resulting mixture may be labeled as suitable for treating metabolic syndrome or a related disorder, or the prevention of metabolic syndrome.

Disclosed are methods for treating an animal considered to be at risk of suffering from metabolic syndrome or a related disorder. Such methods typically include synthesizing or isolating a protease that cleaves C-peptide or a fragment there of such that it can no longer bind to EBP. In examples, the protease may cleave at xG:xP, Gx:xP, Gx:xPG, or xG:xPG (where the ":" represents the cleavage site), such as, but not limited to A2pro. The protease may or may not be mixed with a pharmacologically acceptable excipient. If desired, the resulting mixture may be labeled as suitable for an animal considered to be at risk of suffering from metabolic syndrome or a related disorder.

Disclosed are methods for producing a pharmaceutical composition for treating an animal suffering from type-2 diabetes or considered to be suffering from type-2 diabetes, or at risk of suffering from metabolic syndrome or a related disorder. Such methods typically include synthesizing or isolating a protease that cleaves C-peptide or a fragment there of such that it can no longer bind to EBP. In examples, the protease may cleave at xG:xP, Gx:xP, Gx:xPG, or xG:xpG (where the ":" represents the cleavage site), such as, but not limited to A2pro. The protease may or may not be mixed with a pharmacologically acceptable excipient. If desired, the resulting mixture may be labeled as suitable for treating type-2 diabetes, or a related disorder

Disclosed are methods for treating an animal suffering from or considered to be suffering from type-2 diabetes. Such methods typically include synthesizing or isolating a protease that cleaves C-peptide or a fragment there of such that it can no longer bind to EBP. In examples, the protease may cleave at xG:xP, Gx:xP, Gx:xPG, or xG:xpG (where the ":" represents the cleavage site), such as, but not limited to A2pro. The protease may or may not be mixed with a pharmacologically acceptable excipient. If desired, the resulting mixture may be labeled as suitable for treating type-2 diabetes, or the prevention of type-2 diabetes.

Disclosed are methods for treating an animal considered to be at risk of suffering from type-2 diabetes. Such methods typically include synthesizing or isolating a protease that cleaves C-peptide or a fragment there of such that it can no longer bind to EBP. In examples, the protease may cleave at xG:xP, Gx:xP, Gx:xPG, or xG:xpG (where the ":" represents the cleavage site), such as, but not limited to A2pro. The protease may or may not be mixed with a pharmacologically acceptable excipient. If desired, the resulting mixture may be labeled as suitable for the treatment or the prevention of type-2 diabetes.

Also provided are methods for treating an animal suffering from or considered to be suffering from metabolic syndrome or a related disorder. Such methods typically include removing active C-peptide and C-peptide fragments from circulation in the animal. By way of nonlimiting example, the removal may take place by using dialysis. In examples, removal may take place by passing the blood and/or lymph in circulation in the animal over a substance coated with a material that binds to or cleaves C-peptide. In non-limiting examples, the substance may be coated with antibodies specific for C-peptide. In further nonlimiting examples, the substance may be coated with a protease capable of cleaving C-peptide. Such a protease may cleave at xG:xP, Gx:xP, Gx:xPG, or xG:xpG (where the ":" represents the cleavage site), such as, but not limited to A2pro.

Disclosed are methods for treating an animal considered to be at risk of suffering from metabolic syndrome or a related disorder. Such methods typically include removing active C-peptide from circulation in the animal. By way of nonlimiting example, the removal may take place by using dialysis. In examples, removal may take place by passing the blood and/or lymph in circulation in the animal over a substance coated with a material that binds to or cleaves C-peptide. In non-limiting examples, the substance may be coated with antibodies specific for C-peptide. In further nonlimiting examples, the substance may be coated with a protease capable of cleaving C-peptide. Such a protease may cleave at xG:xP, Gx:xP, Gx:xPG, or xG:xpG (where the ":" represents the cleavage site), such as, but not limited to A2pro.

Disclosed are methods for producing a pharmaceutical composition for treating an animal suffering from type-2 diabetes or considered to be suffering from type-2 diabetes, or at risk of suffering from metabolic syndrome or a related disorder. Such methods typically include removing active C-peptide and C-peptide fragments from circulation in the animal. By way of nonlimiting example, the removal may take place by using dialysis. In examples, removal may take place by passing the blood and/or lymph in circulation in the animal over a substance coated with a material that binds to or cleaves C-peptide. In non-limiting examples, the substance may be coated with antibodies specific for C-peptide. In further nonlimiting examples, the substance may be coated with a protease capable of cleaving C-peptide. Such a protease may cleave at xG:xP, Gx:xP, Gx:xPG, or xG:xpG (where the ":" represents the cleavage site), such as, but not limited to A2pro.

Disclosed are methods for treating an animal suffering from or considered to be suffering from type-2 diabetes. Such methods typically include removing active C-peptide and C-peptide fragments from circulation in the animal. By way of nonlimiting example, the removal may take place by using dialysis. In examples, removal may take place by passing the blood and/or lymph in circulation in the animal over a substance coated with a material that binds to or cleaves C-peptide. In non-limiting examples, the substance may be coated with antibodies specific for C-peptide. In further nonlimiting examples, the substance may be coated with a protease capable of cleaving C-peptide. Such a protease may cleave at xG:xP, Gx:xP, Gx:xPG, or xG:xpG (where the ":" represents the cleavage site), such as, but not limited to A2pro.

Disclosed are methods for treating an animal considered to be at risk of suffering from type-2 diabetes. Such methods typically include removing active C-peptide and C-peptide fragments from circulation in the animal. By way of nonlimiting example, the removal may take place using dialysis. In examples, removal may take place by passing the blood and/or lymph in circulation in the animal over a substance coated with a material that binds to or cleaves C-peptide. In non-limiting examples, the substance may be coated with antibodies specific for C-peptide. In further nonlimiting examples, the substance may be coated with a protease capable of cleaving C-peptide. Such a protease may cleave at xG:xP, Gx:xP, Gx:xPG, or xG:xpG (where the ":" represents the cleavage site), such as, but not limited to A2pro.

In a further aspects, provided are peptides derived from the C-peptide (Ulniprot identifier >sp | P01308|57-87) or functional mammalian equivalents thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif. Note that no stereoisomers of glycine exist, herein L-glycine and D-glycine both stand for glycine. By way of non-limiting example, the peptides may be synthesized by a solid-phase method with an automated peptide synthesizer (such as model 990; Beckman Instrument, Fullerton, CA). The peptides may be purified by reverse phase high-performance liquid chromatography (such as Capcell Pak C-18, Shiseido, Tokyo, Japan). The sequence of the peptide may be confirmed with a mass spectrometer (such as Voyager, Linear-DE/K, Preseptive Biosystems, TX).

An exemplary octapeptide as provided herein comprising the xGxP or GxxP motif derived from the C-peptide is the octapeptide SEQ ID NO: 32 (LGGGPGAG) that is selected from the C-peptide sequence as a whole and very well suited for non-human primate use considering its 100% homology over the stretch of 8 amino acids in C-peptide from which it is derived. Also provided is the retro-inverso variant, the all-D-amino acid peptide GAGPGGGL, and peptides or peptidometics of at most 30, preferably of at most 25, preferably of at most 20, preferably of at most 12, preferably of at most 9 amino acids, comprising the all-D-amino acid peptide GAGPGGGL. Note: no stereoisomers of glycine exist, here (and in retro-inverso peptides bearing GxxP or xGxP motifs) G is not, whereas other amino acids, such as L, P and A are, instrumental to the all-D-amino acid character of the retro-inverso peptide herein provided.

An exemplary heptapeptide as provided herein comprising the xGxP or GxxP motif derived from the C-peptide is the heptapeptide SEQ ID NO: 8 (GGGPGAG) that is selected from the C-peptide sequence as a whole and very well suited for non-human primate use considering its 100% homology over the stretch of 7 amino acids in C-peptide from which it is derived. Also provided is the retro-inverso variant, the all-D-amino acid peptide GAGPGGG, and peptides or peptidometics of at most 30, preferably of at most 25, preferably of at most 20, preferably of at most 12, preferably of at most 8 amino acids, comprising the all-D-amino acid peptide GAGPGGG.

Another exemplary heptapeptide as provided herein comprising the xGxP or GxxP motif derived from the C-peptide is the heptapeptide SEQ ID NO: 47 (LGGGPGA) that is selected from the C-peptide sequence as a whole and very well suited for non-human primate use considering its 100% homology over the stretch of 7 amino acids in C-peptide from which it is derived. Also provided is the retro-inverso variant, the all-D-amino acid peptide GAGPGGG, and peptides or peptidometics of at most 30, preferably of at most 25, preferably of at most 20, preferably of at most 12, preferably of at most 9, preferably of at most 7 amino acids, comprising the all-D-amino acid peptide AGPGGGL.

A most exemplary hexapeptide as provided herein comprising the xGxP or GxxP motif derived from the C-peptide is the hexapeptide SEQ ID NO: 48 (GGPGAG) that is selected from the C-peptide sequence as a whole and very well suited for non-human primate use considering its 100% homology over the stretch of 6 amino acids in C-peptide from which it is derived. Also provided is the retro-inverso variant, the all-D-amino acid peptide GAGPGG, and peptides or peptidometics of at most 30, preferably of at most 25, preferably of at most 20, preferably of at most 12, preferably of at most 9, preferably of at most 6 amino acids, comprising the all-D-amino acid peptide GAGPGG.

Another exemplary heptapeptide as provided herein comprising the xGxP or GxxP motif derived from the C-peptide is the heptapeptide SEQ ID NO: 34 (LGGGPG) that is selected from the C-peptide sequence as a whole and very well suited for non-human primate use considering its 100% homology over the stretch of 6 amino acids in C-peptide from which it is derived. Also provided is the retro-inverso variant, the all-D-amino acid peptide GPGGGL, and peptides or peptidometics of at most 30, preferably of at most 25, preferably of at most 20, preferably of at most 12, preferably of at most 9, preferably of at most 6 amino acids, comprising the all-D-amino acid peptide GPGGGL.

Another exemplary heptapeptide as provided herein comprising the xGxP or GxxP motif derived from the C-peptide is the hexapeptide SEQ ID NO: 49 (GGGPGA) that is selected from the C-peptide sequence as a whole and very well suited for non-human primate use considering its 100% homology over the stretch of 6 amino acids in C-peptide from which it is derived. Also provided is the retro-inverso variant, the all-D-amino acid peptide AGPGGG, and peptides or peptidometics of at most 30, preferably of at most 25, preferably of at most 20, preferably of at most 12, preferably of at most 9, preferably of at most 6 amino acids, comprising the all-D-amino acid peptide AGPGGG.

A most exemplary pentapeptide as provided herein comprising the xGxP or GxxP motif derived from the C-peptide is the heptapeptide SEQ ID NO: 40 (GGGPG) that is selected from the C-peptide sequence as a whole and very well suited for non-human primate use considering its 100% homology over the stretch of 6 amino acids in C-peptide from which it is derived. Also provided is the retro-inverso variant, the all-D-amino acid peptide GPGGG, and peptides or peptidometics of at most 30, preferably of at most 25, preferably of at most 20, preferably of at most 12, preferably of at most 9, preferably of at most 5 amino acids, comprising the all-D-amino acid peptide GPGGG,

Another exemplary pentapeptide is peptide SEQ ID NO: 46 (GAGPG), and peptides or peptidometics of at most 30, preferably of at most 25, preferably of at most 20, preferably of at most 12, preferably of at most 9, preferably of at most 5 amino acids, comprising the peptide SEQ ID NO: 46 (GAGPG).

Another exemplary pentapeptide is a retro-inverso variant, the all-D-amino acid peptide GPGAG, and peptides or peptidometics of at most 30, preferably of at most 25, preferably of at most 20, preferably of at most 12, preferably of at most 9, preferably of at most 5 amino acids, comprising the all-D-amino acid peptide GPGAG.

A most exemplary tetrapeptide as provided herein comprising the xGxP or GxxP motif derived from the C-peptide is the tetrapeptide SEQ ID NO: 38 (GGGP) that is selected from the C-peptide sequence as a whole and very well suited for non-human primate use considering its 100% homology over the stretch of 6 amino acids in C-peptide from which it is derived. Also provided is the retro-inverso variant, the all-D-amino acid peptide PGGG, and peptides or peptidometics of at most 30, preferably of at most 25, preferably of at most 20, preferably of at most 12, preferably of at most 9, preferably of at most 4 amino acids, comprising the all-D-amino acid peptide PGGG.

Another tetrapeptide as provided herein comprising the GxxP motif is the tetrapeptide SEQ ID NO: 39 (GAGP). Also provided is the retro-inverso variant, the all-D-amino acid peptide PGAG, and peptides or peptidometics of at most 30, preferably of at most 25, preferably of at most 20, preferably of at most 12, preferably of at most 9, preferably of at most 4 amino acids, comprising the all-D-amino acid peptide PGAG.

In certain aspects is provided the use of a peptide derived from the C-peptide (Ulniprot identifier >sp|P01308|57-87) or from functional mammalian equivalents thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif for treating metabolic syndrome, preferably of diabetes mellitus, preferably of type-1 diabetes or of nephropathies, neuropathies or microvascular disease associated with type-1 diabetes.

As such, provided herein are methods for treating an animal for type-1 diabetes. Such methods typically include administering to the animal a peptide derived from the C-peptide (Ulniprot identifier >sp |P01308|57-87) or from functional mammalian equivalent thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif. In certain aspects, the methods of treatment may be for one or more pathology associated with type-1 diabetes including, but not limited to, nephropathies, neuropathies or microvascular disease.

In certain aspects, provided are peptides derived from the C-peptide (Ulniprot identifier >sp|P01308|57-87) or from functional mammalian equivalent thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif for treating metabolic syndrome, preferably of diabetes mellitus, preferably of type-1 diabetes or of nephropathies, neuropathies or microvascular disease associated with type-1 diabetes.

Also provided is the use of a peptide derived from the C-peptide (Ulniprot identifier >sp|P01308|57-87) or from functional mammalian equivalent thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif for producing a medicament for treating metabolic syndrome, preferably of diabetes mellitus, preferably of type-1 diabetes or of nephropathies, neuropathies or microvascular disease associated with type-1 diabetes.

As such, provided herein are methods for producing a medicament for treating of an animal for type-1 diabetes. Such methods typically include synthesizing or isolating a peptide derived from the C-peptide (Ulniprot identifier >sp | P01308|57-87) or from functional mammalian equivalent thereof consisting of an octapeptide, hexapeptide, heptapeptide, pentapeptide or tetrapeptide comprising a GxxP or xGxP motif wherein G is glycine, P is proline, and x is any amino acid, and retro-inverso variants of the octapeptide, hexapeptide, heptapeptide, pentapeptide or tetrapeptide comprising the xGxP or GxxP motif.

In beta-cells, insulin is produced in conjunction with C-peptide from the same precursor molecule, preproinsuline. Both insulin and C-peptide are excreted in equal amounts into the blood, whereby insulin can act on peripheral tissues for a short time only, having a half-life of approximately 5 minutes. C-peptide, however, has a reported half-life of 30 minutes, and circulates much longer in the blood. Although C-peptide's function has long not been understood, many activities are currently being ascribed to it among which are pro-inflammatory activities. The source of theses pro-inflammatory activities has not been explained. As provided herein, structural analysis of C-peptide reveals the presence of an elastin receptor-binding motif, which, without being bound to a particular theory, is the cause of the pro-inflammatory effect.

Common C-peptide's amino acid sequence is: SEQ ID NO: 1 (EAEDLQVGQVELGGGPGAGSLQPLALEGSLQ). The mid-portion, SEQ ID NO: 42 (ELGGGPGAGS), bears an additional, hitherto unnoticed, characteristic PG-domain that explains the so-called pro-inflammatory character of C-peptide, and in particular explains the low-grade, and initially heterogenic, chronic inflammation (herein identified as blood vessel over-repair) that is seen with diabetes type-2 and metabolic syndrome, and more in particular, explains the onset, the etiology, of diabetes type-2 as a whole. It is herein recognized that SEQ ID NO: 42 (ELGGGPGAGS) bears a canonical xGxP, GxxP, GxxPG and xGxPG (x being any amino acid, preferably a hydrophobic amino acid) sequence found in peptides reactive with the elastin binding protein, EBP. The elastin binding protein (EBP), a spliced variant of lysosomal beta-galactosidase, is the primary receptor of elastin peptides that for example have been linked to emphysema, aneurism and cancer progression. The sequences recognized by EBP share the GxxP consensus pattern found in numerous matrix proteins, notably in elastin where the SEQ ID NO: 41 (VGVAPG) motif is repeated. Herein, C-peptide is recognized for the first time as being a ligand of EBP or the elastin receptor. C-peptide thus has a same set of chemotaxic, matrix-metallo-proteinase (MMP) activating, proliferative and low-inflammatory or vascular repair activities that known elastin-peptides derived from extra-cellular matrix (ECM) proteins have. This receptor interaction has not publicly been recognized before, neither by persons skilled in the art of diabetes research or of metabolic disorder research, nor by those skilled in the art of ECM or elastin peptide research.

Having identified a binding site EBP in C-peptide, the vascular repair or low-inflammatory modulation by C-peptide is now explained. Pericytes, smooth muscle cells, fibroblasts, adipose tissue cells, pancreatic stellate cells, and other connective tissue cells, together with endothelial cells, and circulating innate immune cells such as leucocytes and monocytes, may respond to binding of EBP to GxxPG bearing proteins and peptides by interleukin-1beta mediated proliferation and low-grade inflammatory activation. Analysis of the human proteome shows that proteins with multiple GxxPG motifs are highly related to the extracellular matrix (ECM). Matrix proteins with multiple GxxP, xGxP or GxxPG sites include fibrillin-1, -2, and -3, elastin, fibronectin, laminin, and several tenascins and collagens.

Secondly, circulating leucocytes and monocytes show strong chemotaxis to GxxP, xGxP, GxxPG oe xGxPG bearing proteins and peptides, C-peptide will thus attract those cells to wherever C-peptide is present.

Thirdly, binding of EBP to GxxP, xGxP, xGxPG or GxxPG bearing proteins and peptides has been associated with shedding of EBP from cellular surfaces and increased presentation of the interleukin-I receptor having affinity for interleukin-1beta, allowing for hampered endocytosis or for a continued interleukin-1beta mediated proliferation and inflammatory activation wherever C-peptide deposits are present.

Fourthly, binding of EBP to GxxP or GxxPG bearing proteins and peptides has been associated with the activation of neuraminidase and the release of sialic acid from proteins that induces insulin resistance, in particular of adipocytes and hepatocytes.

Fifthly, binding EBP to GxxP or GxxPG bearing proteins and peptides has been associated with shedding of EBP from cellular surfaces and decreased presentation of PPCA having proteolytic activity towards endothelin-1, whereby increased endothelin-1 levels due to decreased proteolytic acitivity of PPCA result in increased hypertension.

The picture that arises from these observations now explains the fibrotic islet destruction and beta-cell destruction seen with diabetes type-2 and places that destruction proximal in the sequence of events leading to full-blown diabetes type-2 and metabolic syndrome.

The disclosure also provides means to detect a collection of peptides in a sample, each peptide comprising at least one GxPx, GxxP, xGxP, xGxPG or GxxGP motif, said sample preferably a sample from a vertebrate, preferably from a primate, most preferably from a human, preferably wherein said sample is a serum or a plasma or a urine sample, preferably wherein at least one peptide comprises a VAPG sequence, preferably a SEQ ID NO: 41 (VGVAPG) sequence or wherein at least one peptide comprises a GPG sequence, preferably a SEQ ID NO: 34 (GGGPG) sequence, and the disclosure also provides use of such means to determine a risk on developing or having insulin resistance or hypertension or atherosclerosis or diabetes type-2 or metabolic disorder.

In particular, provided herein is a diagnostic test or the use of means in a diagnostic test that detects the presence in serum, plasma, urine or other bodily fluids (such as sputum or ascites or blood) or in bodily tissues of a collection of proteins or peptides bearing a GxxP, xGxP, xGxPG or GxxPG motif for identifying a vertebrate, such as a horse, or a primate, preferably a human, having a risk for the development of or having metabolic syndrome, in particular for identifying the risk of developing or having insulin resistance and/or hypertension.

Certain aspects of such a diagnostic test as provided herein relate to methods of detecting a collection of proteins or peptides bearing a GxxP, xGxP, xGxPG or GxxPG motif interacting with the "elastin receptor type", a receptor type disclosed herein as interacting with C-peptides or C-peptide fragments to promote an anti-inflammatory effect associated with alleviation of nephrophaties and endothelial dysfunction in type-1 diabetes. A collection of peptides of particular interest to detect is those that act as agonist to the elastin receptor. Diagnostic testing can for example be directed at two or more GxxP, xGxPG, GxxPx, GxxPG, xGxxP, xGxxPx, xGxxPG motif bearing peptides, such as two or more peptides for example selected from the group SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 89 (GIPPA), SEQ ID NO: 90 (GGIPPA) or SEQ ID NO: 91 (GGYPGASYPGAYPGQAPPGAYPGQAPPGAYPGAPGAYPGAPAPGVYPGPPSGPGAYPS) or SEQ ID NO: 92 (GGYPGASYP) or SEQ ID NO: 93 (GAYPGQAPP) or SEQ ID NO: 94 (GAYPGQA) or SEQ ID NO: 95 (GAYPGAP) or SEQ ID NO: 96 (GAYPG) or SEQ ID NO: 97 (APAPGVYPG) or SEQ ID NO: 98 (GAYPS) or ID NO: 57 (GVYPG) or related peptides.

The invention provides a diagnostic method and test comprising measuring peptides from two or more or all these various sources or causes to reflect a level of damage that has activation of the elastin receptor complex in common, with peptides having the PG-domain motifs binding to the elastin receptor complex (ERC) at the cell surface. This invention thus unifies testing for causes arterial risk such as of insulin resistance and/or hypertension, the common denominator being testing for circulating agonists of elastin receptor (and EBP) activation, bearing the motif GxxP.

It is herein provided to simultaneously detect C-peptide levels and elastin remnants in an individual, preferably a vertebrate, preferably a primate, most preferably a human, with commonly available tests such as the well-known C-peptide test (Wang L, Lovejoy NF, Faustman DL. Diabetes Care. 2012;35:465-470. doi: 10.2337/dc11-1236. Gunther EW, Lewis BL, Koncikowski SM. Laboratory procedures used for the Third National Health and Nutrition Examination Survey (NHANES III) 1988-1994. Atlanta (GA): US Department of Health and Human Services; 1996), or the desmosine test (HUANG JT et al., Thorax. 2012 Jun;67(6):502-8), or the elastine peptide test (Fullop et al, Clin Physiol Biochem. 1990;8(6):273-8). Combining C-peptide and desmosine tests or C-peptide and elastine peptide tests to generate outcomes that reflect risks on insulin resistance and/or hypertension is thus herein for the first time provided. Such testing can be performed on different samples of the same individual, but preferably a single sample of an an individual is used, such as for example is provided herein by combining testing one individual or sample derived from that individual with a C-peptide test such as a C-peptide binding assay and also testing that sample with a desmosine detection test or an elastin peptide test, which detects remnants of elastin, such as often used for COPD testing. Combining or cumulating the outcomes of these tests then allows estimating the risk on disease associated for example with insulin resistance and hypertension derived from elastin receptor being activated by GxxP, xGxP, xGxPG or GxxPG motif bearing peptides, thereby more accurately determining the combined effects of these peptides, preferably of C-peptides combined with elastin peptides, of various origin on the same receptor. As provided herein such a use of means should preferably be directed at detecting the collection of peptides or proteins having the SEQ ID NO: 34 (GGGPG) motif, as derived from C-peptide and detecting peptides or proteins having the SEQ ID NO: 41 (VGVAPG) motif as derived from elastin peptides, preferably combined with detecting peptides of other sources having amino acid sequences bearing a GxxP, xGxP, xGxPG or GxxPG motif, the collection of these motif-bearing peptides being instrumental in causing insulin resistance and hypertension.

In a further aspect, the disclosure provides use of (recombinant or synthetic) EBP in a Ligand-Binding Assay (LBA) to detect a collection of peptides bearing a GxxP, xGxP, xGxPG or GxxPG motif. Recombinant or synthetic EBP is known in the art and available for use in such an LBA to detect the collection of peptides interacting or binding with it. In yet another aspect, the disclosure also provides use of a fragment of (recombinant or synthetic) EBP in a Ligand-Binding Assay (LBA) to detect a collection of peptides bearing a GxxP, xGxP, xGxPG or GxxPG motif, said fragment at least comprising the essential ligand-binding peptide sequence SEQ ID NO: 105 (AQDEAS), such as SEQ ID NO: 100 (LPGSCGQVVGSPSAQDEASPLSEWRASYNSAG) and other peptides shown herein above. Ligand-Binding assays, such as surface-plasmon resonance assays or Enzym-Linked-Immuno-Sorbent-Assays (ELISA) or Lateral Flow Immuno Assays (LFIA) are well known in the art and utilize the affinity between the ligand and the receptor as the base for the detection system. Of course, it is also possible to use an antibody specifically recognizing a collection of peptides bearing a PG-domain with such a Ligand-Binding Assay.

Although peptides and proteins are mostly determined using ligand-binding assays (LBAs), there is also a trend towards the use of LC-MS. This can be explained by the fact that LC-MS offers many advantages, such as excellent selectivity for the analysis of compounds in complex biological matrices, high sensitivity, good precision and accuracy, and a wide dynamic range. An established bioanalytical workhorse is LC-MS using the triple-quadrupole mass spectrometer (TQMS) interfaced to high-performance liquid chromatography (HPLC) or ultra-HPLC (UHPLC). The invention thus provides a use of means, such as in a diagnostic test, comprising detecting or determining the level of of proteins or peptides bearing a PG-domain. Herewith, the invention provides a diagnostic test for identifying a vertebrate at risk for havening or developing insulin resistance and/or hypertension and /or metabolic syndrome. Such a means preferably comprises use of means for mass spectrometry such as LC/MC-MS or tandem MS. General techniques are for example described in Mass Spectrometry Handbook, Michael S Lee (Editor), ISBN: 978-0-470-53673-5. Additional techniques to detect a collection of peptides by mass spectrometry having a common motif are for example described in Liebler DC, Hansen BT, Davey SW, Tiscareno L, Mason DE. Peptide sequence motif analysis of tandem MS data with the SALSA algorithm. Analytical Chemistry. 2002;74:203-10 and Erassov JLA, Halina P, Canete M, Vo ND, Chung C, Cagney G, et al. Sequential interval motif search: Unrestricted database surveys of global MS/MS data sets for detection of putative post-translational modifications. Analytical Chemistry. 2008;80:7846-54.

In particular, provided herein is the use of means in a diagnostic test that detects the presence of an amino acid substitution (i.e. a difference in peptide sequence among individuals, groups, or populations) in a C-peptide, for identifying a vertebrate, such as a horse, or a primate, preferably a human, having a different inflammatory activity derived from the C-peptide than a vertebrate not having the amino acid substitution in the C-peptide. In particular, provided is the use of means that detect the presence of an amino acid substitution in a C-peptide in a human having a different pro-inflammatory activity derived from the C-peptide than a human having a C-peptide amino acid sequence as identified in Table 1 with C-peptide interspecies comparisons as provided herein under Uniprot identifier >sp|P01308|57-87. That a human individual with a variant C-peptide exists has been known, however such a variant C-peptide has not earlier been associated with variant pro-inflammatory activity in the human individual. In particular, provided is the use of means that detect the presence of an amino acid substitution (i.e. a difference in peptide sequence among individuals, groups, or populations) in a C-peptide, for identifying a primate, preferably a human, having a different pro-inflammatory activity derived from the C-peptide than a primate wherein the C-peptide amino acid sequence at least comprises the amino acid sequenceSEQ ID NO: 19 (LQVGQVELGGGPGAGSLQPLAL) more in particular wherein the C-peptide amino acid sequence at least comprises the amino acid sequence SEQ ID NO: 32 (LGGGPGAG) more in particular the provided is the use of means to detect human individuals having a different pro-inflammatory activity derived from the C-peptide, preferably having C-peptide amino acid sequences that differ from a C-peptide that at least comprises the amino acid sequence SEQ ID NO: 32 (LGGGPGAG),

Also provided is the use of means that detect the presence of an amino acid substitution in a C-peptide in a horse having a different pro-inflammatory activity derived from the C-peptide than a horse having a C-peptide amino acid sequence as identified in the Table with C-peptide interspecies comparisons as provided herein under Uniprot identifier >sp|P01310|33-63. That a horse (or pony) individual with a variant C-peptide exists has been known, however such a variant C-peptide has not earlier been associated with variant anti- or pro-inflammatory activity in the horse or pony individual. In particular, provided is the use of means that detect the presence of an amino acid substitution (i.e. a difference in peptide sequence among individuals, groups, or populations) in a C-peptide, for identifying a horse having a different pro-inflammatory activity derived from the C-peptide than a horse wherein the C-peptide amino acid sequence at least comprises the amino acid sequence SEQ ID NO: 157 (EAEDPQVGEVELGGGPGLGGLQPLALAGPQQ).Also provided is the use of means that detect the presence of an amino acid substitution in a C-peptide in a cow having a different pro-inflammatory activity derived from the C-peptide than a cow having a C-peptide amino acid sequence as identified in the Table with C-peptide interspecies comparisons as provided herein under Uniprot identifier >sp|P01317|57-82. That a cow individual with a variant C-peptide exists has been known, however such a variant C-peptide has not earlier been associated with variant anti- or pro-inflammatory activity in a cow individual. In particular, provided is the use of means that detect the presence of an amino acid substitution (i.e. a difference in peptide sequence among individuals, groups, or populations) in a C-peptide, for identifying a cow having a different pro-inflammatory activity derived from the C-peptide than a cow wherein the C-peptide amino acid sequence at least comprises the amino acid sequence SEQ ID NO: 228 (EVEGPQVGALELAGGLGAGGLEGPPQ), more in particular wherein the C-peptide amino acid sequence at least comprises the amino acid sequence SEQ ID NO: 229 (AGGLGAG), more in particular provided is the use of means to detect cow individuals having a different pro-inflammatory activity derived from the C-peptide, preferably having C-peptide amino acid sequences that differ from a cow C-peptide that at least comprises the amino acid sequence SEQ ID NO: 230 (AGGPGAG).

In certain aspects, provided is the use of means that detect the presence of an amino acid substitution (i.e. a difference in peptide sequence among individuals, groups, or populations) in a C-peptide, for identifying an animal, preferably a mammal, having a different pro-inflammatory activity derived from the C-peptide than an animal not having the amino acid substitution in the C-peptide. The detection and verification of amino acid substitutions in proteins and peptides can for example be achieved by use of means of mass spectrometry (MS) which is a common technique in protein characterization. A large protein is proteolytically cleaved into peptides and analyzed by MS. Smaller peptides may not need to be cleaved before being analyzed by MS. Peptide sequences are then determined based on the known characteristics of amino acids. General techniques are for example described in Mass Spectrometry Handbook, Michael S Lee (Editor), ISBN: 978-0-470-53673-5. Other use of means to detect C-peptide variations typically include use of antibodies specifically directed against C-peptide variants, and Elisa techniques or other means of antibody facilitated diagnosis known in the art.

In certain aspects, provided is the use of means that detect the presence of a genetic polymorphism (i.e. a difference in DNA sequence among individuals, groups, or populations) in a preproinsulin allele resulting in an amino acid substitution in a C-peptide, for identifying an animal, preferably a mammal, having a different pro-inflammatory activity derived from the C-peptide than an animal not having the amino acid substitution in the C-peptide. Polymerase chain reactions (PCR) can be used because these are considered to be a means of rapidly detecting genetic polymorphisms. Among the many molecular methods currently available for genetic studies, it appears particularly suitable for analysis of any species, revealing a high degree of polymorphism in many cases. PCR can be used with a single short oligonucleotide primer that randomly amplifies short fragments of genomic DNA or can be used with specific primers that amplify specifically a preproinsulin allele or coding sequence. Reverse transcriptase PCR (RT-PCR) can be used when the source of nucleotide sequence is RNA, such as mRNA. General techniques are for example described in Molecular Cloning A Laboratory Manual, Third Edition Joe Sambrook et al. ISBN 978-0879695774. As single amino acid substitutions are often caused by single nucleotide polymorphism (SNP), another use of means of choice is discovery/detection of SNP in the preproinsulin coding sequence. Single nucleotide polymorphism (SNP) detection technologies are routinely used to scan for new polymorphisms and to determine the allele(s) of a known polymorphism in target sequences. Methods for SNP involve a set of biochemical reactions that isolates the precise location of a suspected SNP and then directly determines the identity of the SNP and many SNPs already been detected by comparing different sequenced genomes. This information is then used for SNP mapping.

Aspects relate also in part to the identification of a receptor type which binds C-peptides or C-peptide fragments, thus inducing C-peptide related bioactivity associated with various disorders, such as immune disorders such as metabolic syndrome and diabetes. Such a receptor type which binds or interacts with C-peptides or C-peptide fragments as disclosed herein, is a mammalian elastin- receptor known to bind elastin peptides including but not limited to the elastin receptor complex, including a 67-kDa elastin-binding protein (EBP) identified as an spliced variant of beta-galactosidase, and related homologues and isoforms thereof, that is ubiquitously found on innate immune cells, extra cellular matrix cells, fibroblasts, vascular smooth muscle cells and certain tumor cells. Also binding these motifs are pancreatic elastases, herein understood to also have elastin binding protein type binding activity. Elastin binding protein type, typically binds to canonical xGxP, GxxP, GxxPG and xGxPG (x being any amino acid, preferably an hydrophobic amino acid) motifs in extracellular matrix proteins, such as elastin, laminins, collagen type IV, and fibrillin-1, and such a motif are herein for the first time identified in C-peptides. Elastin binding protein/elastin peptide interaction can also be found with integrins and galectin, EBP, integrins and galectins and other receptors capable of binding to xGxP, GxxP, GxxPG and xGxPG motifs herein commonly called elastin binding protein type. The identification of an "elastin receptor" which interacts with C-peptide to promote a biological response modulating associated with metabolic and immune disorders in turn provides a valuable and essential component when practicing additional aspects, including but not necessarily limited to methods, uses and identified compositions for treating various disorders.

Certain aspects relate to methods of identifying modulators of an "elastin binding protein type", a receptor type disclosed herein as interacting with C-peptides or C-peptide fragments to promote an anti-inflammatory effect associated with alleviation of nephrophaties and endothelial dysfunction in type-1 diabetes. A modulator of particular interest is a compound that acts as an agonist to the elastin binding protein type. Such an agonist may be useful in the treatment of type-1 diabetes or other disorders characterized by relative or absolute C-peptide deficiency such as late-phase type-2 diabetes. While not being bound by theory, such a peptide will show the ability to mediate a signal to an extra-cellular matrix cell or white blood cell (such as a fibroblast or monocyte cell) causing chemotaxic and proliferative effects, for example causing leucocyte chemotaxis or smooth muscle cell or fibroblast proliferation. An agonist can for example be selected from GxxP, xGxPG, GxxPx, GxxPG, xGxxP, xGxxPx, xGxxPG motif bearing peptides, such as peptides SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 89 (GIPPA), SEQ ID NO: 90 (GGIPPA) or SEQ ID NO: 91 (GGYPGASYPGAYPGQAPPGAYPGQAPPGAYPGAPGAYPGAPAPGVYPGPPSGPGAYPS) or SEQ ID NO: 92 (GGYPGASYP) or SEQ ID NO: 93 (GAYPGQAPP) or SEQ ID NO: 94 (GAYPGQA) or SEQ ID NO: 95 (GAYPGAP) or SEQ ID NO: 96 (GAYPG) or SEQ ID NO: 97 (APAPGVYPG) or SEQ ID NO: 98 (GAYPS) or related peptides. While not being bound by theory, such an agonist peptide will show the ability to mediate a signal to an extra-cellular matrix cell or white blood cell (such as a fibroblast or monocyte cell) stimulating chemotaxic and proliferative effects, for example stimulating leucocyte chemotaxis or smooth muscle cell or fibroblast proliferation.

Another modulator of particular interest is a peptide that acts as an antagonist to the elastin binding protein type. Such an antagonist may be useful in the treatment or prevention of type-2 diabetes or other disorders characterized by relative or absolute C-peptide excess, such as atherosclerosis, rheumatoid arthritis, macrovascular disease and cardiovascular disease following the onset of metabolic syndrome. Useful antagonists may be selected from GxxP, xGxPG, GxxPx, GxxPG, xGxxP, xGxxPx, xGxxPG motif binding peptides or binding domains, such as (commonly called) V32- or V14-peptides and fragments therof as for example SEQ ID NO: 99 (QTLPGSCGQVVGSPSAQDEASPLSEWRASYNSAGSNITDA), SEQ ID NO: 100 (LPGSCGQVVGSPSAQDEASPLSEWRASYNSAG), SEQ ID NO: 101 (VVGSPSAQDEASPLSEWRASY), SEQ ID NO: 102 (VVGSPSAQDEASPLS), SEQ ID NO: 103 (PSAQDEASPL), SEQ ID NO: 104 (SPSAQDEASP), SEQ ID NO: 105 (AQDEAS), SEQ ID NO: 106 (PSAQ), SEQ ID NO: 107 (SAQD), SEQ ID NO: 108 (DEAS), SEQ ID NO: 31 (QDEA), SEQ ID NO: 109 (SPSA), SEQ ID NO: 110 (VVGGTEAQRNSWPLQ), SEQ ID NO: 111 (VVGGTEAQRNSWPSQ), SEQ ID NO: 112 (TEAQRNSWP), SEQ ID NO: 113 (AQRN), SEQ ID NO: 114 (IVGGRRARPHAWPFM), SEQ ID NO: 115 (VVGGEDAKPGQFPWQ), SEQ ID NO: 116 (VVGGRVAQPNSWPWQ), SEQ ID NO: 117 (RVAQPNSW), SEQ ID NO: 118 (VVGGAEARRNSWPSQ), SEQ ID NO: 119 (AEARRNSW), SEQ ID NO: 120 (VVGGQEATPNTWPWQ), SEQ ID NO: 121 (QEATPNTW), SEQ ID NO: 122 (VVGGEEARPNSWPWQ), SEQ ID NO: 123 (EEARPNSW), SEQ ID NO: 124 (VVGGTEAGRNSWPSQ), SEQ ID NO: 125 (TEAGRNSWP), SEQ ID NO: 126 (EDYRPSQQDECSPRE), SEQ ID NO: 127 (PSQQDECSP), SEQ ID NO: 128 (QQDEC), QDE, or related peptides. While not being bound by theory, such an antagonist peptide will show the ability to modulate a signal to an extra-cellular matrix cell or white blood cell (such as a fibroblast or monocyte cell) inhibiting chemotaxic and proliferative effects, for example inhibiting leucocyte chemotaxis or smooth muscle cell or fibroblast proliferation.

Other useful agonist or antagonist peptides may for example be found *in silico* employing the homology model of the elastin-binding site of human EBP. Blanchevoy et al recently build a homology model of this protein and showed docking of SEQ ID NO: 41 (VGVAPG) in this model (Blanchevoye et al, INTERACTION BETWEEN THE ELASTIN PEPTIDE VGVAPG AND HUMAN ELASTIN BINDING PROTEIN, doi: 10.1074/jbc.M112.419929 jbc.M112.419929*.*

The assay methods used to practice these aspects may be any method currently available to the artisan, including but not limited to chemotaxis assays, proliferation assays, binding assays utilizing isolated elastin binding protein type, isolated membrane fractions containing elastin binding protein type, binding or cell-based activation assays innate immune cells, as well a functional sensor/effector cell assay measuring the ability of a to stimulate a sensor cell (expressing an elastin binding protein type) to mediate an up-regulation of the interleukin-1beta production or interleukin 1 receptor expression in an effector cell. While reference to a full-length receptor is made throughout this specification, such a reference is not meant as a limitation. Instead, it is understood that such a full-length receptor or a biologically relevant fragment of the receptor (such as a fragment at least comprising the xGxP, GxxP, xGxGP or GxxPG binding domain) may be utilized in practicing the methodology. Thus, certain aspects relate in part to methods of screening for compounds that modulate (i.e., stimulate or inhibit) activity of the elastin binding protein type, and/or by acting as an agonist or antagonist of the elastin binding protein type receptor protein.

To this end, certain aspects relate to a method of identifying a test compound that modulates an elastin binding protein type cellular receptor so as to activate or suppress biological activity related to chemotaxis or proliferation or interleukin-1 upregulation or down-regulation of interleukin 1 receptor upregulation or downregulation. Such a method typically includes providing an amino acid sequence comprising at least the xGxP, GxxP, GxxGP or xGxPG, preferably the SEQ ID NO: 38 (GGGP) or SEQ ID NO: 34 (GGGPG), binding domain of an elastin binding protein type; contacting the elastin binding protein type with a test compound; and measuring the extent of binding of the test compound to the receptor. A test compound shown to have measurable affinity to such a receptor may be a candidate for further testing as a potential compound for use in treating various disorders, such as diabetes and disorders in metabolic syndrome.

Additional aspects relate to a method of identifying a test compound which modulates an elastin binding protein type so as to activate anti-inflammatory activity associated with vascular or endothelial dysfunction. Such a test compound may act as an agonist of a respective elastin binding protein type. Thus, such methodology typically include providing an amino acid sequence comprising a xGxP, GxxP, GxxGP or xGxPG, preferably the SEQ ID NO: 38 (GGGP) or SEQ ID NO: 34 (GGGPG), binding domain of an elastin binding protein type; contacting the elastin binding protein type xGxP, GxxP, GxxGP or xGxPG binding domain of the receptor with a test compound; and measuring the extent of binding of the test compound to the xGxP, GxxP, GxxGP or xGxPG binding domain. Again, any such test compound shown to have measurable affinity to such an elastin binding protein type may be a candidate for additional testing as a compound to promote anti-inflammatory activity useful in the treatment of type I diabetes. Such methods may be cell free high throughput methods. Such methods are particularly advantageous as a first-step screening methods demonstrating that the test compound is capable of binding the elastin binding protein type xGxP, GxxP, GxxGP or xGxPG, preferably the SEQ ID NO: 38 (GGGP) or SEQ ID NO: 34 (GGGPG), binding domain or that the test compound affects binding of a control antibody or a control C-peptide to the elastin binding protein type xGxP, GxxP, GxxGP or xGxPG binding domain. Such assays will measure the binding of a test compound to the elastin binding protein type (such as to the ligand binding domain) or, in other aspects, the response of cells expressing elastin binding protein type or functional fragments thereof. Such methods are especially beneficial in identification of the candidate compounds or test compounds which may be useful as replacements of C-peptides or C-peptides comprising a xGxP, GxxP, GxxGP or xGxPG, preferably the SEQ ID NO: 38 (GGGP) or SEQ ID NO: 34 (GGGPG), binding motif. In different aspects, the presence or the amount of the complex between the candidate compound and the receptor xGxP, GxxP, GxxGP or xGxPG binding domain may be measured. In other aspects, such as cell-based assays, the response of the cell expressing full-length elastin binding protein type or functional fragments thereof may also be measured.

The disclosure further relates to a method of identifying a test compound which modulates an elastin binding protein type so as to activate or suppress anti-inflammatory activity associated with vascular or endothelial dysfunction after C-peptide deficiency, wherein such a method comprises providing a first amino acid sequence comprising at least the xGxP, GxxP, GxxGP or xGxPG preferably the SEQ ID NO: 38 (GGGP) or SEQ ID NO: 34 (GGGPG), binding domain of a elastin binding protein type; contacting the receptor with a control compound such as a control antibody or control C-peptide or fragment or variant thereof and measuring the extent of binding of the control compound to the receptor and/or relevant xGxP, GxxP, GxxGP or xGxPG binding domain in order to determine a baseline binding value. This baseline binding value can be used to compare to binding of a test compound which involves providing a second amino acid sequence comprising an elastin binding protein type; contacting the receptor and/or relevant GxxP, GxxGP or xGxPG binding domain from this second amino acid sequence with the test compound and measuring the extent of binding of the test compound to the receptor xGxP, GxxP, GxxGP or xGxPG binding domain. Thus, the baseline binding value may then be compared to the extent of binding of the test compound.

The methods of the disclosure may also be cell-based. If a cell-based assay is used, such techniques as, for example, cell sorting, may also be used to determine the amount of the complex of interest, such as, for example, the complex between the test compound and the elastin binding protein type xGxP, GxxP, GxxGP or xGxPG binding domain. Thus, assays cells, which are (i) host cells transfected or transformed with an expression vector comprising a elastin binding protein type or biologically relevant fragment (e.g., expressing the xGxP, GxxP, GxxGP or xGxPG binding domain or possibly an elastin binding protein type fusion which expresses at least a portion of the extracellular domain which contains the xGxP, GxxP, GxxGP or xGxPG binding domain); (ii) a host cell line which has been genetically modified to overexpress host elastin binding protein type, preferably resulting in at least a 5-fold increase over expression in a chosen "wild-type" host cell (such improvements of overexpression can be brought about by any means presently known in the art, including but not limited to introducing a promoter by homologous recombination while leaving the coding region intact), and/or (iii) host cells that for whatever biological reason express a high level of the elastin binding protein type (e.g., including but not limited to innate immune cells or fibroblast or smooth muscle cells, pericytes or progenitor cells). Additionally, the methods described herein may be modified such that the assay of interest is carried out in the presence of membrane preparations. The cells, or alternatively, an elastin binding protein type (or biologically relevant fragment) may be utilized to screen test compounds that show affinity for the receptor.

Test compounds identified by the methods described herein preferably act as an agonist or antagonist of the elastin binding protein-type and may be an antibody, an antibody fragment (such as an Fc fragment), a peptide, a protein, a non-proteinaceous organic molecule, ribozyme, and/or anti-sense molecule, any of which may be useful in promoting agonistic or antagonistic activity towards C-peptide activity.

The disclosure relates in part to a compound which acts to modulate a elastin binding protein type (e.g., such as an agonist of the receptor), such that the compound modulates the elastin binding protein type so as to mediate a therapeutically effective signal so as to activate anti-inflammatory activity associated with vascular or endothelial dysfunction after C-peptide deficiency. To this end, the disclosure further relates to a pharmaceutical composition that comprises such a peptide in combination with at least one pharmaceutically effective excipient, such that this pharmaceutical composition is present in a therapeutically effective concentration for administration to a mammal, excluding human uses.

The disclosure also relates to methods of treating one or more disorders related to C-peptide deficiency, such as type-1 diabetes, as disclosed herein, through administration to a mammalian host (excluding huma uses) of a modulator (such as an elastin binding protein type agonist) which activates an elastin binding protein type. Such an elastin binding protein type agonist may be identified through the methods described herein and will be useful in treating disorders, including but not limited type-1 diabetes. In a particular aspects, such an elastin binding protein agonist as provided herein may preferably be used together with an agonist of alpha-enolase (for example a peptide comprising SEQ ID NO: 129 (LALEGSLQ) or the pentapeptide SEQ ID NO: 6 (EGSLQ) or functional parts thereof) or an agonist of GPR 146. The disclosure also provides use of a an agonist of elastin binding protein type for treating type-1 diabetes or a disorder comprising relative or absolute C-peptide deficiency, useful agonists can be selected from xGxP, GxxP, xGxPG, GxxPx, GxxPG, xGxxP, xGxxPx, xGxxPG motif bearing peptides, such as peptides SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 81 (GGIPP), SEQ ID NO: 89 (GIPPA), SEQ ID NO: 90 (GGIPPA) or retro-inverso variants of peptides, such as peptides SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 81 (GGIPP), SEQ ID NO: 89 (GIPPA), SEQ ID NO: 90 (GGIPPA) or SEQ ID NO: 91 (GGYPGASYPGAYPGQAPPGAYPGQAPPGAYPGAPGAYPGAPAPGVYPGPPSGPGAYPS) or SEQ ID NO: 92 (GGYPGASYP) or SEQ ID NO: 93 (GAYPGQAPP) or SEQ ID NO: 94 (GAYPGQA) or SEQ ID NO: 95 (GAYPGAP) or SEQ ID NO: 96 (GAYPG) or SEQ ID NO: 97 (APAPGVYPG) or SEQ ID NO: 98 (GAYPS) or related peptides (or retro-inverso variants thereof).

Also provided is a peptide having a sequence essentially being homologous to a fragment of mammalian insulin C-peptide, the peptide comprising the sequence SEQ ID NO: 32 (LGGGPGAG) or a fragment thereof, or the sequence SEQ ID NO: 33 (LAGGPGAG) or a fragment thereof, and having the ability to interact with elastin binding protein type binding or modulate inflammatory activity of innate immune cells, the disclosure preferably provides a peptide having, most preferably consisting of the sequence SEQ ID NO: 34 (LGGGPG) or SEQ ID NO: 35 (LAGGPG) or a fragment thereof, preferably the fragment is selected from SEQ ID NO: 36 (LGGGP), SEQ ID NO: 37 (LAGGP), SEQ ID NO: 38 (GGGP), SEQ ID NO: 130 (AGGP), and these may be combined into a pharmaceutical composition, for example with insulin of with interleukin-1 receptor antagonist.

Also provided is an isolated or synthetic peptide, essentially being homologous to a fragment of mammalian insulin C-peptide, the peptide comprising the sequence SEQ ID NO: 32 (LGGGPGAG) or a fragment thereof, or the sequence SEQ ID NO: 33 (LAGGPGAG) or a fragment thereof, and having the ability to interact with elastin binding protein type binding or modulate inflammatory activity of innate immune cells, the peptide preferably having, most preferably consisting of the sequence SEQ ID NO: 34 (LGGGPG) or SEQ ID NO: 35 (LAGGPG) or a fragment thereof, preferably wherein the fragment is selected from SEQ ID NO: 36 (LGGGP), SEQ ID NO: 37 (LAGGP), SEQ ID NO: 38 (GGGP), SEQ ID NO: 130 (AGGP) and these may be combined into a pharmaceutical composition, for example with insulin of with interleukin-1 receptor antagonist.

Also provided is a retro-inverso variant of a peptide or fragment relating to the hydrophobic midportion of C-peptide, examples are all-D-amino acid peptides GAGPGGGL, GAGPGGAL, AGPGGGL, GPGGGPA, GPGGAL, GPGGGL, GPGGG, GPGAG and these may be combined into a pharmaceutical composition, for example with insulin of with interleukin-1 receptor antagonist. It is preferred that these retro-inverso variants, preferably for use treatment of microvascular complications in tyope 1 diabetes, are 4 to 8 amino acids in length. These variants are for example provided herein for treating diabetes and/or diabetic complications, or for reducing inflammatory activity, for example in diabetes type 1.

Also provided is a pharmaceutical composition comprising at least one peptide or fragment selected from the group all-D-amino acid peptides GAGPGGGL, GAGPGGAL, AGPGGGL, GPGGGPA, GPGGAL, GPGGGL, GPGGG, GPGAG, together with at least one pharmaceutically acceptable carrier or excipient and the pharmaceutical may further comprise at least one additional active agent effective to combat diabetes or diabetic complications or to treat an inflammatory condition, for example wherein the additional active agent is insulin or an interleukin-1 receptor antagonist or an antibody directed against an interleukin-1, preferably directed against interleukin-1beta. These compositions are provided for use in the treatment of diabetes and diabetic complications or for reducing inflammatory activity or for preparing a medicament for treating diabetes and diabetic complications or for reducing inflammatory activity. Other uses are provided as well, such as wherein the medicament is used for treating type-1 diabetes, optionally with nephropathy, neuropathy or retinopathy or for retarding the development of late type-2 diabetic complications, or wherein the medicament is used for treating an inflammatory condition. The peptide may be used with at least one additional active agent effective to combat diabetes or diabetic complications as a combined preparation for simultaneous, separate or sequential use in the treatment diabetes and/or diabetic complications or with at least one additional active agent effective to rheumatoid arthritis or for preparing a medicament for treating rheumatoid arthritis for treating atherosclerosis or for preparing a medicament for treating atherosclerosis or for the treatment macrovascular disease or for preparing a medicament for the treatment macrovascular disease or for treating osteochondrosis disseccans or for preparing a medicament for treating osteochondrosis disseccans or for treating laminitis or for preparing a medicament for treating laminitis or for treating microvascular disease or for preparing a medicament for treating microvascular disease or for treating metabolic syndrome or for preparing a medicament for treating metabolic syndrome or for treating nephropathy or for preparing a medicament for treating nephropathy or for treating neuropathy or for preparing a medicament for treating neuropathy or for treating retinapathy or for preparing a medicament for treating retinapathy or for treating interleukine-1 mediated inflammation or for preparing a medicament for treating interleukine-1 mediated inflammation.

Also provided is a method for producing a pharmaceutical composition for the prevention and/or treatment of an inflammatory disease, preferably type-1 diabetes, comprising the steps of:
providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one xGxP, PxxG, GPxxG or GPxGx motif, the peptide capable of interacting with an elastin binding protein type
formulating the at least one peptide provided in step a) or a pharmaceutically acceptable salt thereof in a pharmaceutical composition together with at least one anti-diabetic agent such as insulin.

Also provided is a composition obtainable by a method for producing a pharmaceutical composition for the prevention and/or treatment of an inflammatory disease, preferably type-1 diabetes, comprising the steps of: providing at least one peptide consisting of 4-30 amino acids the peptide comprising at least one xGxP, GxxP, GxxPG, xGxPG motif (G being Glycine, P being Proline), or providing a retro-inverso variant peptide comprising at least one xGxP, PxxG, GPxxG or GPxGx motif, the peptide capable of interacting with an elastin binding protein type, formulating the at least one peptide provided in step a) or a pharmaceutically acceptable salt thereof in a pharmaceutical composition together with at least one anti-diabetic agent such as insulin.

Also provided is pharmaceutical composition comprising an anti-diabetic agent, and an peptide consisting of 4-30 amino acids the peptide comprising at least one xGxP, GxxP, GxxPG, or xGxPG motif (G being Glycine, P being Proline), or providing a retro-inverso variant peptide comprising at least one xGxP, PxxG, GPxxG or GPxGx motif, the peptide capable of interacting with an elastin binding protein type, and a pharmaceutically acceptable carrier.

The disclosure also relates to methods of treating one or more disorders related to C-peptide excess, such as insulin resistance, hypertension, atherosclerosis and early phases of type-2 diabetes, as disclosed herein, through administration to a mammalian host (excluding human uses) of a modulator (such as an elastin binding protein type antagonist) which inhibits an elastin binding protein type, either directly, or indirectly by binding to C-peptide and blocking its actions. Such an elastin binding protein type antagonist may be identified through the methods described herein and will be useful in treating disorders, including metabolic syndrome, type-2 diabetes, and related disorders. In a particular aspect, such an elastin binding protein antagonist as provided herein may preferably be used together with an antagonist of alpha-enolase or of GPR 146. Useful elastin binding protein antagonists can be selected from peptides, such as SEQ ID NO: 99 (QTLPGSCGQVVGSPSAQDEASPLSEWRASYNSAGSNITDA), SEQ ID NO: 100 (LPGSCGQVVGSPSAQDEASPLSEWRASYNSAG), SEQ ID NO: 101 (VVGSPSAQDEASPLSEWRASY), SEQ ID NO: 102 (VVGSPSAQDEASPLS), SEQ ID NO: 103 (PSAQDEASPL), SEQ ID NO: 104 (SPSAQDEASP), SEQ ID NO: 105 (AQDEAS), SEQ ID NO: 106 (PSAQ), SEQ ID NO: 107 (SAQD), SEQ ID NO: 108 (DEAS), SEQ ID NO: 31 (QDEA), SEQ ID NO: 109 (SPSA), SEQ ID NO: 110 (VVGGTEAQRNSWPLQ), SEQ ID NO: 111 (VVGGTEAQRNSWPSQ), SEQ ID NO: 112 (TEAQRNSWP), SEQ ID NO: 113 (AQRN), SEQ ID NO: 114 (IVGGRRARPHAWPFM), SEQ ID NO: 115 (VVGGEDAKPGQFPWQ), SEQ ID NO: 116 (VVGGRVAQPNSWPWQ), SEQ ID NO: 117 (RVAQPNSW), SEQ ID NO: 118 (VVGGAEARRNSWPSQ), SEQ ID NO: 119 (AEARRNSW), SEQ ID NO: 120 (VVGGQEATPNTWPWQ), SEQ ID NO: 121 (QEATPNTW), SEQ ID NO: 122 (VVGGEEARPNSWPWQ), SEQ ID NO: 123 (EEARPNSW), SEQ ID NO: 124 (VVGGTEAGRNSWPSQ), SEQ ID NO: 125 (TEAGRNSWP), SEQ ID NO: 126 (EDYRPSQQDECSPRE), SEQ ID NO: 127 (PSQQDECSP), SEQ ID NO: 128 (CODEC), QDE, or related peptides.

Also provided is a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist.

Also provided is a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist the composition intended for the prevention and/or treatment of type-2 diabetes in a mammal.

Also provided is a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist the composition intended for the prevention and/or treatment of atherosclerosis in a mammal.

Also provided is a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist the composition intended for the prevention and/or treatment of rheumatoid arthritis in a mammal.

Also provided is a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist the composition intended for the prevention and/or treatment of osteochondrosis in a mammal.

Also provided is a method for producing a pharmaceutical composition for the prevention and/or treatment of an inflammatory disease, preferably a chronic inflammatory disease, comprising the steps of: providing at least one oligopeptide consisting of 4-30 amino acids the peptide comprising at least one xGxP, GxxP, GxxPG, or xGxPG motif (G being Glycine, P being Proline), or providing a retro-inverso variant peptide comprising at least one PxGx, PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist the composition intended for the prevention and/or treatment of laminitis in a mammal.

It is exemplary that the interleukin 1 receptor antagonist (IL-1Ra) is a recombinant protein (rIL-1Ra), preferably a recombinant human protein (rhIL-1Ra), preferably anakinra.

The disclosure also provides bringing together a first fragment of a C-peptide or fragment thereof with a second fragment of an elastin binding protein or fragment thereof, allowing determining binding and interaction between said two fragments, thereby modeling or modulating binding or interaction of a C-peptide with an elastin binding protein in a proteinaceous substance. In describing a proteinaceous substance herein, reference is made to protein containing material such as an organism or a part thereof, microbial organism, virus, tissue, cell, cell culture, cell culture precipitate, cell culture supernatant, cell content such as cytoplasm, nucleoplasm, nuclei, nucleoli, cell organelles, mitochondria, ribosome, tubuli, plasma, blood, serum, lymph, drainage fluid, and to a protein containing preparation, such as a buffer, dilution, precipitate, extraction, pull-down sample, test sample, spray, chromatographic sample, or a crystal.

The disclosure also provides a proteinaceous substance comprising a first fragment of a C-peptide, preferably said substance having been provided with an isolated first fragment of a C-peptide, alternatively said substance having been selected for the factual presence of said first fragment and comprising a second fragment of an elastin binding protein, preferably said substance having been provided with an isolated second fragment of an elastin binding protein, alternatively said substance having been selected for the factual presence of said second fragment of an elastin binding protein. It is preferred that said C-peptide or peptide or peptide fragment has a sequence as depicted in Table 1, preferably wherein said peptide at least comprises a GxxP or xGxP motif or a retro-inverso variant thereof and said elastin binding protein at least comprises the sequence SEQ ID NO: 100 (LPGSCGQVVGSPSAQDEASPLSEWRASYNSAG). The disclosure also provides a substance according to the disclosure wherein said substance comprises a cell expressing said first fragment and/or said second fragment. Said first fragment preferably be an oligopeptide consisting of 4-30 amino acids the peptide comprising at least one GxPx, GxxP, GxxPG, or xGxPG, preferably SEQ ID NO: 38 (GGGP) or SEQ ID NO: 34 (GGGPG) motif (G being Glycine, P being Proline), or a retro-inverso variant peptide comprising at least one PxGx, PxxG, GPxxG or GPxGx, preferably PGGG or GPGGG, motif, the peptide capable of interacting with an elastin binding protein type.

In a preferred aspect at least one of said fragments has been provided with an affinity tag, such as a tag that has affinity to binding with Protein A or a tag having affinity for binding with streptavidin, for example allowing pull-down experiments or detecting of said fragments in said substance. In a particular aspect the disclosure provides a substance consisting essentially of an isolated first fragment and an isolated second fragment.

The disclosure also provides a proteinaceous substance comprising a first fragment of a C-peptide, preferably said substance having been provided with an isolated first fragment of a C-peptide, alternatively said substance having been selected for the factual presence of said first fragment and comprising a second fragment of an elastin binding protein.

The disclosure also provides a container, preferably a closed container for storing or shipping, provided with a proteinaceous substance comprising a first fragment of a C-peptide (preferably selected from Table 1), preferably having been provided with an isolated first fragment of a C-peptide, and provided with a second fragment of an elastin binding protein, preferably having been provided with an isolated second fragment of an elastin binding protein. In describing a container herein, reference is made to a test device, test tube (commonly Eppendorf tubes are used), test vessel, pipette, pipette tip, reaction device, reaction chamber, cover slip, crystallization chamber, crystallization device, crystallization well, microplate well, crystallization plate well, gel, column wherein, on or under a proteinaceous substance according to the disclosure may be placed or contained or that are useful for storing, shipping, testing or handling a proteinaceous substance provided herein.

The disclosure provides a method of identifying a candidate modulator or test compound or candidate agent as an agent that modulates binding or interaction of a C-peptide with an elastin binding protein, said method comprising providing a a proteinaceous substance comprising a first fragment of a C-peptide, preferably said substance having been provided with an isolated first fragment of a C-peptide, alternatively said substance having been selected for the factual presence of said first fragment and comprising a second fragment of an elastin binding protein in the presence and absence of a candidate modulator under conditions permitting binding of said first fragment with said second fragment. Measuring binding of said first fragment to said second fragment, wherein a decrease or increase in binding in the presence of said candidate modulator, relative to binding in the absence of said candidate modulator, identifies said candidate modulator as an agent that modulates binding or interaction of a C-peptide with an elastin binding protein. It is preferred that said first fragment and/or said second fragment is detectably labeled with a moiety, said moiety preferably selected from the group consisting of a radioisotope, a fluorophore, a quencher of fluorescence, an enzyme, and an affinity tag.

The disclosure also provides a method of detecting, in a sample, the presence of an agent that modulates binding or interaction of a C-peptide with an elastin binding protein comprising providing a proteinaceous substance comprising a first fragment of a C-peptide, preferably said substance having been provided with an isolated first fragment of a C-peptide, alternatively said substance having been selected for the factual presence of said first fragment and comprising a second fragment of an elastin binding protein with a sample under conditions permitting binding of said first fragment with said second fragment. Measuring binding of said first fragment to said second fragment, wherein a decrease or increase in binding in the presence of said sample, relative to binding in the absence of said sample, identifies said sample as comprising an agent that modulates binding or interaction of a C-peptide with an elastin binding protein. It is preferred that said first fragment and/or said second fragment is detectably labeled with a moiety, said moiety preferably selected from the group consisting of a radioisotope, a fluorophore, a quencher of fluorescence, an enzyme, and an affinity tag.

The disclosure also provides an *in vitro* or *ex vivo* chemotaxis method of identifying an agent suitable for therapy of metabolic syndrome or diabetes or a disorder of metabolic syndrome selected from: atheromatous disease, atherosclerosis, arteriosclerosis, coronary heart disease, impaired glucose tolerance, insulin resistance, restenosis, stroke, angina pectoris, hypertension, transient ischemic attacks (TIA) or peripheral artery disease (PAD), comprising the step of determining whether a candidate agent affects serum C-peptide vascular cell chemotaxis controlling activity of the elastin binding protein, wherein C-peptide is a peptide identifiable with Uniprot identifier P01308[57-87] (amino acid sequence SEQ ID NO: 1 (EAEDLQVGQVELGGGPGAGSLQPLALEGSLQ), or a sequence with at least 55%, preferably at least 65%, more preferably at least 75%, more preferably at least 85%, more preferably at least 95% sequence identity thereto, and wherein the elastin binding protein comprises a peptide identifiable with Uniprot identifier P16278-2 containing amino acid sequence SEQ ID NO: 100 (LPGSCGQVVGSPSAQDEASPLSEWRASYNSAG), or containing a sequence with at least 70%, preferably at least 75%, more preferably at least 80%, more preferably at least 85%, more preferably at least 95% sequence identity to said amino acid sequence. In another aspect, the disclosure provides an *in vitro* or *ex vivo* chemotaxis method wherein said vascular cell is an inflammatory cell, preferably a white blood cell such as a leukocyte, a monocyte or a lymfocyte. In another aspect, the disclosure provides an *in vitro* or *ex vivo* chemotaxis method wherein the agent is an antagonist that inhibits the serum C-peptide vascular cell chemotaxis controlling activity of the elastin binding protein. In another aspect, the disclosure provides an *in vitro* or *ex vivo* chemotaxis method wherein the agent is an agonist that enhances the serum C-peptide vascular cell chemotaxis controlling activity of the elastin binding protein. In another aspect, the disclosure provides an *in vitro* or *ex vivo* chemotaxis method wherein the elastin binding protein is contacted with the candidate agent to determine whether the candidate affects the serum C-peptide vascular cell chemotaxis controlling activity of the elastin binding protein. In another aspect, the disclosure provides an *in vitro* or *ex vivo* chemotaxis method wherein C-peptide is contacted with the candidate agent to determine whether the candidate affects the serum C-peptide vascular cell chemotaxis controlling activity of the elastin binding protein. In another aspect, the disclosure provides an *in vitro* or *ex vivo* chemotaxis method wherein the candidate agent is contacted with a cell expressing an elastin binding protein. In another aspect, the disclosure provides an *in vitro* or *ex vivo* chemotaxis method wherein the candidate agent is contacted with a cell expressing a C-peptide. In a preferred aspect, the disclosure provides an *in vitro* or *ex vivo* chemotaxis method wherein the candidate agent is contacted with a cell expressing a C-peptide wherein the step of determining whether the agent affects the activity of the elastin binding protein is carried out on a sample obtainable from an animal expressing the C-peptide having been administered with the candidate agent, and determining from the sample whether the animal exhibits altered C-peptide levels and/or alterered insuline resistance, it is preferred that said animal expresses a functional elastin binding protein. The disclosure also provides a non-human transgenic animal having a functionally-disrupted endogenous preproinsuline gene, wherein the gene is encoding a C-peptide as defined above. It is preferred that said animal has a mutation replacing the proline (P) situated in P01308 at position 72 with another amino acid, such as a leucine (L). In another aspect, the disclosure provides a non-human transgenic animal having a deletion in a gene encoding for C-peptide at least deleting the proline (P), situated in P01308 at position 72. In another preferred aspect, the disclosure provides an *in vitro* or *ex vivo* chemotaxis method wherein the candidate agent is contacted with a cell expressing an elastin binding protein wherein the step of determining whether the agent affects the activity of the elastin binding protein is carried out on a sample obtainable from a non-human animal expressing the elastin binding protein having been administered with the candidate agent, and determining from the sample whether the animal exhibits altered C-peptide levels and/or alterered insuline resistance, it is preferred that said non-human animal expresses a functional elastin binding protein. The disclosure also provides a non-human transgenic animal having a functionally-disrupted endogenous elastin binding protein gene, wherein the gene is encoding an elastin binding protein as defined above.

The disclosure also provides a laboratory animal, preferably a mouse or a rat or a fat sand rat or a naked mole rat, provided with a gene construct allowing overexpression of C-peptide or fragment therof. The injection of a recombinant adenoviral vector into the tail vein of a mouse or rat results in highly preferential infection of the liver and subsequent liver-specific expression of the gene construct encoding the C-peptide (fragment) of interest that is inserted into the adenoviral backbone. These characteristics of systemic adenovirus injection, and the fact that adenoviral vectors are relatively easy to generate and amplify to high titers, provide an exquisite and extremely powerful means to investigate the effects of liver-specific expression of the gene construct encoding the C-peptide of interest. Moreover, since any transgenic mouse model can be injected with adenoviral vectors, this technology allows rapid analysis of (trans)gene-gene interaction.

The disclosure also provides use of an isolated fragment of a C-peptide as an agent that modulates binding or interaction of a C-peptide with an elastin binding protein and use of an isolated fragment of an elastin binding protein as an agent that modulates binding or interaction of a C-peptide with an elastin binding protein.

Also provided is a composition obtainable by a method for producing a pharmaceutical composition for the prevention and/or treatment of an inflammatory disease, preferably a chronic inflammatory disease, comprising the steps of: providing at least one peptide consisting of 4-30 amino acids the peptide comprising at least one GxPx, GxxP, GxxPG, or xGxPG motif (G being Glycine, P being Proline), or providing a retro-inverso variant peptide comprising at least one PxxG, GPxxG or GPxGx motif, the peptide capable of interacting with an elastin binding protein type, formulating the at least one peptide provided in step b) or a pharmaceutically acceptable salt thereof in a pharmaceutical composition together with at least one interleukin-1 receptor antagonist.

Useful antagonists to be included in a combination medicine can be can be selected from GxxP, xGxPG, GxxPx, GxxPG, xGxxP, xGxxPx, xGxxPG motif binding peptides, such as SEQ ID NO: 99 (QTLPGSCGQVVGSPSAQDEASPLSEWRASYNSAGSNITDA), SEQ ID NO: 100 (LPGSCGQVVGSPSAQDEASPLSEWRASYNSAG), SEQ ID NO: 101 (VVGSPSAQDEASPLSEWRASY), SEQ ID NO: 102 (VVGSPSAQDEASPLS), SEQ ID NO: 103 (PSAQDEASPL), SEQ ID NO: 104 (SPSAQDEASP), SEQ ID NO: 105 (AQDEAS), SEQ ID NO: 106 (PSAQ), SEQ ID NO: 107 (SAQD), SEQ ID NO: 108 (DEAS), SEQ ID NO: 31 (QDEA), SEQ ID NO: 109 (SPSA), SEQ ID NO: 110 (VVGGTEAQRNSWPLQ), SEQ ID NO: 111 (VVGGTEAQRNSWPSQ), SEQ ID NO: 112 (TEAQRNSWP), SEQ ID NO: 113 (AQRN), SEQ ID NO: 114 (IVGGRRARPHAWPFM), SEQ ID NO: 115 (VVGGEDAKPGQFPWQ), SEQ ID NO: 116 (VVGGRVAQPNSWPWQ), SEQ ID NO: 117 (RVAQPNSW), SEQ ID NO: 118 (VVGGAEARRNSWPSQ), SEQ ID NO: 119 (AEARRNSW), SEQ ID NO: 120 (VVGGQEATPNTWPWQ), SEQ ID NO: 121 (QEATPNTW), SEQ ID NO: 122 (VVGGEEARPNSWPWQ), SEQ ID NO: 123 (EEARPNSW), SEQ ID NO: 124 (VVGGTEAGRNSWPSQ), SEQ ID NO: 125 (TEAGRNSWP), SEQ ID NO: 126 (EDYRPSQQDECSPRE), SEQ ID NO: 127 (PSQQDECSP), SEQ ID NO: 128 (QQDEC), QDE, or related peptides and these may be combined into a pharmaceutical composition, for example with insulin of with interleukin-1 receptor antagonist.

Described herein are methods of doing business. Such methods may comprise one or more of: receiving a request or an order for a composition, substance or animal described hererein from a customer; receiving a request or an order from a customer for a compositon, substance or animal identified by a method described herein; receiving a request to practice a method described herein from a customer; delivering a composition, substance or animal described herein, preferably contained in a container described herein; delivering a composition, substance or animal identified by a method described herein, preferably contained in a container described herein providing a license or the right to practice a method described herein; providing a license or the right to make and/or sell a composition, substance or animal described herein; providing a license or the right to make and/or sell a composition, substance or animal identified by a method described herein; delivering instructions on the practice of a method described herein; receiving payment from a customer; and combinations of the foregoing.

In aspects, compositions, or substances or animal may be labelled with or accompanied by literature indicating or describing the use of the composition, substance for the development of drugs or treatment of one or more of diabetes, diabetic complications, inflammatory conditions, rheumatoid arthritis, atherosclerosis, macrovascular disease, osteochondrosis disseccans, laminitis, microvascular disease, metabolic syndrome, nephropathy, neuropathy, retinopathy, or reducing inflammatory activity.

### DETAILED DESCRIPTION

C-peptide is found a ligand of the elastin receptor.

| | |
|---|---|
| Elastin receptor | shall mean a chemical group or molecule on the cell surface or in the cell interior that has an affinity for a peptide having an amino acid motif GxxP, wherein G represents the one-letter code for the amino acid glycine, P for the amino acid proline and x for any amino acid, the amino acid following P preferably allowing for a type VIII-beta turn, a condition which is met when P is C-terminally followed by a G, said elastin receptor typically represented in humans by the elastin binding protein known in the publicly accessible database Uniprot as GLB1 - isoform 2 under identifier: P16278-2. |
| C-peptide | shall mean a peptide typically produced by beta-cells in the pancreas together with insulin, said C-peptide represented in humans by the peptide known in the publicly accessible database Uniprot as INS - isoform 1 under identifier: P01308-1, position 57-87. |

C-peptide, connecting immature insulin chains A and B and secreted in a 1:1 ratio with mature insulin into the portal circulation, has traditionally been considered inert, despite ever increasing evidence of its biological activity. I show that in dietary excess, excess serum C-peptide leads to chronic-low grade inflammation, insulin resistance and hypertension and is causal to metabolic syndrome. I show C-peptide carrying a hitherto unrecognized xGxxPG motif specific for binding of elastin peptides to the elastin receptor, said receptor fulfilling various roles in tissue inflammation and tissue repair. Recent findings show this receptor to promote insulin resistance, dislipidaemia, hypertension and atherogenesis, all characteristic of metabolic syndrome. This finding takes C-peptide into the limelight, tying in metabolic syndrome with other conditions of insulin resistance, such as COPD, when circulating elastin-derived peptides may combine with C-peptide to stimulate elastin receptor-mediated insulin resistance and inflammation.

### Insulin resistance

Insulin resistance (IR) is central to metabolic syndrome ^{1,2}. It occupies a crucial place in the aetiology of chronic inflammatory, lifestyle-, diet- or age-related, conditions as atherosclerotic cardiovascular disease and diabetes type 2. Hallmarks of metabolic syndrome are IR, hypertension, dyslipidaemia, hyperinsulinaemia, and impaired glucose tolerance. Uncertainties exist to the cause of IR. Simplified, the main view ^{1,3} holds chronic-low grade inflammation to drive IR and subsequent hyperinsulinaemia; a seemingly opposed view ² holds increased hyperinsulinaemia to drive IR and subsequent inflammation.

In humans in dietary excess, excess serum C-peptide causes chronic-low grade inflammation as well as IR and hypertension leading to metabolic syndrome, C-peptide being hitherto unrecognized as a ligand for the elastin receptor.

### The elastin receptor

The elastin receptor ⁴⁻⁶ is involved in chemotaxis of leukocytes and activation of matrix-metallo-proteinases, in endothelial cell migration and angiogenesis and in proliferation of fibroblasts and vascular smooth-muscle cells. The receptor is activated by (proteolytic) fragments of extracellular matrix in granulating tissue after tissue injury or inflammation, fulfilling handyman jobs towards tissue repair.

The receptor consists of an alternatively spliced variant of beta-galactosidase. It binds to a hexapeptide x-Gly-x-x-Pro-Gly (xGxxPG) motif in (proteolytic fragments of) extracellular matrix proteins such as elastin and fibrillin-1 ⁴. The best-known representative of the motif is hexapeptide SEQ ID NO: 41 (VGVAPG) found in (tropo)elastin, but many other biologically active peptides conforming to the signature sequence xGxxPG, generally called elastin peptides, have been reported as agonist ^{4,5}. A minimally essential sequence for biological activity is GxxP, with the peptide at P adopting a type VIII beta-turn ⁵. Lactose and V14 peptide (SEQ ID NO: 131 (VVGSPSAQDEASPL)) corresponding to the binding site of the receptor, is used to antagonise elastin peptide binding ⁶.

The elastin receptor forms a complex with neuraminidase (Neu-1) and protective protein-cathepsin A (PPCA) on the cell surface ⁴. After binding to its ligand, the complex internalises to endosomal compartments in the cell and triggers numerous cellular responses. In mice, elastin peptides potentiate atherosclerosis through Neu-1 ⁷ and regulate IR ⁸ due to an interaction between Neu-1 and the insulin receptor. Moreover, in mice, PPCA is required for assembly of elastic fibres and inactivation of endothelin-1, impaired activation of endothelin-1 resulting in hypertension ⁹.

### C-peptide

Herein recognized, C-peptide (₁SEQ ID NO: 1 (EAEDLQVGQVELGGGPGAGSLQPLALEGSLQ)₃₁) contains the xGxxPG motif, surprisingly identifying it as a ligand for the elastin receptor. The implications of that find are discussed below. Classically, C-peptide connects the A- and B-chain of insulin in the preproinsulin produced in pancreatic beta-cells from the insulin gene and facilitates folding and binding of chains A en B. After processing, mature insulin and C-peptide are secreted into the portal circulation. Be it under dietary frugality or excess, insulin and C-peptide are produced and secreted in equimolar concentrations. However, C-peptide's plasma half-life of ~30 min versus insulin's half-life of ~4 min ¹⁰ causes dietary excess to maintain persistently higher levels of circulating C-peptide than of insulin. The traditional view holds circulating C-peptide essentially inert and, because of its longer half-life, particularly useful as a surrogate marker of insulin release. However, accumulating evidence points at biological functions for C-peptide ¹¹⁻¹⁴. Excess C-peptide in mice experimentally elicits inflammatory effects in vasculature and around glomeruli and C-peptide is found deposited in atherosclerotic lesions of patients ¹⁵. Fasting serum C-peptide levels significantly relate to hazards of cardiovascular and overall death in non-diabetic adults ¹⁶. These recent findings establish pathophysiological importance to C-peptide in its own right.

### C-peptide receptor

Until now, a distinct C-peptide receptor is unknown. A binding study ¹² of C-peptide to human cell membranes indicates the existence of at least two C-peptide/receptor complexes, one with high affinity and low mobility and one with low affinity and high mobility and recent studies suggest alpha-enolase ¹⁷, a cell surface receptor of plasminogen, or GPR146 ¹⁸, associated with dyslipidaemia ¹⁹, as possible receptor candidates for C-peptide. Biologically active sites in C-peptide. At least two biologically active sites have been identified in the C-peptide.

### Pentapeptide ₂₇ SEQ ID NO: 6 (EGSLQ) ₃₁

A first concerns the pentapeptide ₂₇ SEQ ID NO: 6 (EGSLQ) ₃₁, corresponding to the C-terminal five residues of C-peptide, which mimics several effects of the full-length peptide. The pentapeptide displaces cell membrane-bound C-peptide, increases intracellular Ca(2+) and stimulates MAP kinase signalling pathways and Na(+),K(+)-ATPase ⁸. Of note, the glutamate at position 27 was shown essential to activation of alpha-enolase by C-peptide ¹⁴, hinting that the C-terminal pentapeptide site may be involved in interaction of C-peptide with alpha-enolase.

### Midportion ₁₃ SEQ ID NO: 8 (GGGPGAG) ₁₉

A second site, and main focus of this application, the midportion of C-peptide 13 SEQ ID NO: 8 (GGGPGAG) ₁₉, was detected when structural features of C-peptide critical for mediating its effects on vascular dysfunction were investigated in a skin chamber granulation tissue model in rats ¹⁴. ₁₃ SEQ ID NO: 8 (GGGPGAG) ₁₉ was shown to be central to C-peptide's biological activity. However, as synthetic reverse sequence (retro) and all-D-amino acid (enantio) C-peptides were found equipotent to native C-peptide, it was concluded ¹⁴ that the effects of this midportion must rely on non-chiral interactions, thereby teaching away from any possible stereospecific receptor binding to ₁₃ SEQ ID NO: 8 (GGGPGAG) ₁₉. This teaching has since then dominated the literature on C-peptide. However, I here conclude that an xGxxPG elastin receptor binding motif is overlapping with C-peptide's bioactive midportion in SEQ ID NO: 1 (EAEDLQVGQVELGGGPGAGSLQPLALEGSLQ), distinctly associated with effects on vascular function in granulation tissue, identifying C-peptide as a biologically active ligand of the elastin receptor.

### Non-chirality is revoked

Surprisingly, studying reference 14 anew, the xGxxPG motif is also present in the biologically active retro C-peptide SEQ ID NO: 136 (QLSGELALPQLSGAGPGGGLEVQGVQLDEAE). Also, the biologically active enantio C-peptide (D--EAEDLQVGQVELGGGPGAGSLQPLALEGSLQ) carries the motif, being hidden as the retro-enantio sequence D--GPGAGS; retro-enantio peptides being stereometrically nearly identical as their parent peptides, maintaining overall side-chain topology albeit for different N-terminal and C-terminal endings ²⁰. These observations revoke the teaching ¹⁴ of non-chirality and instead allow for stereospecific binding of these peptides to a receptor recognising said motif: the elastin receptor.

C-peptide is a species of the genus of elastin peptides.

Moreover, additional examples of fragments of the C-peptide are provided herein and IDO et al., all bearing a midportion hexapeptide ₁₂ SEQ ID NO: 34 (LGGGPG) ₁₇, that all prevented vascular dysfunction whereas other C-peptide fragments, wherein the hexapeptide midportion was disrupted, were found not active. Rat C-peptide, comprising a hexapeptide (₁₂ SEQ ID NO: 134 (LGGGPE) ₁₇) GxxP motif (the P allowing a type VIII beta-turn required for biological activity ⁵) was found active as well, whereas pig C-peptide (midportion ₁₂ SEQ ID NO: 135 (LGGGLG) ₁₇ ) not containing the essential P in the elastin binding motif, was found inactive. Of note, all 11 C-peptide(fragments) with the GxxP motif prevented vascular dysfunction, whereas all 5 without the motif did not, showing that even fragments of circulating C-peptide may contribute to elastin receptor activation, as long as the GxxP motif and the type VIII beta-turn is present. C-peptide and its xGxxPG containing fragments may thus be considered an unexpected species of the genus of a larger class of peptides: elastin peptides capable of elastin receptor activation, whereby excess C-peptide may be meddling with elastin receptor mediated tissue repair, modulating chronic-low grade inflammation, IR and hypertension. Insulin resistance extends beyond metabolic syndrome. Based on the above, I pose that in humans and in companion animals in dietary excess and prone to develop metabolic syndrome excess C-peptide binds to the elastin receptor, eliciting three effects, chronic-low grade inflammation (rather to be seen as excess vascular repair activity), IR and hypertension. The finding ties together conditions seen with metabolic syndrome with conditions possibly caused by circulating elastin degradation products, such as COPD, caused by smoking or by exposure to fine particulate matter, or by physiological conditions, such as pregnancy and growth; allowing for a cumulative pathology when both C-peptide and elastin-derived peptides are increased, which provides a substantial jump in our understanding of the causes of metabolic syndrome and other lifestyle- or age-related conditions of IR. Elastin peptide/elastin receptor binding has been demonstrated for synthetic peptides such as SEQ ID NO: 41 (VGVAPG) and inhibited by antagonist lactose and by antagonist V14 peptide ⁴⁻⁶. It is provided to redo these tests with synthetic C-peptide variants or fragments, provided with or without the sequence GxxP, to study binding, including two classical elastin receptor antagonist V14 peptide to study inhibition of binding. Similarly, one can do the C-peptide tests in a skin chamber granulation tissue model of vascular function ¹⁴, or test synthetic, inducibly or constitutively expressed C-peptide in established models of atherosclerosis, Neu-1 mediated IR or PPCA mediated hypertension ⁷⁻⁹. For example, in a classical Boyden chamber experiment, an 100% increase of migration of CD4+ immune cells in 1% serum medium was demonstrated in vitro by C-peptide at 10 nM which CD4-migration was then antagonized and diminished by >50% by V14-peptide at 1.3 microM, specifically demonstrating reduction of C-peptide specific biological activity by elastin receptor antagonist V14 peptide as well as by elastin receptor antagonist lactose.

### REFERENCES INCLUDED HEREIN

1 Shoelson SE, Lee J, Goldfine AB. J Clin Invest. 2006; 116:1793-1801*.*
2 Shanik MH, Xu Y, Skrha J, Dankner R, Zick Y, Roth J. Diabetes Care. 2008; Suppl 2:5262-8
3 Bolton CE, Evans M, lonescu AA, et al.,. COPD. 2007; Jun; 4(2):121-6.
4 Adair-Kirk TL, Senior RM. Int J Biochem Cell Biol. 2008;40(6-7):1101-10.
5 Blanchevoye et al,idoi: 10.1074/jbc.M112.419929 *jbc.M112.419929*
6 Robinet A, Fahem A, Cauchard JH, et al., J Cell Sci. 2005; Jan 15;118(Pt 2):343-56.
7 Gayral S, Garnotel R, Castaing-Berthou A, et al., Cardiovasc Res. 2013; Dec 19. [Epub ahead of print]
8 Blaise S, Romier B, Kawecki C, et al., Diabetes. 2013; Nov;62(11):3807-16.
9 Seyrantepe V, Hinek A, Peng J, et al.,. Circulation. 2008; Apr 15;117(15):1973-81.
10 Faber OK, Hagen C, Binder C, eta al., J Clin Invest 1978; 62 : 197-203
11 Hills CE, Brunskill NJ. Clin Sci (Lond). 2009; Apr;116(7):565-74.
12 Rigler, R., Pramanik, A. and Jonasson, P. Proc. Natl. Acad. Sci. U.S.A.1999; 96, 13318-13323
13 Johansson J, Ekberg K, Shafqat J, et al., Biochem Biophys Res Commun. 2002; Aug 2;295(5):1035-40.
14 Y, Ido Vindigni A, Chang K, et al., Science. 1997; Jul 25;277(5325):563-6.
15 Vasic D, Walcher D. Int J Inflam. 2012; 2012:932725.
16 Patel N, Taveira TH, Choudhary G, Whitlatch H, Wu WC. J Am Heart Assoc. 2012; Dec;1(6):e003152.
17 Ishii T, Fukano K, Shimada K, et al., J Biochem. 2012; Jul;152(1):53-62.
18 Yosten GL, Kolar GR, Redlinger LJ, Samson WK. J Endocrinol. 2013;218(2):B1-8
19 *International Patent Application* PCT/GB2006/003759 2005;
20 Muller S, Benkirane N, Guichard G, Van Regenmortel MH, Brown FExpert Opin Investig Drugs; 1998 Sep;7(9):1429-38.

Again, when we eat too much, we provide the beta-cells of our pancreas with continued glucose signaling to produce insulin, to harbor the ever-excess glucose derived from our food in peripheral liver, muscle and fat cells. When eating in excess, we demand ever increasing insulin production from our beta-cells, and therewith demand ever increasing production of C-peptide from our beta-cells, C-peptide and insulin being produced and excreted in equal amounts. As insulin has a typical half-life of about 4 minutes, conditions of excess insulin may be easily coped with. However, C-peptide has a much longer half-life, typically of 30 minutes, and depositions of excess C-peptide (and of partly or whole unprocessed pro-insulin) will be formed around the rim of the beta-cells and the islets of Langerhans, and also in the vascular wall of our blood-vessels. Pericytes, smooth muscle cells, fibroblasts, adipose tissue cells, pancreatic stellate cells, and others, together with endothelial cells, and possibly circulating leucocytes, respond to binding of EBP to GxxPG bearing C-peptide, thereby causing matrix-metallo-proteinase (MMP) induced hydrolysis accompanied by interleukin-1beta mediated proliferation and subsequent low-grade inflammatory activation, in and around beta-cells in the islets of Langerhans. IL-1beta thus drives tissue inflammation that impacts on both beta-cells functional mass and subsequently may also drive insulin sensitivity in type-2 diabetes. Binding of EBP to C-peptide's GxxPG sequences may further facilitate shedding of EBP from cellular surfaces and increased presentation of the interleukin-I receptor, allowing for a continued interleukin-1beta mediated proliferation and inflammatory activation wherever C-peptide deposits are present, again driving insulin sensitivity in type-2 diabetes. In a patient thus developing diabetes type-2 or metabolic syndrome damage to and destruction of the beta-cells in the pancreas is following excess C-peptide production by those cells. Phenomena commonly seen as insulin resistance are then often secondary to initial events in the pancreatic beta-cells and arise out of interaction of fibroblasts, smooth muscle cells pericytes and leucocytes with vascular or peripheral C-peptide overload.

C-peptide exerts chemotactic and bioactive effects via interaction of its GXXPG and XGXPG motif with the elastin-binding protein. We here in this patent application teach that C-peptide exerts chemotactic and bioactive influence on monocytes, pericytes, smooth muscle cells, fibroblasts, and other cells via interaction with the elastin-binding protein (EBP) (Privitera et al., J. Biol. Chem. 1998; 273:6319-6326). This receptor recognizes Gly-X-X-Pro-Gly (XGXXPG) or X-Gly-X-Pro-Gly (XGXPG) motifs found in C-peptides, wherein X can be any amino acid, and preferably a hydrophobic amino acid. The identity of this receptor protein, commonly called the elastin binding protein (EBP), has been established as an enzymatically inactive, alternatively spliced variant of beta-galactosidase. EBP forms a complex with protective protein/cathepsin A (PPCA) and lysosomal sialidase (neuraminidase-1, Neu-1). As C-peptide is released in equimolar concentrations together with insulin, but has a much longer half-life, increased insulin excretion as result of increased food-intake will result in even higher C-peptide levels. This evokes an oversupply of C-peptide and deposits of the C-peptide are observed in the periphery of beta-cells and even in the (micro)vasculature where these C-peptide deposits evoke the low-grade inflammation so typical of what is commonly called insulin-resistance. GXXPG and XGXPG motif binding to the EBP induces interleukin-lbeta mediated proliferation of vascular and connective tissue cells.

Pericytes, smooth muscle cells, fibroblasts, adipose tissue cells, pancreatic stellate cells, and others, together with endothelial cells, and circulating leucocytes, respond to binding of EBP to GxxPG or xGxPG bearing proteins and peptides by interleukin-1beta mediated proliferation and low-grade inflammatory activation. Analysis of the human proteome shows that proteins with multiple GxxPG or xGxPG motifs are highly related to the extracellular matrix (ECM). Matrix proteins with multiple GxxPG or xGxPG sites include fibrillin-1, -2, and -3, elastin, fibronectin, laminin, and several tenascins and collagens.

Recent studies have shown that the Neu-1 component of the EBP complex is responsible for triggering cellular activation. EBP is present on many cell types, including various types of leukocytes, mesenchymal cells, vascular smooth muscle cells, and skin fibroblasts. Whereas the hexapeptide SEQ ID NO: 41 (VGVAPG), a commonly repeated sequence in elastin, is the most well-recognized ligand for this receptor, C-peptide, galectin-3, the amino acid sequence SEQ ID NO: 25 (FRAAPLQGMLPGLLAPLRT) in collagen 6 A3 (COL6A3, Uniprot identifier P1211) and the beta-2 loop of choriogonadotropin (hCG) are now herein also recognized as also capable of binding to the EBP. In addition to SEQ ID NO: 41 (VGVAPG), (all elastin-derived) peptides that follow the motif GXXPG or XGXPG (where X is a hydrophobic amino acid) display chemotaxis for monocytes in vitro (Bisaccia F, et al., Int. J. Pept. Protein Res. 1994 ;44:332-341, Castiglione Morelli MA, et al., J. Pept. Res. 1997 ;49:492-499). This is noteworthy, albeit not having been observed before, because primate C-peptide sequences do not contain the SEQ ID NO: 41 (VGVAPG) sequence; however, primate C-peptide contain significant quantities of both GXXP, GXXPG and XGXPG motifs that show similar activities. C-peptide's GxxP, GxxPG and xGxPG interactions explain IL-1beta involvement. C-peptide's GxxP, GxxPG and xGxPG interactions have until now been overlooked by those skilled in the art of diabetes or metabolic disorder research as well as by those skilled in the art of elastin peptide and extracellular matrix (ECM) research. This earlier unobserved fact explains the macrophage-predominant, IL-1beta mediated chronic inflammatory disease process as seen in for example adipose tissue in patients suffering from diabetes type-2, it explains the intima thickening and smooth muscle cell proliferation seen in vessels of patients suffering from atherosclerosis, the direct insulitis and peri-islet inflammation around beta cells in the pancreas as seen in the early phases of diabetes, and many other disease manifestations of metabolic syndrome wherein the patients suffer from C-peptide overproduction and C-peptide deposits, likely as a consequence of over-eating. C-peptide's GxxP, GxxPG and xGxPG interactions also explain leucocyte involvement. In addition, IL-1beta signaling results in the production of pro-inflammatory mediators that act in a feed-forward autocrine/paracrine manner in beta-cells and local innate immune cells to amplify these effects, amplified by the fact that circulating leucocytes show strong chemotaxis to GxxPG or xGxPG bearing proteins and peptides; again C-peptide will thus attract those cells to wherever C-peptide is present, and in situations of C-peptide overload or even C-peptide deposits, this will exacerbate disease. As indicated herein, the concept that C-peptide and degradation products thereof can drive a macrophage-predominant, chronic inflammatory disease process via its GxxPG and xGxPG motif is now elucidating the etiology of diabetes of all types and is applicable to all diseases that occur in vasculature-rich organs and tissues, including coronary artery disease, peripheral vascular disease, and aortic aneurysm.

By "peptide" the inventor includes not only molecules in which amino acid residues are joined by peptide (-CO-NH-) linkages but also functionally equivalent molecules in which the peptide bond is reversed. Retro-inverse peptides are composed of D-amino acids assembled in a reverse order from that of the parent L-sequence, thus maintaining the overall topology of the native sequence. Such retro-inverso peptidomimetics may be made using methods known in the art, for example such as those described in Meziere et al. (1997) J. Immunol. 159, 3230-3237, and Carver et al. (1997) Biopolymers. 1997 Apr 15; 41(5):569-90. This approach involves making pseudopeptides containing changes involving the backbone, and not the orientation of side chains. Meziere et al. and Carver et al. (1997) show that these pseudopeptides are useful. Retro-inverse peptides are much more resistant to proteolysis. Retro-inversion is a way of protecting peptide substances against proteolysis. It entails retro-inverting those peptide bonds most susceptible to enzymatic hydrolysis by inverting the direction of the peptide bonds. The "retro-inverso peptides" are structural isomers of the reference peptides and as such preserve their biological activity while being more resistant to enzymatic hydrolysis. A peptidomimetic is a small protein-like chain designed to mimic a peptide. They typically arise from modification of an existing peptide in order to alter the molecule's properties). Chemically synthesized peptides generally have free N- and C-termini. N-terminal acetylation and C-terminal amidation reduce the overall charge of a peptide; therefore, its overall solubility might decrease. However, the stability of the peptide could also be increased because the terminal acetylation/amidation generates a closer mimic of the native protein. These modifications might increase the biological activity of a peptide and are herein also provided.

Anti-elastin receptor antibody, specifically directed against the 67 kDa elastin receptor, alternatively spliced beta-galactosidase is produced as follows. Briefly, a peptide, SEQ ID NO: 131 (VVGSPSAQDEASPL) corresponding to the unique sequence to the alternatively spliced form of human beta-galactosidase is synthesized, e.g. by a solid phase procedure, then injected intraperitoneally into a mammal, such as a rabbit. Rabbit serum is harvested at 5-8 weeks after injection. Antibody was purified from the anti-serum by protein A-TSK gel (Amersham). The peptide-agarose affinity gel is prepared by coupling the peptide SEQ ID NO: 131 (VVGSPSAQDEASPL) to Affi-Gel 15 (Bio-Rad Laboratories, Richmond, CA) according to the manufacturer's protocol. The peptide-specific antibody is eluted with 0.1 M citrated buffer, pH 3.0. After neutralization, the antibody (designated as anti-S-Gal) is preferably dialyzed overnight with phosphate-buffered saline (pH 7.4). The antibody is used in competitive binding assays to test candidate drug compounds for their capacity to interfere with binding of C-peptide or elastine peptide with the elastine receptor.

Anti-C-peptide antibody, specifically directed against the C-peptide, preferably directed against the PG-domain or GxxP comprising fragments therof is produced as follows. Briefly, a C-peptide or PG-domain or GxxP containing fragment thereof, preferably having a sequence as selected from Table 1, is synthesized by a solid phase procedure described above, then injected intraperitoneally into a mammal, such as a rabbit. Rabbit serum is harvested at 5-8 weeks after injection. Antibody is purified from the anti-serum by protein A-TSK gel (Amersham). The peptide-agarose affinity gel was prepared by coupling C-peptide to Affi-Gel 15 (Bio-Rad Laboratories, Richmond, CA) according to the manufacturer's protocol. The peptide-specific antibody is eluted with 0.1 M citrated buffer, pH 3.0. After neutralization, the antibody (designated as anti-C-peptide) is preferably dialyzed overnight with phosphate-buffered saline (pH 7.4). The antibody is used in competitive binding assays to test candidate drug compounds for their capacity to interfere with binding of C-peptide or elastine peptide with the elastine receptor.

Anti-elastine-peptide antibody, specifically directed against the elastin-peptide or fragments therof is produced as follows. Briefly, an elastin-peptide or PG-domain or GxxP containing fragment thereof, is synthesized by a solid phase procedure described above, then injected intraperitoneally into a mammal, such as a rabbit. Rabbit serum is harvested at 5-8 weeks after injection. Antibody is purified from the anti-serum by protein A-TSK gel (Amersham). The peptide-agarose affinity gel was prepared by coupling elastin-peptide to Affi-Gel 15 (Bio-Rad Laboratories, Richmond, CA) according to the manufacturer's protocol. The peptide-specific antibody is eluted with 0.1 M citrated buffer, pH 3.0. After neutralization, the antibody (designated as anti-elastin-peptide) is preferably dialyzed overnight with phosphate-buffered saline (pH 7.4). The antibody is used in competitive binding assays to test candidate drug compounds for their capacity to interfere with binding of C-peptide or elastine peptide with the elastine receptor. Peptides are optionallty purified and desalted using reversed phase (RP) micro-columns (Applied Biosystems) prior to nanoLC-MS-MS analysis as for example described in the literature (Thingholm TE, Larsen MR: Methods Mol Biol 2009, 527:57-6). Peptides are suspended in 100% formic acid, diluted with H₂O and loaded directly onto a 18 cm RP capillary column using a nano-Easy-LC system (Proxeon, Thermo Scientific). Peptides are eluted using a gradient from 100% phase A (0.1% formic acid) to 35% phase B (0.1% formic acid, 95% acetonitrile) over 43 min directly into an LTQ-Orbitrap XL mass spectrometer (Thermo Scientific). For each MS scan (Orbitrap), acquired t a resolution of 60000, 300-1800 Da range, the five most abundant precursor ions are selected for fragmentation (CID). The raw data files are converted to mgf files and searched in Mascot 2.2 software using Proteome Discoverer (Thermo Scientific). Peptides with a mascot probability score p < 0.05 are further analysed.

Immunization procedure Six 4-6 week old Balb/C mice are immunized subcutaneously in the abdomen with a peptide having a GxxP-motif, preferably with 200 µL emulsified antigen (50 µg per immunization) using Freund's incomplete adjuvant comprising a peptide having a GxxP-motif (such as KLH-CGG- with peptide SEQ ID NO: 8 (GGGPGAG), KLH-CGG- with peptide SEQ ID NO: 41 (VGVAPG), KLH-CGG- with peptide SEQ ID NO: 220 (LQGVLPAL), KLH-CGG- with peptide SEQ ID NO: 223 (GVGVGVPG), KLH-CGG- with peptide SEQ ID NO: 217 (GVPGLGVGAGVPGLGV) or KLH-CGG- with peptide SEQ ID NO: 143 (VPGVGISPEA) , obtainable on request from commercial sources such as Chinese Peptide Company, Beijing, China, or Ansynth BV, Roozendaal, The Netherlands). Immunizations are continued until stable titer levels are obtained. Mice with the highest titers are selected for fusion and boosted intravenously with 50 µg immunogen in 100 µL 0.9% sodium chloride solution three days before isolation of the spleen for cell fusion. The fusion procedure has been described elsewhere (Gefter ML, Margulies DH, Scharff MD: Somat Cell Genet 1977, 3:231-236.).

Characterization of clones. Native reactivity and peptide binding of the generated monoclonal antibodies is evaluated by displacement of human serum in a preliminary indirect ELISA using biotinylated peptides (Biotin-GxxP-peptide) on a streptavidin-coated microtitre plate and the supernatant from the growing monoclonal hybridoma. Tested are the specificities of clones to the free GxxP-peptides and non-GxxP peptides. Isotyping of the monoclonal antibodies is performed using the Clonotyping System-HRP kit (Southern Biotech). Selected clones are purified using Protein G columns according to manufacturer's instructions (GE Healthcare Life Science). Assay protocol Selected antibody is labeled with horseradish peroxidase (HRP) using the Lightning link HRP labeling kit according to the instructions of the manufacturer (Innovabioscience). A 96-well streptavidin plate is coated with 0.4 ng/mL biotinylated C-peptide dissolved in assay buffer (25 mM Tris, 1% BSA, 0.1% Tween-20, pH 7.4) and incubated for 30 minutes at 20°C. 20 µL of free peptide calibrator or sample are added in duplicate to appropriate wells, followed by 100 µL of HRPO-conjugated antibody and incubated for 1 hour at 20°C. Finally, 100 µL tetramethylbenzinidine (TMB) (Kem-En-Tec) is added and the plate is incubated for 15 minutes at 20°C in the dark. All the above incubation steps optionally include shaking at 300 rpm. After each incubation step the plate is washed five times in washing buffer (20 mM Tris, 50 mM NaCl, pH 7.2). The TMB reaction is stopped by adding 100 µL of stopping solution (1% HCl) and measured at 450 nm with 650 nm as the reference.

Technical evaluation and specificity. From 2-fold dilutions of quality control (QC) serum and plasma samples, linearity is calculated as a percentage of recovery of the 100% sample. The lower limit of detection is determined from 21 zero serum samples (i.e. buffer) and calculated as the mean+3X standard deviation. The inter- and intra-assay variation is determined by 12 independent runs of 8 QC serum samples, with each run consisting of two replicas of double determinations. The stability of serum is measured using three serum samples, which are frozen and thawed between one and 10 times.

Sample levels of GxxP-peptides such as concentration of C-peptide and elastin-peptide or GxxP-containing fragment concentration, or, alternatively, candidate drug effect, is for example measured by using a competitive binding assay or ELISA method. Maxisorb 96-well microtiter plates (Nunc) are coated with 50 microliter of elastin peptide (0.5 microg/ml, CB573, Elastin Products Company) in PBS, pH 7.4 and incubated overnight at 4°C. The wells are blocked with 100 microl of 0.5% BSA in PBS containing 0.05% Tween 20 (PBS-T). 25 microl of each sample is diluted 2 times with PBS and mixed with 50 microl of 1:1000 diluted anti-elastin-peptide antibody, preferably with anti-GxxP-fragment antibody, such as antibody BA4 (Sigma-Aldrich E4013 or Abcam ab21599). Positive control samples contain C-peptide and elastin peptide VGVAPG. Negative control samples consist of PBS. 100 microliter test mixtures, for example containing biological samples such as blood, serum, plasma or urine or test compounds or control samples, are then added to each well in the elastin peptide coated plate and incubated for 30 min at 37°C, the plates are washed three times with PBS-T, followed by the addition 50 microl of secondary antibody (1:2000 anti-rabbit IgG peroxidase conjugate). After 1 hour incubation at 37°C, the plates are washed three times, 50 microl of tetramethylbenzidine substrate solution (Thermo Fisher Scientific, San Jose, CA) is added, and after 10 minutes incubation at room temperature the reaction is quenched by adding 50 microl 1 M H₂SO₄ to each well. The absorbance is measured at 450 nm using a micro-plate reader. The accuracy and precision of the quantitative range of the ELISA is determined by replicate analyses. If required, the concentration of GxxP containing fragments is normalized against the total protein concentration of the samples.

Sample levels of GxxP-peptides such as concentration of C-peptide and elastin-peptide or GxxP-containing fragment concentration, or, alternatively, candidate drug effect, is for example measured by using a competitive binding assay or ELISA method. Maxisorb 96-well microtiter plates (Nunc) are coated with 50 microliter of C-peptide (0.5 microg/ml, SIGMA) in PBS, pH 7.4 and incubated overnight at 4°C. The wells are blocked with 100 microl of 0.5% BSA in PBS containing 0.05% Tween 20 (PBS-T). 25 microl of each sample is diluted 2 times with PBS and mixed with 50 microl of 1:1000 diluted anti-C-peptide antibody, preferably with anti-GxxP-fragment antibody, such as antibody BA4. Positive control samples contain C-peptide and elastin peptide VGVAPG. Negative control samples consist of PBS. 100 microliter test mixtures, for example containing biological samples such as blood, serum, plasma or urine or test compounds or control samples, are then added to each well in the C-peptide coated plate and incubated for 30 min at 37°C, the plates are washed three times with PBS-T, followed by the addition 50 microl of secondary antibody (1:2000 anti-rabbit IgG peroxidase conjugate). After 1-hour incubation at 37°C, the plates are washed three times, 50 microl of tetramethylbenzidine substrate solution (Thermo Fisher Scientific, San Jose, CA) is added, and after 10 minutes incubation at room temperature the reaction is quenched by adding 50 microl 1 M H₂SO₄ to each well. The absorbance is measured at 450 nm using a micro-plate reader. The accuracy and precision of the quantitative range of the ELISA is determined by replicate analyses. If required, the concentration of GxxP-peptide containing fragments is normalized against the total protein concentration of the samples.

Elastin receptor or receptor fragment concentration, or, alternatively, candidate drug effect, is for example measured by using a competitive binding assay or ELISA method. Maxisorb 96-well microtiter plates (Nunc) are coated with 50 microliter of elastin receptor or fragment therof (0.5 microg/ml,) in PBS, pH 7.4 and incubated overnight at 4°C. The wells are blocked with 100 microl of 0.5% BSA in PBS containing 0.05% Tween 20 (PBS-T). Simultaneously, 25 microl of each sample is diluted 2 times with PBS and mixed with 50 I of 1:1000 diluted anti-elastin-receptor antibody, or with 50 microl of 1:1000 diluted anti-C-peptide antibody, preferably with anti-GxxP-fragment antibody, such as antibody BA4. Ciontrol samples contain V14 peptide (SEQ ID NO: 131 (VVGSPSAQDEASPL)) or lactose. 100 microliter mixtures are then added to each well in the elastin receptor coated plate and incubated for 30 min at 37°C, the plates are washed three times with PBS-T, followed by the addition 50 microl of secondary antibody (1:2000 anti-rabbit IgG peroxidase conjugate). After 1-hour incubation at 37°C, the plates are washed three times, 50 microl of tetramethylbenzidine substrate solution (Thermo Fisher Scientific, San Jose, CA) is added, and after 10 minutes incubation at room temperature the reaction is quenched by adding 50 microl 1 M H₂SO₄ to each well. The absorbance is measured at 450 nm using a micro-plate reader. The accuracy and precision of the quantitative range of the binding assay or ELISA is determined by replicate analyses. If required, the concentration of elastin-receptor containing fragments is normalized against the total protein concentration of the samples.

### Elastin test

Elastin (ELN) BioAssay^{™} ELISA Kit (Human) (catalog nr 191345) utilizes the Sandwich Enzyme Immunoassay technique.

### C-peptide test

Abcam's C-peptide Human *in vitro* ELISA (Enzyme-Linked Immunosorbent Assay) kit (catalog nr ab178641) is designed for the measurement of C-peptide in serum and plasma.

C-peptide interacts with the elastin receptor complex. A new perspective on diagnosis, prevention and treatment of coronary heart disease. To tackle coronary heart disease (CHD), we focus on proinsulin-derived C-peptide's role in vascular disease. A receptor of C-peptide is unknown. However, we observed a hitherto unnoticed sequence motif GxxP in C-peptide, suggesting it to be a ligand of the elastin-receptor-complex (ERC, Blanchevoye et al.) that is involved in vascular repair. Ido et al., showed C-peptide with its midportion GGGPGAG to be essential to normalize vascular dysfunction, but concluded that no receptor is involved. However, it was overlooked that all peptides tested with motif GxxP (including stereochemically equivalent D-form PxxG) normalize vascular dysfunction; none of those without do. Thus, said motif is both necessary and sufficient: C-peptide is likely an important signal-molecule acting via ERC. To corroborate, we confirmed that peptides SEQ ID NO: 34 (GGGPG), SEQ ID NO: 46 (GAGPG) and D-form GPGAG fit a model of ERC developed for docking prototype elastin peptide VGVAPG. Furthermore: - Monocytes, smooth muscle cells, fibroblasts and endothelial cells involved in vascular repair all carry the ERC. - C-peptide modulates vascular repair when given to mammals with diabetes type 1. - Elastin peptide SEQ ID NO: 41 (VGVAPG) as well as C-peptide stimulate vascular repair cells and promote experimental arteriosclerotic lesion development in mice (Gayral et al; Vasic et al.). - Elastin peptide GVAPGIGPGG predicts myocardial infarction, and C-peptide (Min and Min) predicts CHD. These findings all support the inventor's finding that chronic accumulation of GxxP-peptides derived from C-peptide or elastin is the root cause of CHD. These findings ties together several, seemingly unrelated, risk factors for CHD from diet and lifestyle into one central factor: Chronic accumulation of GxxP-peptides derived from C-peptide or elastin leads to vascular disease and CHD by chronic activation of ERC modulated vascular repair. Risk factors that act via GxxP-peptide from C-peptide: Excess intake of sugar or of high glycemic food, optionally combined with a sedentary live, increases blood glucose. Glucose activates pancreatic beta cells to excrete equimolar amounts of insulin and C-peptide into the blood. Long chain free fatty acids may amplify glucose-stimulated insulin and thus also C-peptide, secretion. Degradation of C-peptide produces GxxP-bearing C-peptide fragments. Risk factors that act via GxxP-peptide from elastin: Smoking, inflammation and ageing degrade elastin, typically by proteolysis. That releases GxxP-bearing elastin peptide fragments into the blood. Processed meats may be a dietary source of proteolytically degraded elastin from dispersed gristle and sinews. Risk factors combined: Chronic dosing of mice with elastin-derived GxxP-peptides is found to cause resistance to insulin, and C-peptide is considered a marker of insulin resistance. Elastin peptide induced insulin resistance with subsequent hyperinsulinemia may be an additional cause of rising levels of C-peptide; combining risk factors accelerates build up of GxxP-peptides. Also, our perspective sheds new light on other diseases that associate with arteriosclerosis and insulin resistance, such as stroke, peripheral arterial disease, dementia, chronic kidney disease and pancreatic beta-cell failure. This disclosure provides diagnostic tools to detect cumulative GxxP-peptide levels in blood or urine to allow anyone to proactively monitor his or her own overall vascular health and risk for CHD. Tests for C-peptide or elastin peptide exist; developing the requisite diagnostic tools to specifically test levels of circulating GxxP-peptides may take less then 1 year. Test development and subsequent large-scale multiple center testing to validate a GxxP-peptide test (or arterial risk test, ArtestTM) may take less then 2 years, followed by registration with health authorities. In parallel, ArtestTM devices will be developed for personalized use. This disclosure provides dietary and lifestyle guidance to keep GxxP-peptide at physiological levels to maintain vascular health and prevent CHD. Above ArtestTM development allows us to design and assess human trials to determine the mechanisms in vivo by which diet and/or lifestyle components alter risk for GxxP-mediated disease, aiming to develop recommendations to reduce the occurrence of vascular disease and CHD. This disclosure provides novel drugs that antagonise the action of GxxP-peptides on the elastin receptor complex to treat and cure vascular disease with CHD. As animals may have different strategies to cope with glucose than man (e.g. humans with motif SEQ ID NO: 38 (GGGP) being considerably less tolerant to glucose than pigs with motif GGGL), we may need to carefully develop a fitting animal model for further non-clinical drug-development.

### Arterial risk test (Artest ^{®})

A first or pilot arterial risk test designed for the measurement of GxxP-peptide fragments in urine, serum and plasma is developed as reported here. Sample levels of GxxP-peptides (such as concentration of C-peptide and elastin-peptide or GxxP-peptide fragment concentration) are measured by using a competitive binding assay or ELISA method. Samples that are tested in elastin test and C-peptide test are also tested in the arerial risk test. Maxisorb 96-well microtiter plates (Nunc) are coated with 50 microliter of C-peptide (0.5 microg/ml, SIGMA) in PBS, pH 7.4 and incubated overnight at 4°C. The wells are blocked with 100 microl of 0.5% BSA in PBS containing 0.05% Tween 20 (PBS-T). 25 microl of each sample is diluted 2 times with PBS and mixed with 50 microl of 1:1000 diluted with anti-GxxP-fragment antibody, such as antibody BA4 specifically reacting with GxxP amino acid sequences. Positive control samples contain C-peptide and/or elastin peptide SEQ ID NO: 41 (VGVAPG). Negative control samples consist of PBS. 100 microliter test mixtures, for example containing biological samples such as blood, serum, plasma or urine or test compounds or control samples, are then added to each well in the C-peptide coated plate and incubated for 30 min at 37°C, the plates are washed three times with PBS-T, followed by the addition 50 microl of secondary antibody (1:2000 anti-rabbit IgG peroxidase conjugate). After 1-hour incubation at 37°C, the plates are washed three times, 50 microl of tetramethylbenzidine substrate solution (Thermo Fisher Scientific, San Jose, CA) is added, and after 10 minutes incubation at room temperature the reaction is quenched by adding 50 microl stop solution (1 M H₂SO₄) to each well. The absorbance is measured at 450 nm using a micro-plate reader. The accuracy and precision of the quantitative range of the ELISA is determined by replicate analyses and analyses of control samples. The intensity of signal is reversely proportional to the amount of GxxP-peptide in the sample. A sample having a signal exceeding 50% inhibition relative to a control C-peptide sample tested at 2ng/ml is considered exceeding normal values (+), normal samples are indentified by (-). If required, the concentration of GxxP-peptide containing fragments is normalized against the total protein concentration of the samples. Twelve human sera are tested in elastin test, C-peptide test, pilot arterial risk test, and pilot arterial risk test MST, respectively, as described above and below. Results for serum 1 (S1) are (-,-,-,-), for serum 2 (S2) are (-,+,+,+), S3 (+,+,+,+), S4 (-,-,-,-), S5 (-,-,-,-), S6 (+,+,+,+), S7 (+,-,+,+), S8 (+,+,+,+), S9 (+,+,+,+), S10 (+,-,+,+), S11 (+,-,+,+) and S12 (-,-,+,+).

### Microscale electrophoresis

Herein, we also introduce microscale thermophoresis (MST) as a tool to characterize or measure elastin binding protein binding with small-molecules such as lactose and derivates thereof and/or with small peptide interactions in buffers and biological liquid such as plasma, serum or cell lysates. In contrast to existing techniques, MST works in free-solution and with low consumption of sample. It is an entirely optical method, which is contact-free and therefore minimizes contamination of the sample. The experimental setup consists of an infrared laser coupled into the path of fluorescent excitation/emission using an infrared dichroic mirror. The laser is focused onto the sample through the same objective that is used for fluorescence detection. This allows the observation of thermohoresis in various microfluidic sample compartments such as capillaries or microfluidic channels. High reproducibility and low sample consumption are achieved using 100-µm-diameter glass capillaries with a total volume of about 500 nl. The infrared-laser creates a spatial temperature distribution on the length scale of 25 µm. The temperature increase scales linearly with the laser power. After 150 ms, laser heating and heat dissipation reach equilibrium, and a steady-state temperature increase of typically 2-6 K is obtained. This temperature rise induces a spatial concentration distribution that is visualized by a fluorescent dye covalently attached to one of the binding partners.

Typically, one primary amine per protein is labelled and thus the position of the dye is statistically distributed. To measure thermophoresis of proteins, the change in concentration between the initial state and the steady state is measured. Therefore, two images of the sample are required: one image of the initial state before laser heating, showing a homogenous distribution of molecules, and a second image acquired after a few seconds of infrared-laser heating. This short measurement time is sufficient because of the fast mass diffusion over the small dimension of the temperature distribution. Even if the diffusion is slow and no steady state is reached within the measurement time, the concentration profiles are typically distinguishable after several seconds. Switching off the infrared-laser leads to a reestablishment of the initial homogeneous concentration profile by ordinary diffusion, providing information about the diffusion coefficient of the molecules. To analyse binding events, the measurement is performed at various concentration ratios of the binding partners. Typically, the fluorescent binding partner is kept at a constant concentration and the unlabelled molecule is titrated until a saturation of all binding sites is obtained. From these \ research.

The EBP is labelled with AlexaFluor 647 and kept at a constant concentration of 5 nM in 1× phosphate-buffered saline (PBS) buffer at 5 °C. The small-molucule or peptide is typically titrated from 0.1 to 700 nM, but other conditions may be selected. On binding of the peptide, the thermophoretic concentration signal of EBP changes. The level of depletion, as compared with the unbound state, versus the peptide/small molecule concentration is plotted. The depletion is interpreted as a binding curve with the fraction of EBP in complex with its peptide or small molecule.

### Peptide Synthesis

Synthetic PG-domain or GxxP-type peptides such as SEQ ID NO: 41 (VGVAPG), SEQ ID NO: 138 (GVAPGV), SEQ ID NO: 139 (VAPGVG), SEQ ID NO: 140 (APGVGV), SEQ ID NO: 141 (PGVGVA), SEQ ID NO: 142 (GVGVAP), SEQ ID NO: 60 (PGAIPG), SEQ ID NO: 137 (LGTIPG), SEQ ID NO: 32 (LGGGPGAG), SEQ ID NO: 8 (GGGPGAG), SEQ ID NO: 49 (GGGPGA), SEQ ID NO: 38 (GGGP), SEQ ID NO: 40 (GGGPG), SEQ ID NO: 46 (GAGPG), SEQ ID NO: 50 (GGGPE), SEQ ID NO: 51 (GAIPG), SEQ ID NO: 52 (GGVPG), SEQ ID NO: 53 (GVAPG), SEQ ID NO: 54 (YTTGKLPYGYGPGG), SEQ ID NO: 55 (YGARPGVGVGIP), SEQ ID NO: 56 (PGFGAVPGA), SEQ ID NO: 57 (GVYPG), SEQ ID NO: 58 (GFGPG), SEQ ID NO: 59 (GVLPG), SEQ ID NO: 51 (GAIPG), SEQ ID NO: 60 (PGAIPG), SEQ ID NO: 61 (PGAVGP), SEQ ID NO: 62 (VGAMPG), SEQ ID NO: 63 (VGSLPG), SEQ ID NO: 64 (VGMAPG), SEQ ID NO: 65 (VPGVG), SEQ ID NO: 66 (IPGVG), SEQ ID NO: 63 (VGSLPG), SEQ ID NO: 41 (VGVAPG), SEQ ID NO: 67 (VGVPG), SEQ ID NO: 68 (AGAIPG), SEQ ID NO: 69 (VPGV), SEQ ID NO: 70 (LGITPG), SEQ ID NO: 71 (GDNP), SEQ ID NO: 72 (GAIP), SEQ ID NO: 73 (GKVP), SEQ ID NO: 74 (GVQY), SEQ ID NO: 75 (GVLP), SEQ ID NO: 76 (GVGP), SEQ ID NO: 77 (GFGP), SEQ ID NO: 78 (GGIP), SEQ ID NO: 79 (GVAP), SEQ ID NO: 80 (GIGP), SEQ ID NO: 39 (GAGP), SEQ ID NO: 81 (GGIPP), SEQ ID NO: 82 (GQFP), SEQ ID NO: 83 (GLSP), SEQ ID NO: 84 (GPQP), SEQ ID NO: 85 (GGPQP), SEQ ID NO: 86 (GPQPG), SEQ ID NO: 87 (GGPQPG), SEQ ID NO: 88 (GIPP), SEQ ID NO: 81 (GGIPP), SEQ ID NO: 89 (GIPPA), SEQ ID NO: 90 (GGIPPA), or retro-inverso variants thereof are synthesized according to classical solid phase synthesis. V14 peptide, a peptide reproducing the sequence of S-Gal interacting with elastin peptides bearing the PG-domain, in particular the motif GxxP, is obtained from Neosystem (Strasbourg, France). Alternatively, V14 peptide and variants thereof are synthesised as described herein. Purity of the peptides is confirmed by high performance liquid chromatography and by fast atom bombardment mass spectrometry.

Traditionally, peptides are defined as molecules that consist of between 2 and 50 amino acids, whereas proteins are made up of 50 or more amino acids. In addition, peptides tend to be less well defined in structure than proteins, which can adopt complex conformations known as secondary, tertiary, and quaternary structures. Functional distinctions may also be made between peptides and proteins. Peptides, however, may be subdivided into peptides, which have few amino acids (e.g., 2 to 30-50), and polypeptides, which have many amino acids (>50). Proteins are formed from one or more polypeptides joined together. Hence, proteins essentially are very large peptides. In fact, most researchers, as well as this application, use the term *peptide* to refer specifically to peptides, or otherwise relatively short amino acid chains (<51 amino acids), with the term *polypeptide* being used to describe proteins, or chains of > 50 or much more amino acids.

Treatment of cultured cells with C-peptide or fragments therof.

Cells may be plated at a density of 450 per mm² in a 24-well microplate or 32 mm diameter Petri dish and cultured for 2-4 days or in cultures as described above. On day 2 in culture, cells are treated with various C-peptides (preferably selected from Table 1) or peptide fragments thereof for 1 or 2 days. In an experiment, cells were treated with a combination of C-peptide (1 micro-M) or polyclonal anti-67 kDa elastin receptor antibody (anti-S-Gal antibody) (10 ng per ml) for 2 d. At the end of the treatment, cells may be trypsinized (0.25%) and the cell number determined with a Coulter counter. For determination of thymidine incorporation, cells are labeled with 50 micro-Ci of [methyl-³H] thymidine (3.2 TBq per mmol; Amersham) for the final 18 h of the treatment. Incorporated thymidine is determined as trichloroacetic acid-precipitable counts with a liquid scintillation spectrometer (Beckman LS9800). Binding may be antagonized by adding V32-peptide or V32-peptide fragments or V14 peptide or V14-peptide fragments or lactose.

Detection of the 67 kDa elastin receptor. To select for or confirm the presence of the 67 kDa elastin receptor in cells, reverse transcription-polymerase chain reaction is performed using cellular RNA and synthetic oligoprimers corresponding to the beta-galactosidase cDNA sequences upstream and downstream spanning the region between exons 2 and 5. The reaction is run for 40 cycles with denaturation at 90°C for 1 min, annealing at 50°C for 2 min, and extension at 72°C for 5 min in a DNA Thermal Cycler (Perkin-Elmer Cetus). The polymerase chain reaction products are preferably analyzed on 1% agarose gel.

Determination of chemotactic activity. Human U937 monocytic cells are purchased from the American Type Culture Collection (ATCC catalog number CRL-1593.2, Manassas, Va). Cells are maintained in suspension culture in T-75 flasks containing RPMI 1640 medium supplemented with 10% fetal calf serum and antibiotics, and cultures are split every 3 to 5 days. Three days before use in chemotaxis assays, U937 cells are stimulated to differentiate along the macrophage lineage by exposure to 1 mmol/L dibutyryl cyclic adenosine monophosphate (dbcAMP; Sigma Chemical Co), as described. Cells are washed three times to remove culture medium and then resuspended in chemotaxis medium (Dulbecco's modified essential medium supplemented with 1% lactalbumin hydrolysate) for plating into assay chambers at a final concentration of 2.5 × 106 cells/mL. Chemotaxis assays are performed in 48-well microchemotaxis chambers (Neuro Probe, Cabin John, Md). The bottom wells of the chamber are filled with 25 mL of the chemotactic stimulus (or medium alone) in triplicate. An uncoated 10-mm-thick polyvinylpyrrolidone-free polycarbonate filter with a pore size of 5 mm is placed over the samples (Neuro Probe). The silicon gasket and the upper pieces of the chamber are applied, and 50 mL of the monocyte cell suspension are placed into the upper wells. Chambers are incubated in a humidified 5% CO2 atmosphere for 3 hours at 37° C, and nonmigrated cells are gently wiped away from the upper surface of the filter. The filter is immersed for 30 seconds in a methanol-based fixative and stained with a modified Wright-Giemsa technique (Protocol Hema 3 stain set; Biochemical Sciences, Inc, Swedesboro, NJ) and then mounted on a glass slide. Cells that are completely migrated through the filter are counted under light microscopy, with 3 random high-power fields (HPF; original magnification × 400) counted per well.

Human monocytes are isolated from freshly drawn blood of healthy volunteers using serial Ficoll/Percoll gradient centrifugation, as described elsewhere. Cells are cultured for 16 hours in RPMI-1640 media supplemented with 0.5% human serum to become quiescent after isolation. Purity of the cells is >95% as determined by flow cytometry analysis. Monocyte chemotaxis is assayed in a 48-well microchemotaxis chamber (Neuroprobe, Gaithersburg, MD) in serum-free media. Wells in the upper and lower chamber are separated by a polyvinylpyrrolidone-free polycarbonate membrane (pore size 5 µm; Costar). Freshly isolated monocytes at a density of 5×10⁵/mL are incubated for 2.5 hours with recombinant C-peptide (Sigma), before migrated cells on the bottom face of the filter are stained and counted under the light microscope. Maximal chemotactic activity is measured with 0.1 mmol/L N -formyl-methionyl-leucyl-phenylalanine (f-MLF; Sigma Chemical Co), and checkerboard analysis is used to distinguish chemotaxis from chemokinesis. Inhibition of chemotaxis is tested by competition with VGVAPG, a repetitive peptide sequence found in human and bovine elastin (Sigma Chemical Co), and BA-4, an antielastin-blocking antibody. Controls for BA-4 include mouse immunoglobulin G (IgG; PharMingen, San Diego, Calif). Exposure of cells to lactose is used to specifically dissociate the 67-kD EBP. Controls for lactose included glucose, fructose, and mannose, none of which affect the 67-kD EBP. In each case, monocytes cells are exposed to the relevant concentrations of SEQ ID NO: 41 (VGVAPG) (10-9 to 10-5 mol/L), antibodies (1:1000), or sugars (1 mmol/L) for 30 minutes before the chemotaxis assays are started. SEQ ID NO: 41 (VGVAPG) induces chemotaxis in a concentration-dependent manner with maximal activity at 0.1 mmol/L, and it is considerably more potent than the same concentration of f-MLF. Monocytes in the upper wells of the assay chamber are exposed to varying concentrations of SEQ ID NO: 41 (VGVAPG) for 30 minutes before stimulation by human recombinant C-peptide (0.1 nmol/L - 10nmol/L) in the lower wells. Exposure of cells to SEQ ID NO: 41 (VGVAPG) eliminates monocyte chemotaxis induced by C-peptide, a result consistent with competition by the C-peptide for cellular elastin binding sites. In contrast, preincubation of monocytic cells with SEQ ID NO: 41 (VGVAPG) does not alter the chemotactic response to f-MLF. C-peptide stimulates a concentration-dependent increase in monocyte migration Checkerboard analysis demonstrates that C-peptide stimulates chemotaxis without a chemokinetic effect. C-peptide derived chemotactic activity is eliminated by competition with Val-Gly-Val-Arg-Pro-Gly = SEQ ID NO: 41 (VGVAPG), a repetitive peptide found in human elastin that binds to cellular elastin receptors, and decreases in the presence of BA-4, a monoclonal antibody that can block elastin peptide mediated chemotactic activity. Monocyte chemotaxis in response to both SEQ ID NO: 41 (VGVAPG) and C-peptide is abolished in the presence of lactose, a galactosugar that specifically dissociates the 67-kD EBP, but it is unaffected by glucose, fructose, or mannose. These findings show that C-peptide can attract mononuclear phagocytes through ligand-receptor interactions with the 67-kD EBP, thereby providing a molecular mechanism to explain the inflammatory response that accompanies arteriosclerosis and atherosclerosis. Chemotaxis is also assayed by a double micropore membrane system in modified Boyden chambers. The lower compartment containing 180 micro-I of C-peptide or fragments thereof at various concentrations is separated from the upper compartment containing 200 micro-I of cell suspension (5 × 10⁴ cells, such as endothelial cells or smooth muscle cels or pericytes or keratinocytes of fibroblasts or leukocytes per ml medium) by a 10 micro-m polycarbonate membrane (Millipore, Bedford, MA). The membranes are presoaked in bovine type I collagen (25 micro-g phosphate-buffered saline per ml) (Chemicon International, Temecula, CA) for 24 h at room temperature to facilitate the attachment of cells. The chambers are incubated for 18 h at 37°C in 5% CO₂-balanced air. The chambers are then disassembled, and the membrane pairs are stained with hematoxylin. The cell number of a number, such as five, random and nonoverlapping fields under a microscope is counted. For all experiments, medium alone in the bottom chamber may serve as the baseline control. To confirm directed cell migration, the concentration gradient between the upper and lower compartments may be abolished by adding various doses of elastin to the cell suspension. Chemotaxis is assayed as described above. Chemotaxis may als be studied in an ex vivo aortic ring assay measuring endothelial cell migration and proliferation

Recombinant EBP is produced as follows. It is known that the non-sequential alternative splicing of the primary transcript of the β-galactosidase gene generates two mRNAs, one encoding the precursor of the lysosomal enzyme (β-gal) and the second encoding an enzymatically inactive protein (S-gal or EBP), which is not targeted to the lysosomes. In the S-gal-encoding mRNA, exons 3, 4, and 6 are spliced out, and exon 5 is shifted in frame, thus creating a unique region encoding a 32-amino acid sequence in S-gal, which differs from its counterpart encoded by exon 5 of active β-gal and contains an elastin-peptide binding domain. S-gal cDNA clone is constructed with routine procedures. To construct the full-length alternatively spliced cDNA clone (1986 bp) reflecting the sequence described by Morreau and colleagues, poly(A)⁺ mRNA was isolated from cultured normal human skin fibroblasts using a Quick Prep mRNA purification kit from Pharmacia. This mRNA (500 ng) was reverse-transcribed using random hexamers and superscript reverse transcriptase (Life Technologies, Inc.). To isolate overlapping fragments of the cDNA, two polymerase chain reactions (PCR) were carried out. The 5' portion of the cDNA (357 bp) was amplified using the primers 5'-GGTGGTCATGCCGGGGTTCCT-3' and 5'-ATGTTGCTGCCTGCACTGTT-3'. The primers 5'-CCATCCAGACATTACCTGGC-3' and 5'-CCCTCACACATTCCAGGTGGT-3' were used to amplify the 3' fragment of the cDNA (1598 bp). The reactions were carried out on a Perkin-Elmer thermal cycler using an annealing temperature of 55 °C. The fragments were gel-purified and ligated into the *Eco*RV site of pBluescript SK⁺. The respective fragments contained a 115-bp overlapping sequence at their respective 3' and 5' ends. In addition, the absence of the initial 27 bp located at the 5' end of the 5' fragment and 119 bp at the 3' end of the 3' fragment was detected (attributed to primer positioning in the initial PCR reaction). Final assembly of the full-length clone eliminated the overlapping segment by employing a common *Pvu*II site found in the overlapping region between the two portions of S-gal. Complete double digestion of the 5' clone with the restriction enzymes *Kpn*I and *Pvu*II yielded a 316-bp fragment representing the 5' segment (*i.e.*. 5' to the *Pvu*II site) of S-gal, which included an additional 57 bp of vector at its 5' end. The *kpn*I digestion created a 3' overhang, which was blunt-ended by using *Pfu* DNA polymerase. The 316-bp 5' fragment and a *Pvu*ll-digested 3' clone were then both agarose gel-purified, gene-cleaned, and ligated. The ligation products were transformed into bacterial cell and grown on LB-AMP plates. Restriction digests with *Xho*I and*Pvu*II confirm the correct orientation of the short 5' segment of S-gal in the new construct. *In vitro* transcription/translation was done in accordance to the protocols provided by Promega. S-gal cDNA (5 µg) in pGEM-3Z was linearized (digested with *Xbal*), and*in vitro* transcription was conducted. This was followed by *in vitro* translation using 2 µl of RNA substrate in a nuclease-treated rabbit reticulocyte lysate (minus microsomal membranes and protease inhibitors) in the presence of 0.8 mCi/ml [³⁵S]methionine ([³⁵S]Met). The translation mix (minus mRNA) was used as control. The supernatants were directly analyzed by SDS-polyacrylamide gel electrophoresis (SDS-PAGE), followed by autoradiography to detect for the presence of [³⁵S]Met-labeled reaction products and to compare molecular size. The reaction products were further characterized using immunoprecipitation with antibodies recognizing β-gal, S-gal, and EBP, and then by elastin affinity columns.

One aspect of the present disclosure relates to methods for forming crystals comprising fragments of C-PEPTIDE and elastin receptor as well as crystals comprising fragments of C-PEPTIDE and elastin receptor. In one aspect of the present disclosure, a method for forming crystals comprising fragments of C-PEPTIDE and elastin receptor is provided comprising forming a crystallization volume comprising fragments of C-PEPTIDE (see Table 1) and elastin receptor, one or more precipitants, optionally a buffer, optionally a monovalent and/or divalent salt and optionally an organic solvent; and storing the crystallization volume in a container under conditions suitable for crystal formation. In yet another aspect, a method for forming crystals comprising fragments of C-PEPTIDE and elastin receptor is provided comprising forming a crystallization volume comprising fragments of C-PEPTIDE and elastin receptor in solution comprising PEG precipitant listed herein below; and storing the crystallization volume in a container under conditions suitable for crystal formation. PEG precipitant 5-50% w/v of precipitant wherein the precipitant comprises one or more members of the group consisting of PEG MME having a molecular weight range between 300-10000, and PEG having a molecular weight range between 100-10000 pH 5-9. Buffers that may be used include, but are not limited to tris, bicine, cacodylate, acetate, citrate, MES and combinations thereof. Additives optionally 0.05 to 0.8 M additives wherein the additives comprise sarcosine or 0.5 to 25% additives wherein the additives comprise xylitrol Protein Concentration 1 mg/ml-50 mg/ml Temperature 1 degree C -25 degrees C.

In describing protein or peptide composition, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal, left terminal to right terminal, the N-terminal being identified as a first residue. Ala: alanine residue; Asp: aspartate residue; Glu: glutamate residue; Phe: phenylalanine residue; Gly: glycine residue; His: histidine residue; Ile: isoleucine residue; Lys: lysine residue; Leu: leucine residue; Met: methionine residue; Asn: asparagine residue; Pro: proline residue; Gln: glutamine residue; Arg: arginine residue; Ser: serine residue; Thr: threonine residue; Val: valine residue; Trp: tryptophane residue; Tyr: tyrosine residue; Cys: cysteine residue. The amino acids may also be referred to by their conventional one-letter code abbreviations; A=Ala; T=Thr; V=Val; C=Cys; L=Leu; Y=Tyr; I=Ile; N=Asn; P=Pro; Q=Gln; F=Phe; D=Asp; W=Trp; E=Glu; M=Met; K=Lys; G=Gly; R=Arg; S=Ser; and H=His.

A plasmid encoding C-peptide-Fc is generated by ligating a fragment encoding the C-peptide (residues 57-87 as shown in Table 1 under identifier >sp|P01308|57-87) into the pCAGGS expression vector as an N-terminal fusion with the fragment encoding the Fc domain of human IgG . Likewise, an C-peptide-Fc expression plasmid is made for the C-peptide (residues 57-87 as shown in Table 1 under identifier >sp|P01326|57-87) and the C-peptide (residues 57-87 as shown in Table 1 under identifier >sp|P01325 157-87). C-peptide-Fc proteins are expressed by transfection of the expression plasmids into 293T cells and affinity purified from the culture supernatant using protein A sepharose beads.

A plasmid encoding a fragment of the elastin receptor is generated by ligating a fragment encoding residues 29-546 of human EBP (Uniprot identifier P16278-2) into a pCD5 expression vector encoding the signal sequence of CD5 and a OneSTrEP affinity tag (IBA GmbH). Likewise, a fragment of the elastin receptor is generated by ligating a fragment encoding residues 29-226 of human EBP (Uniprot identifier P16278-2) into a pCD5 expression vector encoding the signal sequence of CD5 and a OneSTrEP affinity tag. Elastin receptor fragment is expressed by transfection of the expression plasmid into 293T cells and affinity-purified from the culture supernatant using Streptactin sepharose beads (IBA GmbH). A plasmid encoding C-peptide-Fc is generated by ligating a fragment encoding the C-peptide (residues 57-87 as shown in Table 1 under identifier >sp|P01308|57-87) into the pCAGGS expression vector as an N-terminal fusion with the fragment encoding the Fc domain of human IgG separated by a thrombin cleavage site. Purified C-peptide-Fc is cleaved with thrombin and soluble C-peptide is purified by gel filtration.

An immunoprecipitation protocol is essentially carried out as described (Liet al., 2003, Nature 426: 450). In short, cells are washed twice with ice-cold PBS, scraped off the plastic with a rubber policeman, pelleted and lysed in ice-cold lysis buffer (0.3% DDM in PBS) containing protease inhibitors (Roche Complete Mini) at a final density of ^{~}2.5×107 cells/mL. Cell lysates are precleared with protein A sepharose beads after which 10 microgram of probe C-peptide-Fc is added to 1ml of cell lysate and incubated for 1h at 4°C under rotation. Precipitates are washed thrice with lysis buffer and once with PBS and animaled to NoVEX^{®} 4-12% Tris-Glycine gradient gel (Invitrogen) under reducing and non-reducing conditions. Cells are washed twice with ice-cold PBS, scraped off the plastic with a rubber policeman and suspended into single cells by pipetting cells up-and-down. C-peptide binding of cells is measured by incubating 2.5x105 cells with 15ug/ml of S1-Fc followed by incubation with fluorescent dye labeled goat-anti-human IgG antibody and analyzed by flow cytometry.

Generation of Adenoviral Vectors by Homologous Recombination in Bacteria (see also ISBN 0-89603-915-3, Hofkers and van Deursen, Transgenic Mouse Methods and Protocols, Methods in Molecular Biology, vol 209, HUMANA PRESS). The generation of recombinant adenoviral vectors by homologous recombination in for example the Ad-Easy System. The gene construct encoding the C-peptide of interest is cloned into shuttle vector 1, which contains the adenovirus left inverted terminal repeat (itr) and a region of several kbs immediately downstream from the E1 region. The deleted E1 region provides space necessary for insertion of the nucleotide encoding C-peptide. Upon homologous recombination with vector 2, which contains the remainder of the adenovirus genome up to the right inverted terminal repeat, full-length adenoviral vectors expressing C-peptide are generated.

### Overview 1. Elastin degradation and elastin peptides with a GxxP motif are associated with vascular disease.

### Elastin-derived peptides and Elastin Receptor Complex (ERC) mediated vascular disease

Activation of ERC by proteolytically degraded elastin peptides is associated with vascular disease.
- Matrix ageing and vascular impacts: focus on elastin fragmentation. Duca L, et al; Cardiovasc Res. 2016 Jun 1;110(3):298-308.
- Hellenthal FA, Buurman WA, Wodzig WK, Schurink GW. Biomarkers of AAA progression. Part 1: extracellular matrix degeneration. Nat Rev Cardiol 2009;6: 464 - 474.
- Monocyte chemotactic activity in human abdominal aortic aneurysms: role of elastin degradation -peptides and the 67-kD cell surface elastin receptor. Hance KA, et al; J Vasc Surg 2002;35:254 - 261.
- Elastin degradation is associated with progressive aortic stiffening and all-cause mortality in predialysis chronic kidney disease. Smith ER, et al; Hypertension. 2012 May;59(5):973-8

### Prototype synthetic elastin peptide SEQ ID NO: 41 (VGVAPG)

Evidence that interaction of SEQ ID NO: 41 (VGVAPG) with ERC may cause atherosclerosis and is involved in macrophage chemotaxis and angiogenesis.
- Elastin-derived peptides potentiate atherosclerosis through the immune Neu1-PI3Kγ pathway. Gayral S, et al; Cardiovasc Res. 2014 Apr 1;102(1):118-27.
- Induction of macrophage chemotaxis by aortic extracts from patients with Marfan syndrome is related to elastin binding protein. Guo G, et al; PLoS One. 2011;6(5): e20138.
- Elastin-derived peptides enhance angiogenesis by promoting endothelial cell migration and tubulogenesis through upregulation of MT1-MMP. Robinet A, et al; J Cell Sci. 2005 Jan 15;118 (Pt 2):343-56.
   Proteolytically degraded elastin peptides SEQ ID NO: 143 (SEQ ID NO: 143 (VPGVGISPEA) ) and SEQ ID NO: 144 (GVAPGIGPGG)
   Evidence that SEQ ID NO: 143 (SEQ ID NO: 143 (VPGVGISPEA) ) and SEQ ID NO: 144 (GVAPGIGPGG) localize in human atherosclerotic lesions and that serum levels of SEQ ID NO: 144 (GVAPGIGPGG) associate with acute myocardial infarction. Note: None of the below authors recognize the GxxP motif in SEQ ID NO: 143 (SEQ ID NO: 143 (VPGVGISPEA) ) and SEQ ID NO: 144 (GVAPGIGPGG)

   - Acute Myocardial Infarction and Pulmonary Diseases Result in Two Different Degradation Profiles of Elastin as Quantified by Two Novel ELISAs. Skjøt-Arkil H, et al; PLoS One. 2013 Jun 21;8(6):e60936.

Additional elastin derived peptides that interact with ERC and have biological activity are extensively discussed in:
- Degradation of tropoelastin by matrix metalloproteinases--cleavage site specificities and release of matrikines. Heinz A, et al; FEBS J. 2010 Apr;277(8):1939-56

### Overview 2. Non-elastin peptides that have a GxxP-motif ánd are associated with vascular disease.

### C-peptide with midportion SEQ ID NO: 8 (GGGPGAG)

Evidence that C-peptide localizes in human atherosclerotic lesions, induces macrophage chemotaxis and angiogenesis, that C-peptide may cause atherosclerosis and that serum levels of C-peptide associate with overall, cardiovascular and diabetes mortality. Typical degradation products of C-peptide are SEQ ID NO: 145 (VELGGGPGAGSLQP), SEQ ID NO: 146 (LGGGPGAGSLQP) and SEQ ID NO: 147 (LGGGPGAGS). Note: None of the below authors recognize the GxxP motif in C-peptide.
- C-peptide colocalizes with macrophages in early arteriosclerotic lesions of diabetic subjects and induces monocyte chemotaxis in vitro. Marx N, et al; Arterioscler Thromb Vasc Biol. 2004 Mar;24(3):540-5.
- Proinsulin C-peptide prevents impaired wound healing by activating angiogenesis in diabetes. Lim YC, et al; J Invest Dermatol. 2015 Jan;135(1):269-78.
- C-peptide promotes lesion development in a mouse model of arteriosclerosis. Vasic D, et al; J Cell Mol Med. 2012 Apr;16(4):927-35.
- Fasting serum C-peptide levels predict cardiovascular and overall death in nondiabetic adults. Patel N, et al; J Am Heart Assoc. 2012 Dec;1(6): e003152.
- C-peptide levels are associated with mortality and cardiovascular mortality in patients undergoing angiography: the LURIC study. Marx N, et al; Diabetes Care. 2013 Mar;36(3):708-14.
- Serum C-peptide levels and risk of death among adults without diabetes mellitus. Min JY, Min KB.

CMAJ. 2013 Jun 11;185(9):E402-8.
- Serum C-peptide levels as an independent predictor of diabetes mellitus mortality in non-diabetic individuals. Min JY, Min KB. Eur J Epidemiol. 2013 Sep;28(9):771-4.

Galectin-3 with N-terminal "collagen-like-stretch" SEQ ID NO: 148 (AGAGGYPGASYPGAYPGQAPPGAYPGQAPPGAYPGAPGAYPGAPAPGVYPGPPSG)

Evidence that galectin-3 plasma levels associate with heart failure. Note: None of the below authors recognize the GxxP motif in galectin-3.
- Galectin-3, a novel marker of macrophage activity, predicts outcome in patients with stable chronic heart failure. Van der Lok, D, et al; J Am Coll Cardiol 2007 49Suppl. A 98A[Abstract]
- Predictive value of plasma galectin-3 levels in heart failure with reduced and preserved ejection fraction. de Boer RA, et al; Ann Med. 2011 Feb;43(1):60-8.

### Fibrillinin-1 with motif SEQ ID NO: 149 (EGFEPG)

Evidence that interaction of SEQ ID NO: 149 (EGFEPG) with ERC is involved in macrophage chemotaxis.
- Induction of macrophage chemotaxis by aortic extracts of the mgR Marfan mouse model and a GxxPG-containing fibrillin-1 fragment. Guo G, et al; Circulation 2006; 114:1855 -1862.

### Laminin with motif SEQ ID NO: 137 (LGTIPG)

Evidence that laminin interacts via motif SEQ ID NO: 137 (LGTIPG) with ERC and induces fibroblast and tumor cell chemotaxis.
- The elastin receptor shows structural and functional similarities to the 67-kDa tumor cell laminin receptor. Mecham RP et al; J Biol Chem. 1989 Oct 5;264(28):16652-7.

**Table 1**

| Species | Uniprot identifier | C-peptide amino acid sequence |
|---|---|---|
| Human | >sp\|P01308\|57-87 | SEQ ID NO: 1 (EAEDLQVGQVELGGGPGAGSLQPLALEGSLQ) |
| human variant | rs121908279 | SEQ ID NO: 150 (EAE DLQVG QVEM GGG PGAGSLQPLALEGS LQ) |
| human variant | rs121908274 | SEQ ID NO: 151 (EAEDLQVGQVELGGGPGAGSLQPLALERSLQ) |
| chimpanzee | >sp\|P30410\|57-87 | SEQ ID NO: 1 (EAEDLQVGQVELGGGPGAGSLQPLALEGSLQ) |
| Gorilla | >sp\|Q6YK33\|57-87 | SEQ ID NO: 1 (EAEDLQVGQVELGGGPGAGSLQPLALEGSLQ) |
| orangutan | >sp\|Q8HXV2\|57-87 | SEQ ID NO: 1 (EAEDLQVGQVELGGGPGAGSLQPLALEGSLQ) |
| Gibbon | G1RSS5 | SEQ ID NO: 152 (EAEDPQVGQVELGGGPGAGSLQPLALEGSLQ) |
| macaque | >sp\|P30406\|57-87 | SEQ ID NO: 152 (EAEDPQVGQVELGGGPGAGSLQPLALEGSLQ) |
| green monkey | >sp\|P30407\|57-87 | SEQ ID NO: 152 (EAEDPQVGQVELGGGPGAGSLQPLALEGSLQ) |
| mouse insulin 2 | >sp\|P01326\|57-87 | SEQ ID NO: 153 (EVEDPQVAQLELGGGPGAGDLQTLALEVAQQ) |
| mouse insulin 1 | >sp\|P01325\|57-85 | SEQ ID NO: 167 (EVEDPQVEQLELGGSPGDLQTLALEVARQ) |
| rat insulin 2 | >sp\|P01323\|57-87 | SEQ ID NO: 154 EVEDPQVAQLELGGGPGAGDLQTLALEVARQ) |
| rat insulin 1 | >sp\|P01322\|57-87 | SEQ ID NO: 155 (EVEDPQVPQLELGGGPEAGDLQTLALEVARQ) |
| Horse | F6QQU6 | SEQ ID NO: 156 (EAE DPQVG QE ELGGG PG LGG LQPLALAG PQQ) |
| Horse | >sp\|P01310\|33-63 | SEQ ID NO: 157 (EAE DPQVG EVE LGGG PG LGG LQPLALAG PQQ) |
| Horse | Most horses | SEQ ID NO: 158 (EAE DPQVG QVE LGGG PG LGG LQPLALAG PQQ) |
| chinchilla | >sp\|P01327\|33-63 | SEQ ID NO: 159 (ELEDPQVGQADPGVVPEAGRLQPLALEMTLQ) |
| Guinea pig | >sp\|P01329\|57-87 | SEQ ID NO: 160 (E LE DPQVEQTE LG M G LGAGG LQPLALE MALQ) |
| Rabbit | >sp\|P01311\|57-87 | SEQ ID NO: 161 (EVEELQVGQAELGGGPGAGGLQPSALELALQ) |
| Bovine | >sp\|P01317\|57-82 | SEQ ID NO: 164 (EVEGPQVGALELAGGPGAGGLEGPPQ) |
| Bovine | Fleckvieh variant | SEQ ID NO: 165 (EVEGPQVGALELAGGLGAGGLEGPPQ) |
| Sheep | >sp\|P01318\|57-82 | SEQ ID NO: 164 (EVEGPQVGALELAGGPGAGGLEGPPQ) |
| Pig | >sp\|P01315\|57-85 | SEQ ID NO: 166 (EAENPQAGAVELGGGLGGLQALALEGPPQ) |
| Dog | >sp\|P01321\|57-87 | SEQ ID NO: 162 (EVEDLQVRDVELAGAPGEGGLQPLALEGALQ) |
| Cat | >sp\|P06306\|57-87 | SEQ ID NO: 163 (EAEDLQGKDAELGEAPGAGGLQPSALEAPLQ) |

TABLE 1: C-peptide, interspecies comparisons and alignments

**Table 2**

| A, elastic fiber proteins | |
|---|---|
| Elastin, P15502, H. sapiens | ₅₀₁ SEQ ID NO: 170 (GLVPGVGVAPGVGVAPGVGVAPGVGLAPGVGVAPGVGVAPG)₅₄₁ |
| Fibrillin-1, P35555, | ₄₁₁ SEQ ID NO: 168 (PVLPVPPGFPPGPQIPVPRP) ₄₃₀ - |
| H. sapiens | ₂₁₉₁ SEQ ID NO: 169 (TCEEGFEPGPM) ₂₂₀₁ |
| Fibrillin-2, P35556, | ₄₂₁ SEQ ID NO: 171 (LPMGGIPGSAGSRPGGTGGN) ₄₄₀- |
| H. sapiens | ₂₂₃₇ SEQ ID NO: 172 (NCNEGFEPGPM) ₂₂₄₇ |

| B, C-peptides | |
|---|---|
| P01308, H. sapiens | ₅₇ SEQ ID NO: 1 (EAEDLQVGQVELGGGPGAGSLQPLALEGSLQ) ₈₇ |
| P30410, P. troglodytes | ₅₇ SEQ ID NO: 1 (EAEDLQVGQVELGGGPGAGSLQPLALEGSLQ) ₈₇ |
| Q6YK33, G. gorilla | ₅₇ SEQ ID NO: 1 (EAEDLQVGQVELGGGPGAGSLQPLALEGSLQ) ₈₇ |
| Q8HXV2, P. pygmaeus | ₅₇ SEQ ID NO: 1 (EAEDLQVGQVELGGGPGAGSLQPLALEGSLQ) ₈₇ |
| P01325, M. musculus;(Ins-1) | ₅₇ SEQ ID NO: 167 (EVEDPQVEQLELGGSPGDLQTLALEVARQ) ₈₅ |
| P01326, M. musculus;(lns-2) | ₅₇ SEQ ID NO: 153 (EVEDPQVAQLELGGGPGAGDLQTLALEVAQQ) ₈₇ |
| P01322, R. norvegicus;(lns-1) | ₅₇ SEQ ID NO: 155 (EVEDPQVPQLELGGGPEAGDLQTLALEVARQ) ₈₇ |
| P01323, R. norvegicus;(lns-2) | ₅₇ SEQ ID NO: 154 (EVEDPQVAQLELGGGPGAGDLQTLALEVARQ) ₈₇ |
| Q62587, P. obesus | ₅₇ SEQ ID NO: 173 (GVDDPQMPQLELGGSPGAGDLRALALEVARQ) ₈₇ |
| G5C2F2, H. glaber | ₅₇ SEQ ID NO: 174 (ELENLQVGQAEPGMGLEAGGLQPLAQELALQ) ₈₇ |
| P01315, S. scrofa | ₅₇ SEQ ID NO: 166 (EAENPQAGAVELGGGLGGLQALALEGPPQ) ₈₅ |

Table 2 The presence of the elastin receptor binding motif GxxP (underlined) in vascular matrix proteins elastin and fibrillin and in C-peptides. Peptides are shown with their respective identifiers and amino acids are numbered as shown in the database Uniprot.

### Furthert identification of ERC-docking sites

The elastin-receptor-complex (ERC) is thought to cause vascular disease by binding excess peptide ligands derived from proteolysis of extra-cellular-matrix (ECM) after aging or smoking. We now identified novel ERC-ligands, notably in well-known biomarkers of vascular disease C-peptide (induced with insulin by high blood-glucose) and NTproBNP (induced in cardiomyocyte stress). We propose A) to investigate accumulation of ERC-ligands as central etiology of vascular disease, B) to early detect vascular disease risk by testing for ERC-ligands arising from accumulated risks diet, lifestyle and aging, that may all result in vascular disease.

**Background.** ERC is a complex of elastin binding protein (EBP), protective protein/cathepsin A and neuraminidase-1, found on leucocytes, fibroblasts and smooth muscle cells. ERC-ligands confirm to binding motifs xGxxPG or xxGxPG (G being glycine, P proline, x any amino acid), or xGxxPx if adapted to a type VIII beta-turn. Prototype ERC-ligand SEQ ID NO: 41 (VGVAPG) and others, such as SEQ ID NO: 197 (YGYGPG), SEQ ID NO: 198 (YGARPG), SEQ ID NO: 199 (FGAVPG), are derived by proteolysis from repeat areas in elastin. Others are SEQ ID NO: 149 (EGFEPG) (fibrilin) and SEQ ID NO: 137 (LGTIPG) (laminin). EBP separately binds galactosides. ERC-ligand binding to EBP is antagonized by V14 peptide. Circulating levels of ERC-ligands, generated from elastin proteolysis in aging or by smoking, have been associated with atherosclerosis, arterial stiffness, abdominal aortic aneurysms and myocardial infarction in humans, providing ample basis to explore early diagnosis, prevention and treatment of ERC-mediated vascular disease. A composite *in silico* model is available to dock ERC-ligands in EBP for structural analyses and candidate drug-development. *In vitro,* ERC-ligand/EBP structure-function relationship may be studied in human cells by testing leukocyte chemotaxis, and proliferation of smooth muscle cells. ERC-ligands induce atherosclerosis and resistance to insulin in mice allowing *in vivo* study of ERC-mediated vascular disease.

### Identification of ERC-ligand motifs derived by proteolysis from non-ECM proteins.

- A first find is C-peptide, a peptide derived by prohormone convertase cleavage (PC) from the pre-proinsulin gene and excreted in equimolar amounts with insulin. C-peptide carries the ERC-ligand motif SEQ ID NO: 34 (LGGGPG). Ido et al how C-peptide fragments with core motif SEQ ID NO: 8 (GGGPGAG) to mitigate glucose-induced vascular dysfunction in rats but do not recognize the ERC-ligand motif. C-peptide has been found atherogenic in mice and an independent marker of human vascular disease. Thus, finding a putative ERC-ligand SEQ ID NO: 34 (LGGGPG) in C-peptide acutely links ERC-mediated vascular disease to high circulating C-peptide levels. It surprisingly provides a common etiology of vascular disease after smoking as well as after diets high in glucose or starch, wherein both etiologies are causally linked to circulating ligands of ERC.
- A second find is galectin-3, which has an N-terminal domain, susceptible to proteolysis, with putative ERC-ligand repeat motifs SEQ ID NO: 44 (PGAYPG). Galectin-3 is an independent marker of human vascular disease as well as obesity that underlies vascular disease. As galectin-3 and EBP both bind galactosides and are causal to insulin resistance in mice we suggest a second relationship of galectin-3 to EBP next to putative ERC-ligand-receptor interaction.
- A third find is ERC-ligand peptide motif SEQ ID NO: 45 (QGVLPA) in loop 2 of beta-chorionic gonadotropin (beta-hCG), expressed during pregnancy, which loop is nicked by proteolysis from beta-hCG and involved in immunomodulation and angiogenesis.
- We docked newly found SEQ ID NO: 34 (LGGGPG), SEQ ID NO: 44 (PGAYPG) and SEQ ID NO: 45 (QGVLPA), and prototype SEQ ID NO: 41 (VGVAPG), in the *in-silico* model of EBP. All fit this composite model. Also, preliminary *in-vitro* results show inhibition of bioactivity of C-peptide by ERC-antagonists V14 peptide.
- We then performed a further search for proteins with xGxxPG or xxGxPG motifs closely flanked by PC cleavage sites, to identify ERC-ligands in refulatory model elements rf fragments thereof that may derive from pro-proteins. We found SEQ ID NO: 200 (GVGAPG), SEQ ID NO: 186 (PLGSPG), SEQ ID NO: 201 (DGAKPG), SEQ ID NO: 202 (QGMLPG), and SEQ ID NO: 196 (AGGAPG) in procalcitonin (PCT), amino-terminal pro-brain natriuretic peptide (NTproBNP), pro-opiomelanacortin (POMC), collagen 6A3 (COL6A3), and pyrin, respectively. PCT and NTproBNP each correlate with heart failure. POMC relates to regulation of feeding behavior and COL6A3 relates to adipocyte function in obesity and insulin resistance. Pyrin relates to innate immunity.

**Table 3 Biomarkers of vascular disease that carry the elastin receptor binding motif**

| Table 3 Biomarkers of vascular disease that carry the elastin receptor binding motif | **name** | relevant in silico hexa- fit in EBP peptide |
|---|---|---|
| Multiple occurences of docking motif | | |
| SEQ ID NO: 216 (VGVAPGVGVAPGVGVAPGVGLAPGVGVAPGVGVAPGVGVAPG) | Elastin | SEQ ID NO: 41 (VGVAPG) + |
| SEQ ID NO: 203 (FGLVPGVGVA) | | SEQ ID NO: 214 (FGLVPG) |
| SEQ ID NO: 144 (GVAPGIGPGG) | Elastin after MMP9/12 | SEQ ID NO: 215 (PGIGPG) |
| SEQ ID NO: 205 (PPGAYPGQAPPGAYPGAPGAYPGAPAPG) | Galectin-3 | SEQ ID NO: 44 (PGAYPG) + |
| Single occurence of docking motif | | |
| SEQ ID NO: 206 (TCEEGFEPGP) | Fibrillin-1 | SEQ ID NO: 149 (EGFEPG) |
| SEQ ID NO: 207 (NPLGTIPGGN) | Laminin beta-1 | SEQ ID NO: 137 (LGTIPG) |
| Single occurence of docking motif regulatory model element peptide | | |
| SEQ ID NO: 208 (RREAEDLQVGQVELGGGPGAGSLQPLALEGSLQKR) | proinsulin C-peptide | SEQ ID NO: 34 (LGGGPG) + |
| SEQ ID NO: 209 (RVLQGVLPALPQVVCNYR) | beta-hCG loop 2 | SEQ ID NO: 45 (QGVLPA) + |
| SEQ ID NO: 210 (KRCGNLSTCMLGTYTQDFNKFHTFPQTAIGVGAPGKKR) | Procalcitonin | SEQ ID NO: 200 (GVGAPG) |
| SEQ ID NO: 211 (RSHPLGSPGSASDLETSGLQEQR) | NT-proBNP | SEQ ID NO: 186 (PLGSPG) |
| SEQ ID NO: 212 (KREDVSAGEDCGPLPEGGPEPRSDGAKPGPREGKR) | Pro-opiomelanacortin | SEQ ID NO: 201 (DGAKPG) |
| SEQ ID NO: 213 (RAAPLQGMLPGLLAPLR) | Collagen 6A3 | SEQ ID NO: 202 (QGMLPG) |
| SEQ ID NO: 192 (RRNASSAGRLQGLAGGAPGQKECR) | Pyrin | SEQ ID NO: 196 (AGGAPG) |

We provide a blood test to early diagnose vascular disease. As we found that well-known circulating biomarkers of vascular disease, C-peptide, amino-terminal pro-B-type natriuretic peptide (NT-proBNP) and galectin-3, and others, share a little-known docking site with circulating elastin-derived-peptides (EDP). Through this docking site, EDP activate the elastin-receptor-complex (ERC) that is expressed on cells throughout the human arterial system. ERC contributes to elastin degradation and arterial wall remodeling. Experimental activation of ERC by EDP induces insulin resistance and atherosclerosis in mice. Excess EDP/ERC docking causes chemotaxis of human leukocytes and proliferation of human smooth muscle cells (SMC) and is associated with loss of arterial elasticity, atherosclerosis, increased arterial stiffness, abdominal aortic aneurysms and myocardial infarction in humans. Said blood test preferably detects ERC-docking sites shared by circulating C-peptide, NT-proBNP, galectin-3 and EDP, in order to earlier and better determine with more precision our accumulated risks on vascular disease than now is done with each of the biomarkers alone. C-peptide (secreted in equimolar amounts with insulin) predicts diet induced risks on all-cause-mortality, cardiovascular-mortality and new-onset type 2 diabetes. NT-proBNP predicts cardiac stress induced cardio-vascular-mortality within or without type 2 diabetes. Inflammatory mediator galectin-3 predicts development and progression of heart failure and insulin resistance. EDP, generated from elastin proteolysis in aging or by smoking, is associated with human vascular disease as listed above.

In summary, ERC is a complex of elastin binding protein (EBP), protective protein/cathepsin A and neuraminidase-1, found on leucocytes, fibroblasts and smooth muscle cells. ERC-ligands confirm to binding motifs xGxxPG or xxGxPG (G being glycine, P proline, x any amino acid), or xGxxPx if adapted to a type VIII beta-turn, herein said motifs jointly called PG-domain. Prototype ERC-ligand SEQ ID NO: 41 (VGVAPG) and other elastin peptides, such as SEQ ID NO: 143 (VPGVGISPEA), are derived by proteolysis from repeat areas in elastin. Others are SEQ ID NO: 149 (EGFEPG) from fibrillin and laminin SEQ ID NO: 137 (LGTIPG). EBP separately binds galactosides. ERC-ligand binding to EBP is antagonized by V14 peptide or lactose. Circulating levels of ERC-ligands, generated from elastin proteolysis in aging or by smoking have been associated with atherosclerosis, arterial stiffness, abdominal aortic aneurysms and myocardial infarction in humans, providing ample basis to explore early diagnosis, prevention and treatment of ERC-mediated vascular disease. A composite *in silico* model is available to dock ERC-ligands in EBP for structural analyses and candidate drug-development. *In vitro,* ERC-ligand/EBP structure-function relationship may be studied in human cells by testing leukocyte chemotaxis and proliferation of smooth muscle cells. ERC-ligands induce atherosclerosis and resistance to insulin in mice allowing *in vivo* study of ERC-mediated vascular disease.
- A first find is C-peptide, a peptide derived by prohormone convertase cleavage (PC) from the pre-proinsulin gene and excreted in equimolar amounts with insulin. C-peptide carries the ERC-ligand motif LGGGPG. Ido et al show C-peptide fragments with core motif SEQ ID NO: 8 GGGPGAG to mitigate glucose-induced vascular dysfunction in rats but do not recognize the ERC-ligand motif. C-peptide has been found atherogenic in mice and an independent marker of human vascular disease. Thus, finding a putative ERC-ligand SEQ ID NO: 34 (LGGGPG) in C-peptide acutely links ERC-mediated vascular disease to high circulating C-peptide levels. It surprisingly provides a common etiology of vascular disease after smoking as well as after diets high in glucose or starch, wherein both etiologies are causally linked to circulating ligands of ERC.
- A second find is galectin-3, which has an N-terminal domain, susceptible to proteolysis, with putative ERC-ligand repeat motifs SEQ ID NO: 44 (PGAYPG). Galectin-3 is an independent marker of human vascular disease as well as obesity that underlies vascular disease. As galectin-3 and EBP both bind galactosides and are causal to insulin resistance in mice (we suggest a second relationship of galectin-3 to EBP next to putative ERC-ligand-receptor interaction.
- A third find is ERC-ligand peptide motif SEQ ID NO: 45 (QGVLPA) in loop 2 of beta-chorionic gonadotropin (beta-hCG), expressed during pregnancy, which loop is nicked by proteolysis from beta-hCG and involved in immunomodulation and angiogenesis.
- We docked newly found SEQ ID NO: 34 (LGGGPG), SEQ ID NO: 44 (PGAYPG) and SEQ ID NO: 45 (QGVLPA), and prototype elastin peptide SEQ ID NO: 41 (VGVAPG), in the *in-silico* model of EBP. All motifs fit this composite model, showing adaptation to a type VIII beta-turn.
- We then performed a further search for proteins with xGxxPG or xxGxPG motifs closely flanked by PC cleavage sites, to identify ERC-ligands that may derive from pro-proteins. We found SEQ ID NO: 200 (GVGAPG,) SEQ ID NO: 186 (PLGSPG), SEQ ID NO: 201 (DGAKPG), SEQ ID NO: 202 (QGMLPG), and SEQ ID NO: 196 (AGGAPG) in procalcitonin (PCT), amino-terminal pro-brain natriuretic peptide (NTproBNP), pro-opiomelanacortin (POMC), collagen 6A3 (COL6A3), and pyrin, respectively. PCT and NTproBNP each correlate with heart failure POMC relates to regulation of feeding behavior and COL6A3 relates to adipocyte function in obesity and insulin resistance. Pyrin relates to innate immunity.

In conclusion, we find that these biomarkers, albeit each relating to separate risk factors of vascular disease, varying from diet, cardiac stress, inflammation, aging to smoking, have an ERC-docking site in common that merits detection to early diagnose combined risks on human vascular disease.

Cardiovasc Res. 2016 Jun 1;110(3):298; Diabetes. 2013 Nov;62(11):3807; Cardiovasc Res. 2014 Apr 1;102(1):118; Hypertension. 2012 May;59(5):973; J Immunol. 2016 Jun 1;196(11):4536; PLoS One. 2013 Jun 21;8(6): e60936; CMAJ. 2013 Jun 11;185(9): E402; Diabetes Ther. 2017 Jun;8(3):475; Heart Fail Clin. 2018 Jan;14(1):27; Atherosclerosis. 2017 Sep; 264:67; Eur J Heart Fail. 2009 Sep;11(9):811; Cell. 2016 Nov 3;167(4):973; J Clin Invest. 2006 Mar 1; 116(3): 753

## Claims

1. A method for diagnosing vascular disease risk of a human, comprising testing a biological sample of said human for the presence of at least two biomarkers each having a PG-domain different from each other, wherein said PG-domain is an amino acid motif xGxxPG wherein G is a glycine, P is a proline and x is any amino acid, and wherein said biomarkers are selected from the group of C-peptide, fragments of C-peptide, elastin, fragments of elastin, fibrillin, fragments of fibrillin, laminin, fragments of laminin, galectin-3, fragments of galectin-3, POMC, fragments of POMC, COL6A3, fragments of COL6A3, pyrin, fragments of pyrin.

2. The method according to claim 1 wherein said at least two biomarker peptides have a PG-domain motif selected from the group of peptide motifs with SEQ ID NO: 149, SEQ ID NO: 137, SEQ ID NO: 34, SEQ ID NO: 44, SEQ ID NO: 41, SEQ ID NO: 201, SEQ ID NO: 202, and SEQ ID NO: 196.

3. The method according to claim 1 or 2 wherein said at least two biomarker peptides have a PG-domain motif selected from the group of peptide motifs with SEQ ID NO: 34 and SEQ ID NO: 41.

4. The method according to anyone of claims 1 to 3 comprising testing said human for the presence of at least three biomarker peptides each having a PG-domain different from each other.

5. The method according to anyone of claims 1 to 4 comprising testing said sample with a mass-spectrometer for the presence of said biomarker peptides that have a PG-domain motif.

6. The method according to anyone of claims 1 to 4 comprising testing said sample with a multiple antibody test, said antibodies specifically directed against said biomarker peptides that have a PG-domain motif.

7. The method according to anyone of claims 1 to 4 comprising testing said sample with a single-binding-molecule test, said single-binding-molecule specifically directed against said biomarker peptides that have a PG-domain motif.

8. The method according to claim 7 wherein said single-binding-molecule is derived from the elastin-binding-protein.

9. The method according to claim 7 or 8 wherein said single-binding-molecule at least comprises a peptide sequence with motif SEQ ID NO: 31.

10. The method according to claim 7 or 8 wherein said single-binding-molecule at least comprises a peptide sequence with motif SEQ ID NO: 131.

## Patentansprüche

1. Verfahren zur Diagnose des Risikos einer Gefäßerkrankung bei einem Menschen, umfassend Testen einer biologischen Probe dieses Menschen auf das Vorhandensein von mindestens zwei Biomarkern, die jeweils eine PG-Domäne aufweisen, die sich voneinander unterscheiden, wobei die PG-Domäne ein Aminosäuremotiv xGxxPG ist, wobei G ein Glycin ist, P ein Prolin ist und x eine beliebige Aminosäure ist, und wobei die Biomarker ausgewählt sind aus der Gruppe von C-Peptid, Fragmenten von C-Peptid, Elastin, Fragmenten von Elastin, Fibrillin, Fragmenten von Fibrillin, Laminin, Fragmenten von Laminin, Galektin-3, Fragmente von Galektin-3, POMC, Fragmente von POMC, COL6A3, Fragmente von COL6A3, Pyrin, Fragmente von Pyrin.

2. Verfahren gemäß Anspruch 1, wobei die mindestens zwei Biomarker Peptide ein PG-Domänenmotiv aufweisen, das aus der Gruppe der Peptidmotive mit SEQ ID NO: 149, SEQ ID NO: 137, SEQ ID NO: 34, SEQ ID NO: 44, SEQ ID NO: 41, SEQ ID NO: 201, SEQ ID NO: 202, und SEQ ID NO: 196 ausgewählt ist.

3. Verfahren gemäß Anspruch 1 oder 2, wobei die mindestens zwei Biomarker Peptide ein PG-Domänenmotiv aufweisen, das aus der Gruppe der Peptidmotive mit SEQ ID NO: 34 und SEQ ID NO: 41 ausgewählt ist.

4. Verfahren gemäß irgendeinem der Ansprüche 1 bis 3, umfassend Testen des Menschen auf das Vorhandensein von mindestens drei Biomarker Peptiden, die jeweils eine voneinander verschiedenes PG-Domänenmotiv aufweisen.

5. Verfahren gemäß irgendeinem der Ansprüche 1 bis 4, umfassend das Testen der Probe mit einem Massenspektrometer auf das Vorhandensein der Biomarker Peptide, die ein PG-Domänenmotiv aufweisen.

6. Verfahren gemäß irgendeinem der Ansprüche 1 bis 4, umfassend Testen der Probe mit einem mehrfach Antikörpertest, wobei die Antikörper spezifisch gegen die Biomarker Peptide gerichtet sind, die ein PG-Domänenmotiv aufweisen.

7. Verfahren gemäß irgendeinem der Ansprüche 1 bis 4, umfassend das Testen der Probe mit einem Einzelbindungsmolekültest, wobei das Einzelbindungsmolekül spezifisch gegen die Biomarker Peptide gerichtet ist, die ein PG-Domänenmotiv aufweisen.

8. Verfahren gemäß Anspruch 7, wobei das Einzelbindungsmolekül von dem Elastin-Bindungsprotein abgeleitet ist.

9. Verfahren gemäß Anspruch 7 oder 8, wobei das Einzelbindungsmolekül mindestens eine Peptidsequenz mit dem Motiv SEQ ID NO: 31 umfasst.

10. Verfahren gemäß Anspruch 7 oder 8, wobei das Einzelbindungsmolekül mindestens eine Peptidsequenz mit dem Motiv SEQ ID NO: 131 umfasst.

## Revendications

1. Procédé de diagnostic d'un risque de maladie vasculaire d'un être humain, comprenant le test d'un échantillon biologique dudit être humain pour la présence d'au moins deux biomarqueurs ayant chacun un domaine PG différent l'un de l'autre, dans lequel ledit domaine PG est un motif d'acides aminés xGxxPG dans lequel G est une glycine, P est une proline et x est un quelconque acide aminé et dans lequel lesdits biomarqueurs sont choisis dans le groupe de peptide C, fragments de peptide C, élastine, fragments d'élastine, fibrilline, fragments de fibrilline, laminine, fragments de laminine, galectine-3, fragments de galectine-3, POMC, fragments de POMC, COL6A3, fragments de COL6A3, pyrine, fragments de pyrine.

2. Procédé selon la revendication 1 dans lequel lesdits au moins deux peptides biomarqueurs ont un motif de domaine PG choisi dans le groupe des motifs de peptide avec SEQ ID NO : 149, SEQ ID NO : 137, SEQ ID NO : 34, SEQ ID NO : 44, SEQ ID NO : 41, SEQ ID NO : 201, SEQ ID NO : 202 et SEQ ID NO : 196.

3. Procédé selon la revendication 1 ou la revendication 2 dans lequel lesdits au moins deux peptides biomarqueurs ont un motif de domaine PG choisi dans le groupe des motifs de peptide avec SEQ ID NO : 34 et SEQ ID NO : 41.

4. Procédé selon l'une quelconque des revendications 1 à 3 comprenant le test dudit être humain pour la présence d'au moins trois peptides biomarqueurs ayant chacun un domaine PG différent l'un de l'autre.

5. Procédé selon l'une quelconque des revendications 1 à 4 comprenant le test dudit échantillon avec un spectromètre de masse pour la présence desdits peptides biomarqueurs qui ont un motif de domaine PG.

6. Procédé selon l'une quelconque des revendications 1 à 4 comprenant le test dudit échantillon avec un test d'anticorps multiples, lesdits anticorps étant spécifiquement dirigés contre lesdits peptides biomarqueurs qui ont un motif de domaine PG.

7. Procédé selon l'une quelconque des revendications 1 à 4 comprenant le test dudit échantillon avec un test de molécule de liaison unique, ladite molécule de liaison unique étant spécifiquement dirigée contre lesdits peptides biomarqueurs qui ont un motif de domaine PG.

8. Procédé selon la revendication 7 dans lequel ladite molécule de liaison unique est dérivée de la protéine de liaison à l'élastine.

9. Procédé selon la revendication 7 ou la revendication 8 dans lequel ladite molécule de liaison unique comprend au moins une séquence de peptides avec le motif SEQ ID NO : 31.

10. Procédé selon la revendication 7 ou la revendication 8 dans lequel ladite molécule de liaison unique comprend au moins une séquence de peptides avec le motif SEQ ID NO : 131.
